# EUROPEAN PATENT SPECIFICATION

(11) **EP 3 988 557 B1**
(45) Date of publication and mention of the grant of the patent: **22.11.2023**
(21) Application number: 21204412.7
(22) Date of filing: 25.10.2021
(51) Int. Cl.: C07F 15/00, C09K 11/06, H10K 85/30, H10K 101/10

(54) **ORGANOMETALLIC COMPOUND, ORGANIC LIGHT-EMITTING DEVICE INCLUDING THE SAME, AND DIAGNOSTIC COMPOSITION INCLUDING THE ORGANOMETALLIC COMPOUND**
ORGANOMETALLISCHE VERBINDUNG, ORGANISCHE LICHTEMITTIERENDE VORRICHTUNG DAMIT UND DIAGNOSEZUSAMMENSETZUNG MIT DER ORGANOMETALLISCHEN VERBINDUNG
COMPOSÉ ORGANOMÉTALLIQUE, DISPOSITIF ÉLECTROLUMINESCENT ORGANIQUE LE COMPRENANT ET COMPOSITION DE DIAGNOSTIC COMPRENANT LE COMPOSÉ ORGANOMÉTALLIQUE

(30) Priority: 26.10.2020 KR 20200139747
(43) Date of publication of application: 27.04.2022
(73) Proprietor: Samsung Electronics Co., Ltd., Suwon-si, Gyeonggi-do 16677 (KR)
(72) Inventor: KWON, Ohyun, 16678 Suwon-si (KR); RAI, Virendra Kumar, 16678 Suwon-si (KR); PARK, Bumwoo, 16678 Suwon-si (KR); KIM, Sungmin, 16678 Suwon-si (KR); SIM, Myungsun, 16678 Suwon-si (KR); CHOI, Byoungki, 16678 Suwon-si (KR); KOISHIKAWA, Yasushi, 16678 Suwon-si (KR)
(74) Representative: Elkington and Fife LLP

(56) References cited:
- KR-A- 20110 041 729
- US-A1- 2020 087 334

## Description

### FIELD OF THE INVENTION

One or more embodiments relate to organometallic compounds, organic light-emitting devices including the same, and diagnostic compositions including the same.

### BACKGROUND OF THE INVENTION

Organic light-emitting devices are self-emission devices, which have improved characteristics in terms of viewing angles, response time, brightness, driving voltage, and response speed, and produce full-color images.

In an example, an organic light-emitting device includes an anode, a cathode, and an organic layer between the anode and the cathode, wherein the organic layer includes an emission layer. A hole transport region may be located between the anode and the emission layer, and an electron transport region may be located between the emission layer and the cathode. Holes provided from the anode may move toward the emission layer through the hole transport region, and electrons provided from the cathode may move toward the emission layer through the electron transport region. The holes and the electrons recombine in the emission layer to produce excitons. These excitons transition from an excited state to a ground state to thereby generate light. KR 20110041729 and US 2020/087334 disclose organic light emitting devices comprising condensed aromatic ring compounds.

### SUMMARY OF THE INVENTION

One or more embodiments relate to organometallic compounds, organic light-emitting devices including the same, and diagnostic compositions including the same.

Additional aspects will be set forth in part in the description, which follows and, in part, will be apparent from the description, or may be learned by practice of the presented embodiments of the disclosure.

Provided is an organometallic compound represented by Formula 1

Formula 1 M₁(Ln₁)ₙ₁(Ln₂)ₙ₂,

wherein, in Formula 1,
M₁ is a transition metal,
Ln₁ is a ligand represented by Formula 1-1,
Ln₂ is a ligand represented by Formula 1-2,
n1 is 0, 1, or 2,
n2 is 1, 2, or 3, 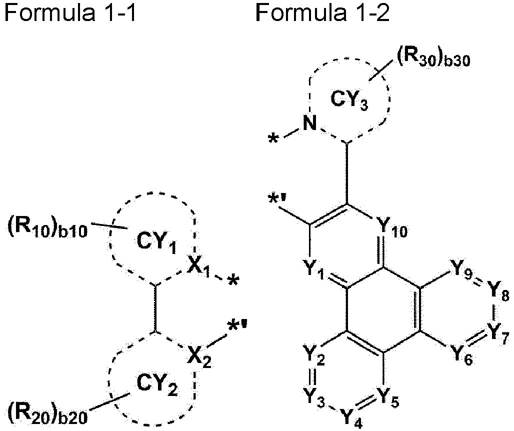
wherein, in Formulae 1-1 and 1-2,
X₁ is C or N, and X₂ is C or N,
Y₁ is C(R₄₁) or N, Y₂ is C(R₄₂) or N, Y₃ is C(R₄₃) or N, Y₄ is C(R₄₄) or N, Y₅ is C(R₄₅) or N, Y₆ is C(R₄₆) or N, Y₇ is C(R₄₇) or N, Y₈ is C(R₄₈) or N, Y₉ is C(R₄₉) or N, and Y₁₀ is C(R₅₀) or N,
CY₁ and CY₂ are each independently a C₅-C₃₀ carbocyclic group or a C₁-C₃₀ heterocyclic group,
CY₃ is a N-containing C₁-C₃₀ heterocyclic group,
R₁₀, R₂₀, R₃₀ and R₄₁ to R₅₀ are each independently hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted monovalent non-aromatic condensed polycyclic group, a substituted or unsubstituted monovalent non-aromatic condensed heteropolycyclic group, -Si(Q₁)(Q₂)(Q₃), -Ge(Q₁)(Q₂)(Q₃), -N(Q₄)(Q₅), - B(Q₆)(Q₇), or -P(=O)(Q₈)(Q₉),
at least one of a ligand represented by Formula 1-1, a ligand represented by Formula 1-2, or a combination thereof comprises one or more -Ge(Q₁)(Q₂)(Q₃),
two or more of a plurality of R₁₀ are optionally bonded together to form a substituted or unsubstituted C₅-C₃₀ carbocyclic group or a substituted or unsubstituted C₁-C₃₀ heterocyclic group,
two or more of a plurality of R₂₀ are optionally bonded together to form a substituted or unsubstituted C₅-C₃₀ carbocyclic group or a substituted or unsubstituted C₁-C₃₀ heterocyclic group,
two or more of a plurality of R₃₀ are optionally bonded together to form a substituted or unsubstituted C₅-C₃₀ carbocyclic group or a substituted or unsubstituted C₁-C₃₀ heterocyclic group,
neighboring two or more of R₁₀, R₂₀, R₃₀ and R₄₁ to R₅₀ are optionally bonded to each other to form a substituted or unsubstituted C₅-C₃₀ carbocyclic group or a substituted or unsubstituted C₁-C₃₀ heterocyclic group,
b10, b20, and b30 are each independently be 1, 2, 3, 4, 5, 6, 7, 8, 9, or 10,
at least one substituent of the substituted C₅-C₃₀ carbocyclic group, the substituted C₁-C₃₀ heterocyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₂-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted C₁-C₆₀ heteroaryloxy group, the substituted C₁-C₆₀ hetero arylthio group, the substituted monovalent non-aromatic condensed polycyclic group, and the substituted monovalent non-aromatic condensed heteropolycyclic group is:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, or a C₁-C₆₀ alkoxy group,
a C₁-C₆₀ alkyl group, a C₂-C₆₀ alkenyl group, a C₂-C₆₀ alkynyl group, or a C₁-C₆₀ alkoxy group, each substituted with deuterium, -F, -Cl, -Br, -I, -CD₃, - CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a C₁-C₆₀ heteroaryloxy group, a C₁-C₆₀ heteroarylthio group, a monovalent non-aromatic condensed polycyclic group, a monovalent non-aromatic condensed heteropolycyclic group, - Si(Q₁₁)(Q₁₂)(Q₁₃), -Ge(Q₁₁)(Q₁₂)(Q₁₃), -N(Q₁₄)(Q₁₅), -B(Q₁₆)(Q₁₇), - P(=O)(Q₁₈)(Q₁₉), or a combination thereof,
a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a C₁-C₆₀ heteroaryloxy group, a C₁-C₆₀ heteroarylthio group, a monovalent non-aromatic condensed polycyclic group, or a monovalent non-aromatic condensed heteropolycyclic group,
a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a C₁-C₆₀ heteroaryloxy group, a C₁-C₆₀ heteroarylthio group, a monovalent non-aromatic condensed polycyclic group, a monovalent non-aromatic condensed heteropolycyclic group, -Si(Q₂₁)(Q₂₂)(Q₂₃), -Ge(Q₂₁)(Q₂₂)(Q₂₃), -N(Q₂₄)(Q₂₅), - B(Q₂₆)(Q₂₇), -P(=O)(Q₂₈)(Q₂₉), or a combination thereof, or
-Si(Q₃₁)(Q₃₂)(Q₃₃), -Ge(Q₃₁)(Q₃₂)(Q₃₃), -N(Q₃₄)(Q₃₅), -B(Q₃₆)(Q₃₇), or - P(=O)(Q₃₈)(Q₃₉),
wherein Q₁ to Q₉, Q₁₁ to Q₁₉, Q₂₁ to Q₂₉, and Q₃₁ to Q₃₉ may each independently be hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a C₁-C₆₀ heteroaryloxy group, a C₁-C₆₀ heteroarylthio group, a substituted or unsubstituted monovalent non-aromatic condensed polycyclic group, or a substituted or unsubstituted monovalent non-aromatic condensed heteropolycyclic group.
Another aspect provides an organic light-emitting device including a first electrode, a second electrode, and an organic layer including an emission layer located between the first electrode and the second electrode, wherein the organic layer includes at least one of the organometallic compounds.

The organometallic compound may be included in the emission layer of the organic layer, and the organometallic compound included in the emission layer may act as a dopant.

Another aspect provides a diagnostic composition including at least one organometallic compound represented by Formula 1.

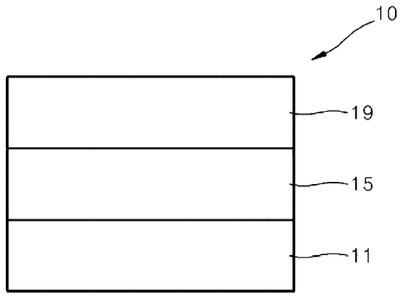

### BRIEF DESCRIPTION OF THE DRAWING

The above and other aspects, features, and advantages of certain embodiments of the disclosure will be more apparent from the following description taken in conjunction with FIGURE which shows a schematic cross-sectional view of an organic light-emitting device according to an exemplary embodiment.

### DETAILED DESCRIPTION OF THE EMBODIMENTS

For the sake of convenience 1 Torr= 133, 322 Pascal. Reference will now be made in detail to embodiments, examples of which are illustrated in the accompanying drawings, wherein like reference numerals refer to like elements throughout the specification. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

It will be understood that when an element is referred to as being "on" another element, it can be directly on the other element or intervening elements may be present therebetween In contrast, when an element is referred to as being "directly on" another element, there are no intervening elements present

It will be understood that, although the terms "first," "second," "third" etc. may be used herein to describe various elements, components, regions, layers and/or sections, these elements, components, regions, layers and/or sections should not be limited by these terms These terms are only used to distinguish one element, component, region, layer or section from another element, component, region, layer or section Thus, "a first element," "component," "region," "layer" or "section" discussed below could be termed a second element, component, region, layer or section without departing from the teachings herein.

The terminology used herein is for the purpose of describing particular embodiments only and is not intended to be limiting. As used herein, "a," "an," "the," and "at least one" do not denote a limitation of quantity, and are intended to cover both the singular and plural, unless the context clearly indicates otherwise. For example, "an element" has the same meaning as "at least one element," unless the context clearly indicates otherwise.

"Or" means "and/or." As used herein, the term "and/or" includes any and all combinations of one or more of the associated listed items It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Furthermore, relative terms, such as "lower" or "bottom" and "upper" or "top," may be used herein to describe one element's relationship to another element as illustrated in the Figures It will be understood that relative terms are intended to encompass different orientations of the device in addition to the orientation depicted in the Figures For example, if the device in one of the figures is turned over, elements described as being on the "lower" side of other elements would then be oriented on "upper" sides of the other elements The exemplary term "lower," can therefore, encompasses both an orientation of "lower" and "upper," depending on the particular orientation of the figure Similarly, if the device in one of the figures is turned over, elements described as "below" or "beneath" other elements would then be oriented "above" the other elements The exemplary terms "below" or "beneath" can, therefore, encompass both an orientation of above and below.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within ± 30%, 20%, 10% or 5% of the stated value.

Unless otherwise defined, all terms (including technical and scientific terms) used herein have the same meaning as commonly understood by one of ordinary skill in the art to which this disclosure belongs It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments are described herein with reference to cross section illustrations that are schematic illustrations of idealized embodiments As such, variations from the shapes of the illustrations as a result, for example, of manufacturing techniques and/or tolerances, are to be expected Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features Moreover, sharp angles that are illustrated may be rounded Thus, the regions illustrated in the figures are schematic in nature and their shapes are not intended to illustrate the precise shape of a region and are not intended to limit the scope of the present claims.

An aspect of the present disclosure provides an organometallic compound represented by Formula 1 below:

Formula 1 M₁(Ln₁)ₙ₁(Ln₂)ₙ₂

M₁ in Formula 1 may be a transition metal.

For example, M₁ may be a Period 1 transition metal, a Period 2 transition metal, or a Period 3 transition metal.

In an embodiment, M₁ may be iridium (Ir), platinum (Pt), osmium (Os), titanium (Ti), zirconium (Zr), hafnium (Hf), europium (Eu), terbium (Tb), thulium (Tm), or rhodium (Rh).

In an embodiment, M₁ may be Ir, Pt, Os, or Rh.

In an embodiment, M₁ may be Ir.

In Formula 1, n1 may be 1 or 2, and n2 may be 1, 2, or 3.

In an embodiment, the sum of n1 and n2 may be 2 or 3.

In an embodiment, M₁ may be Ir and the sum of n1 and n2 may be 3.

In an embodiment, M₁ may be Pt, and the sum of n1 and n2 may be 2.

Ln₁ in Formula 1 may be a ligand represented by Formula 1-1. 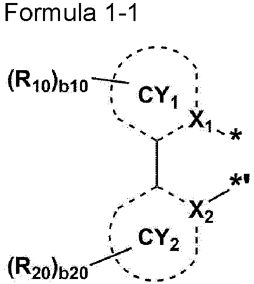 Ln₂ in Formula 1 may be a ligand represented by Formula 1-2. 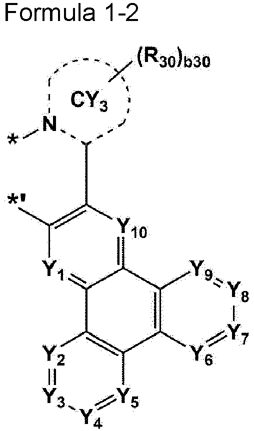 in Formula 1-1 , X₁ may be C or N and X₂ may be C or N.

In Formula 1-2, Y₁ may be C(R₄₁) or N, Y₂ may be C(R₄₂) or N, Y₃ may be C(R₄₃) or N, Y₄ may be C(R₄₄) or N, Y₅ may be C(R₄₅) or N, Y₆ may be C(R₄₆) or N, Y₇ may be C(R₄₇) or N, Y₈ may be C(R₄₈) or N, Y₉ may be C(R₄₉) or N, and Y₁₀ may be C(R₅₀) or N.

CY₁ and CY₂ in Formula 1-1 may each independently be a C₅-C₃₀ carbocyclic group or a C₁-C₃₀ heterocyclic group.

CY₃ in Formula 1-2 may be an N-containing C₁-C₃₀ heterocyclic group.

In an embodiment, rings CY₁ and CY₂ may be i) a first ring, ii) a second ring, iii) a condensed cyclic group in which two or more first rings are condensed with each other, iv) a condensed cyclic group in which two or more second rings are condensed with each other, or v) a condensed cyclic group in which at least one first ring is condensed with at least one second ring,
the first ring may be a cyclopentane group, a cyclopentadiene group, a furan group, a thiophene group, a pyrrole group, a silole group, an indene group, a benzofuran group, a benzothiophene group, an indole group, a benzosilole group, an oxazole group, an isoxazole group, an oxadiazole group, an isoxadiazole group, an oxatriazole group, an isoxatriazole group, a thiazole group, an isothiazole group, a thiadiazole group, an isothiadiazole group, a thiatriazole group, an isothiatriazole group, a pyrazole group, an imidazole group, a triazole group, a tetrazole group, an azasilole group, a diazasilole group, or a triazasilole group, and
the second ring may be an adamantane group, a norbornane group, a norbornene group, a cyclohexane group, a cyclohexene group, a benzene group, a pyridine group, a pyrimidine group, a pyrazine group, a pyridazine group, or a triazine group.

In an embodiment, CY₁ and CY₂ may each independently be a cyclopentane group, a cyclohexane group, a cycloheptane group, a cyclopentene group, a cyclohexene group, a cycloheptene group, a benzene group, a naphthalene group, an anthracene group, a phenanthrene group, a triphenylene group, a pyrene group, a chrysene group, a cyclopentadiene group, a 1 ,2,3,4-tetrahydronaphthalene group, a thiophene group, a selenophene group, a borole group, a phosphole group, a silole group, a germole group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluoren-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluoren-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isooxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group.

In an embodiment, CY₁ and CY₂ may each independently be a benzene group, a naphthalene group, 1,2,3,4-tetrahydronaphthalene group, a phenanthrene group, a pyridine group, a pyrimidine group, a pyrazine group, a triazine group, a quinoline group, an isoquinoline group, a quinoxaline group, a quinazoline group, a phenanthroline group, a benzofuran group, a benzothiophene group, a fluorene group, a carbazole group, a dibenzofuran group, a dibenzothiophene group, a dibenzosilole group, an azafluorene group, an azacarbazole group, an azadibenzofuran group, an azadibenzothiophene group, or an azadibenzosilole group.

In an embodiment, CY₁ may be a pyridine group, a pyrimidine group, a pyrazine group, a pyridazine group, a triazine group, a quinoline group, an isoquinoline group, a quinoxaline group, or a quinazoline group.

In an embodiment, CY₂ may be a benzene group, a naphthalene group, a pyridine group, a pyrimidine group, a pyrazine group, a pyridazine group, a triazine group, a quinoline group, an isoquinoline group, a quinoxaline group, a quinazoline group, a fluorene group, a carbazole group, a dibenzofuran group, a dibenzothiophene group, or a dibenzosilole group.

In an embodiment, CY₃ may be a pyridine group, a pyrimidine group, a pyrazine group, a pyridazine group, a triazine group, a quinoline group, an isoquinoline group, a quinoxaline group, or a quinazoline group.

R₁₀, R₂₀, R₃₀, and R₄₁ to R₅₀ in Formula 1 may each independently be hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted monovalent non-aromatic condensed polycyclic group, a substituted or unsubstituted monovalent non-aromatic condensed heteropolycyclic group, -N(Q₁)(Q₂), - Si(Q₃)(Q₄)(Q₅), -Ge(Q₃)(Q₄)(Q₅), -B(Q₆)(Q₇), or -P(=O)(Q₈)(Q₉).

b10, b20, and b30 in Formula 1 may each independently be 1, 2, 3, 4, 5, 6, 7, 8, 9, or 10.

In an embodiment, b10, b20, and b30 may each independently be 1, 2, 3, 4, 5, 6, 7, or 8.

In an embodiment, b10, b20, and b30 may each independently be 1, 2, 3, or 4.

In an embodiment, b10, b20, and b30 may each independently be 1 or 2.

In an embodiment, b10, b20, and b30 may each independently be 1.

Formula 1-1 or Formula 1-2 may include one or more -Ge(Q₁)(Q₂)(Q₃).

In an embodiment, ligand Ln₁ represented by Formula 1-1 may include one or more -Ge(Q₁)(Q₂)(Q₃). In an embodiment, ligand Ln₂ represented by Formula 1-2 may include one or more -Ge(Q₁)(Q₂)(Q₃).

In an embodiment, R₁₀, R₂₀, R₃₀ and R₄₁ to R₅₀ may each independently be hydrogen, deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a C₁-C₂₀ alkyl group, or a C₁-C₂₀ alkoxy group;
a C₁-C₂₀ alkyl group or a C₁-C₂₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, or a pyrimidinyl group;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, or a combination thereof; or
-Si(Q₁)(Q₂)(Q₃), -Ge(Q₁)(Q₂)(Q₃), -N(Q₄)(Q₅), -B(Q₆)(Q₇), or - P(=O)(Q₈)(Q₉).

In an embodiment, R₁₀, R₂₀, R₃₀, and R₄₁ to R₅₀ may each independently be hydrogen, deuterium, -F, -Cl, -Br, -I, -CD₃, -CD₂H, -CDH₂, -CF₃, -CF₂H, -CFH₂, C₁-C₆₀ alkyl group, a C₂-C₆₀ alkenyl group, a C₂-C₆₀ alkynyl group, a C₁-C₆₀ alkoxy group, - Si(Q₃)(Q₄)(Q₅), or -Ge(Q₃)(Q₄)(Q₅); or
a group represented by one of Formulae 9-1 to 9-61, 9-201 to 9-237, 10-1 to 10-129, and 10-201 to 10-350: 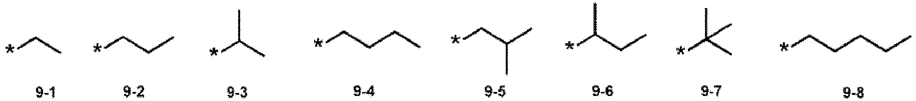 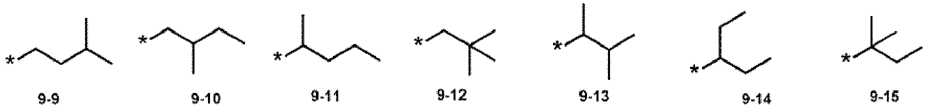 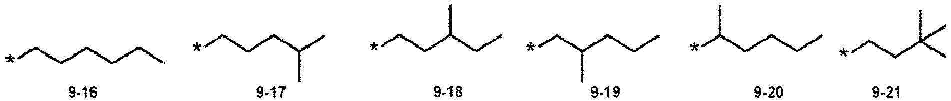 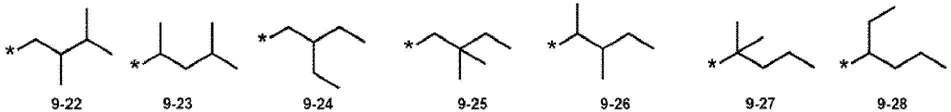 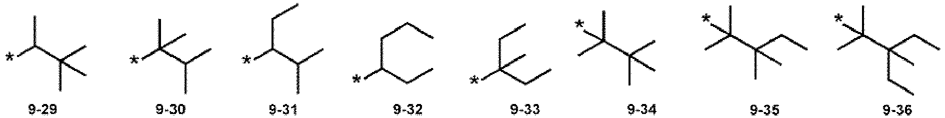 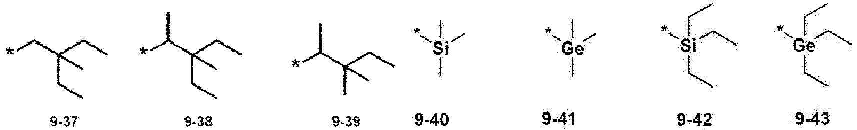 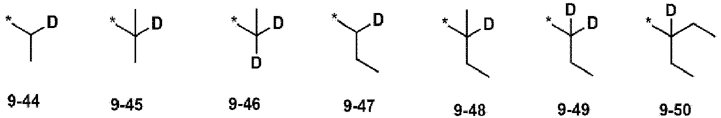 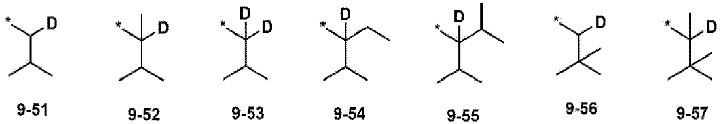 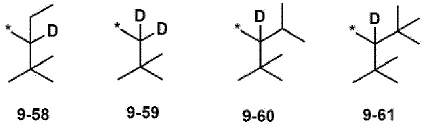 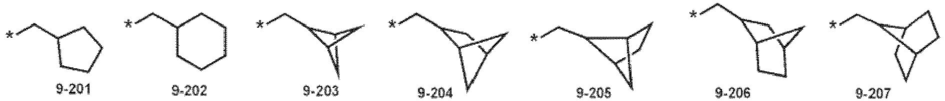 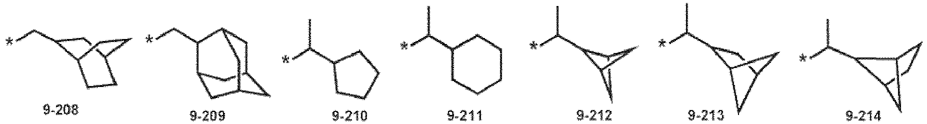 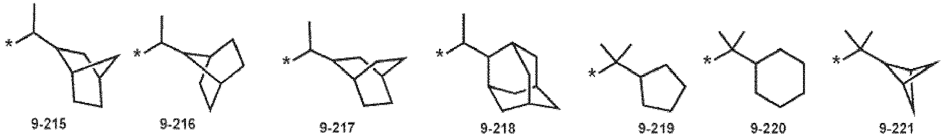 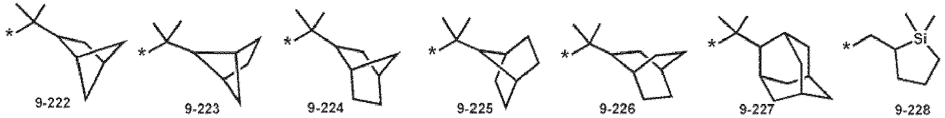 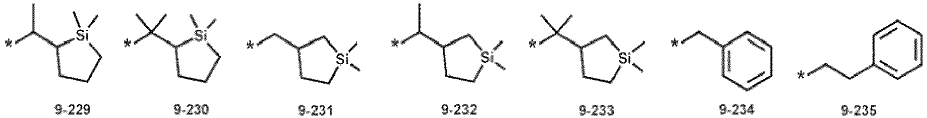 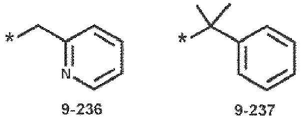 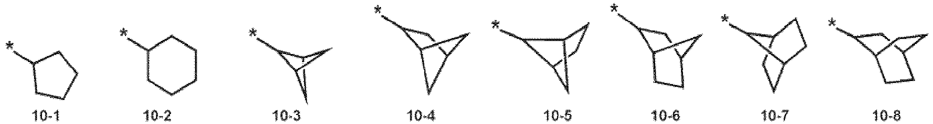 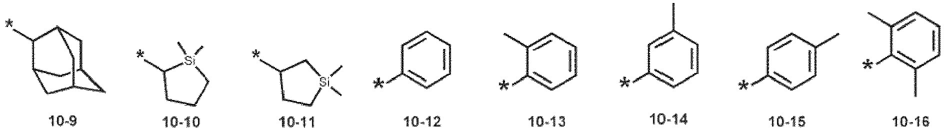 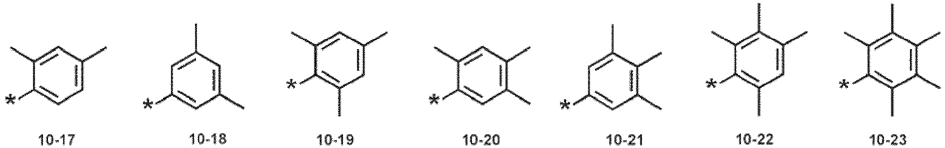 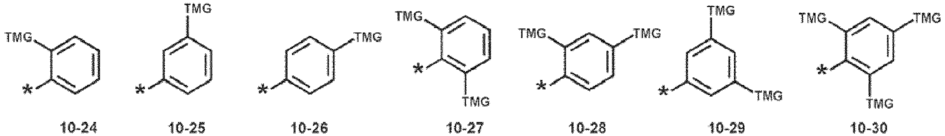 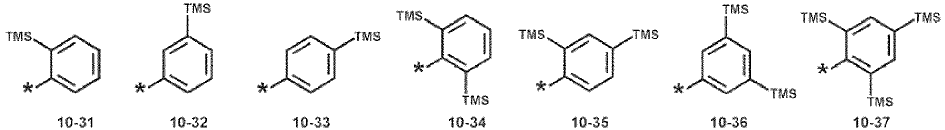 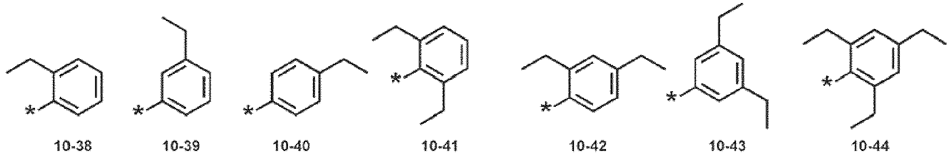 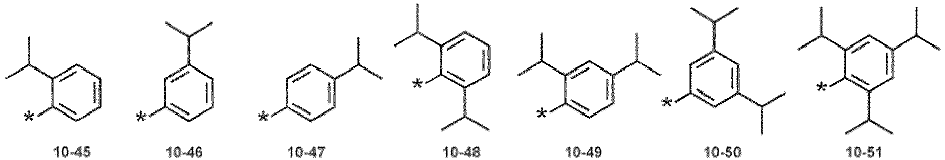 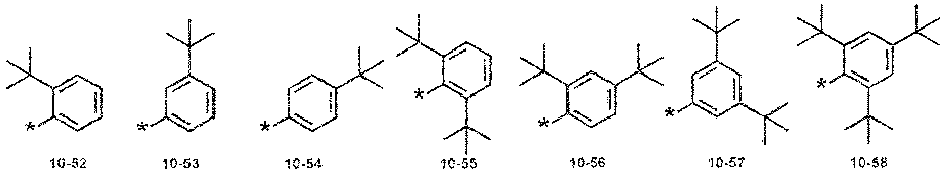 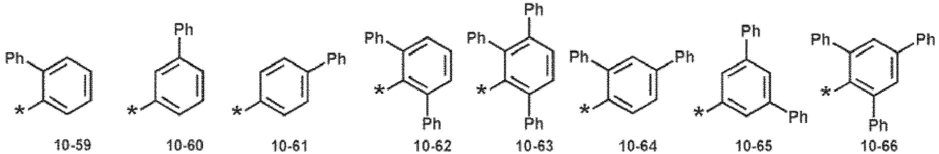 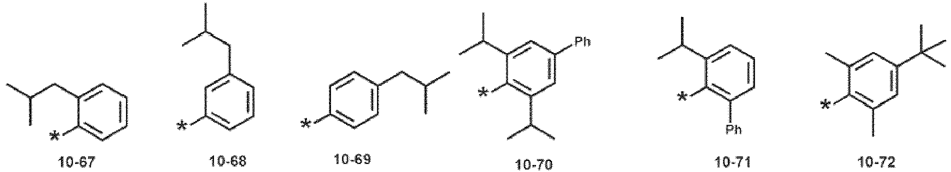 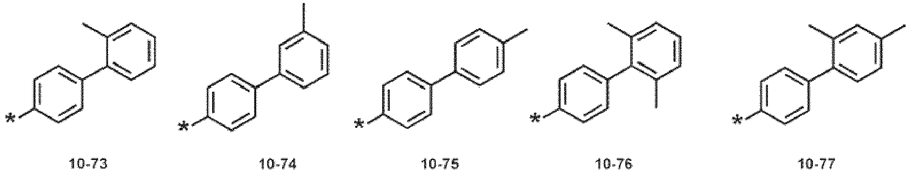 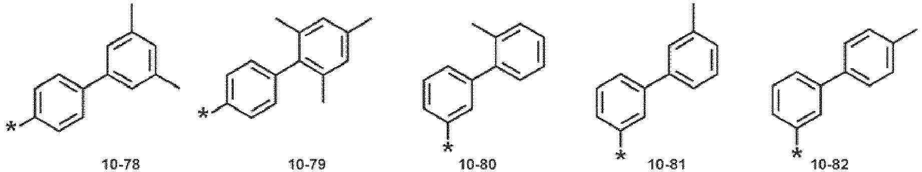 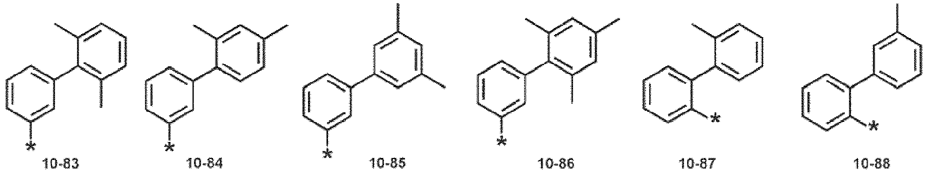 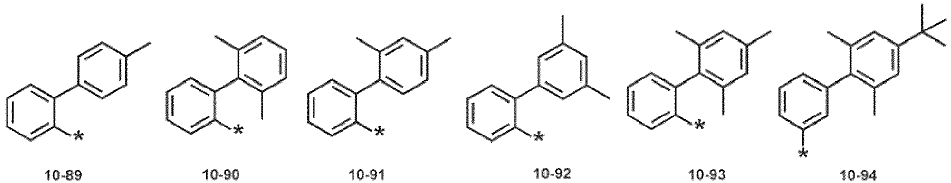 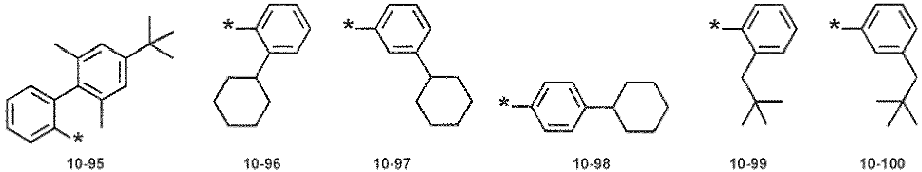 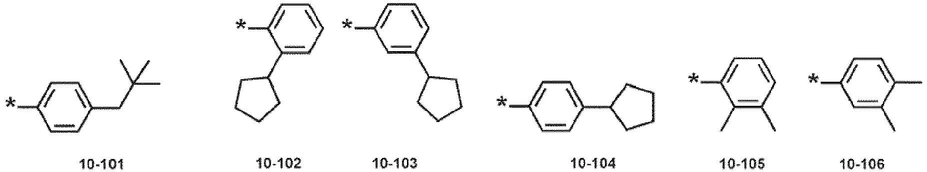 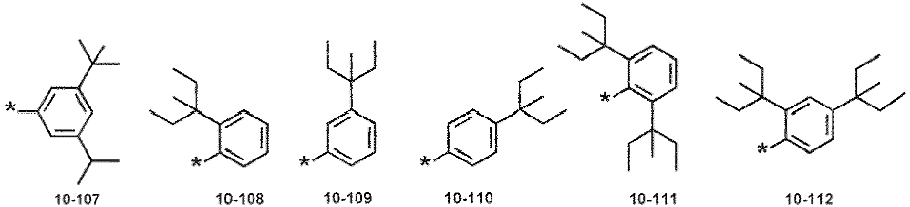 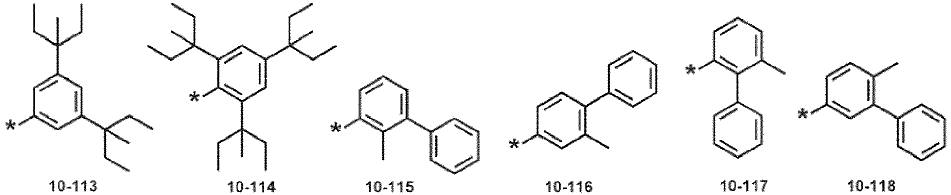 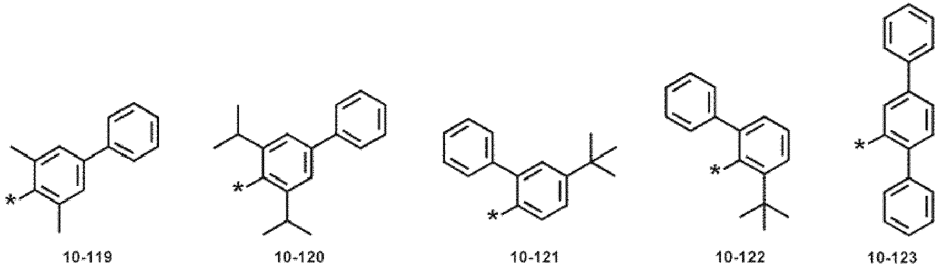 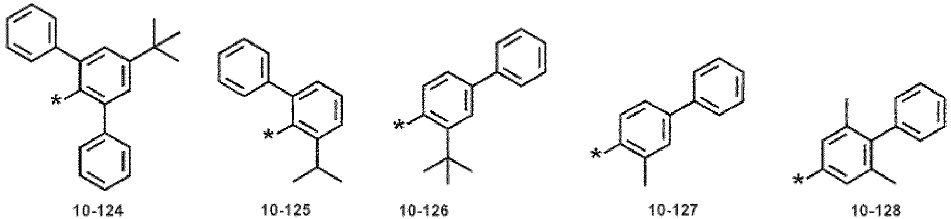 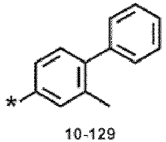 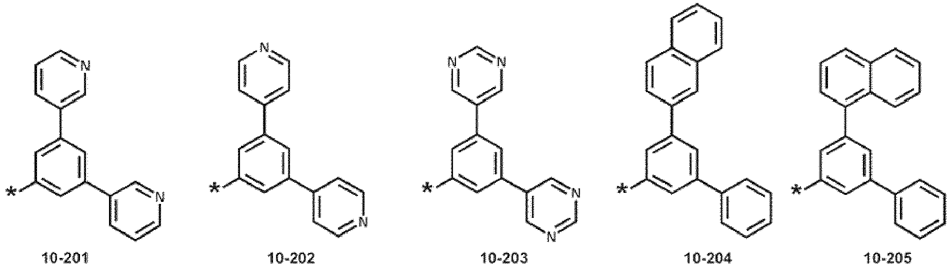 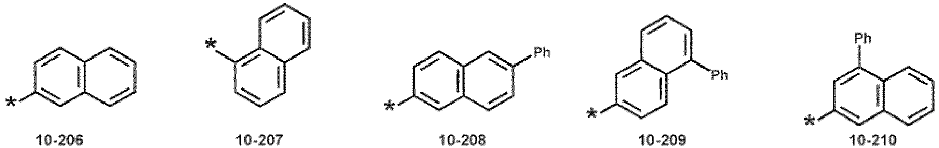 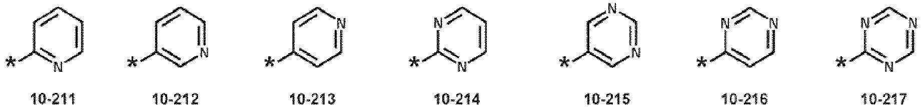 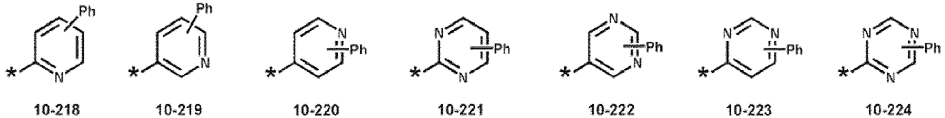 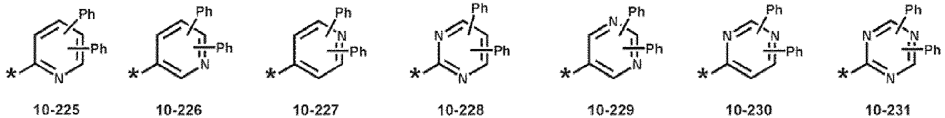 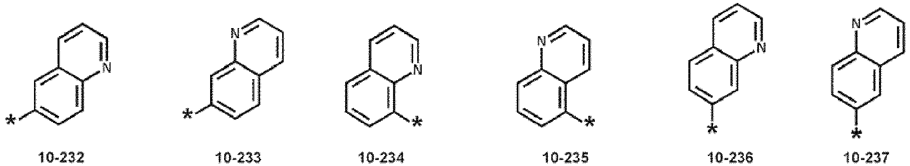 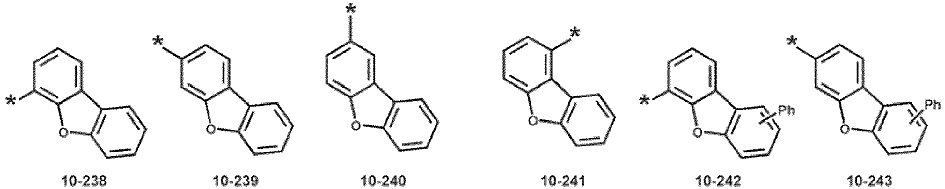 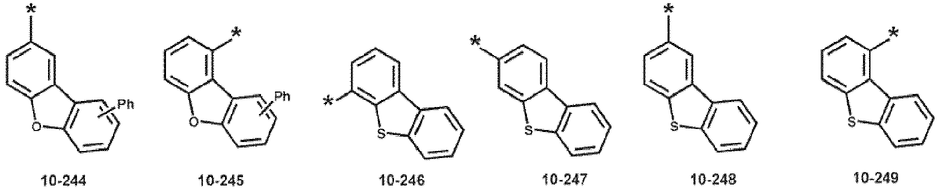 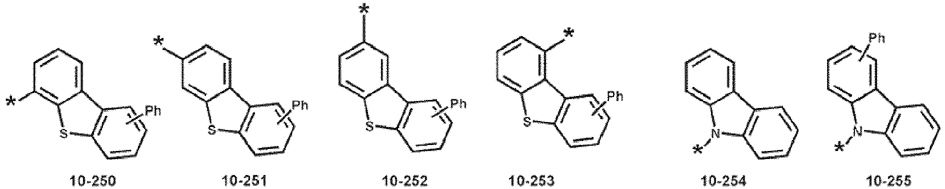 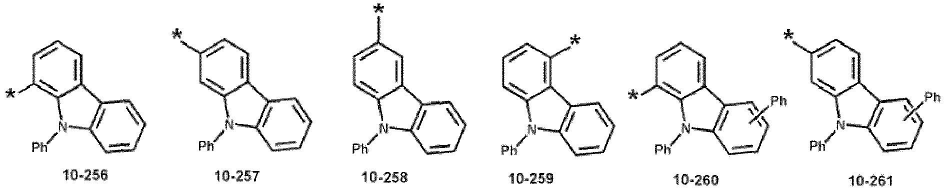 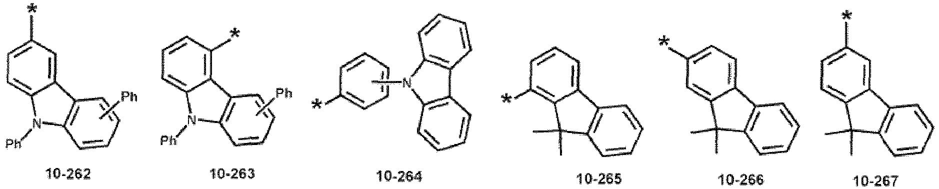 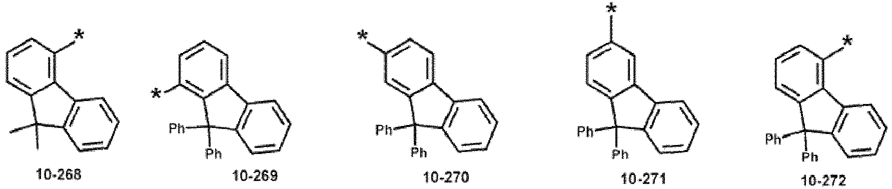 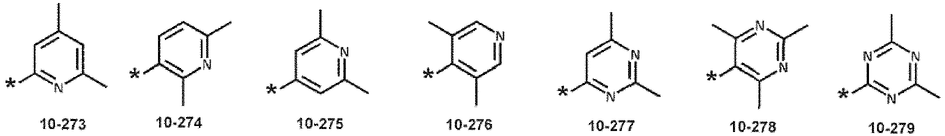 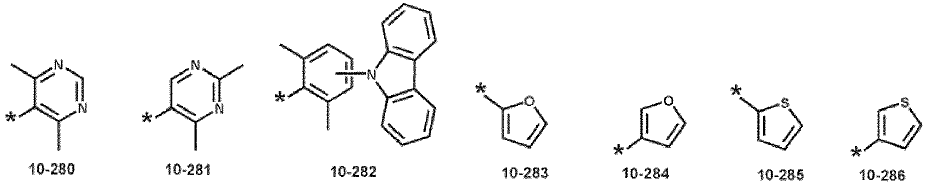 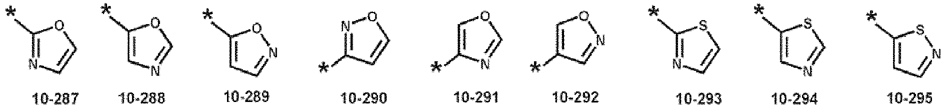 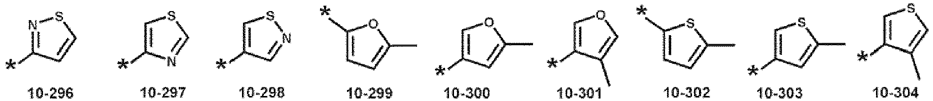 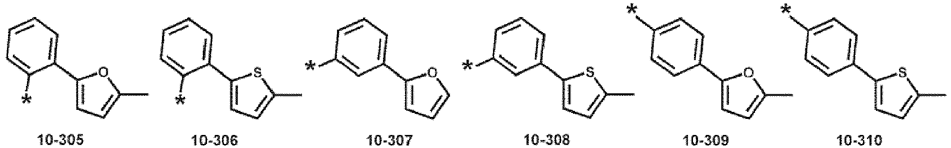 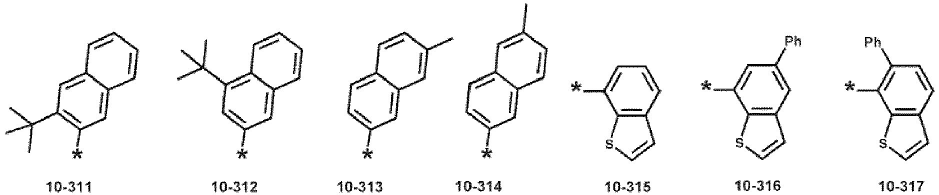 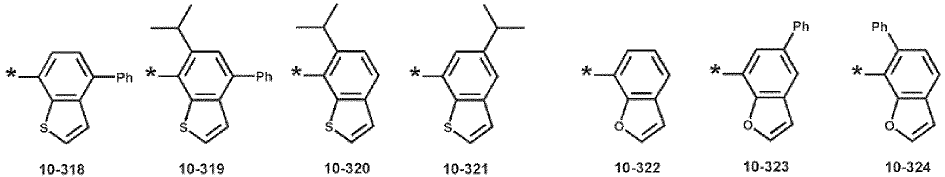 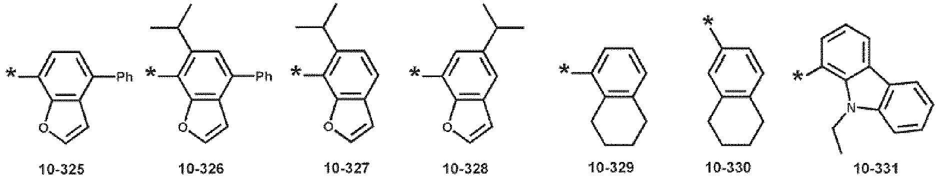 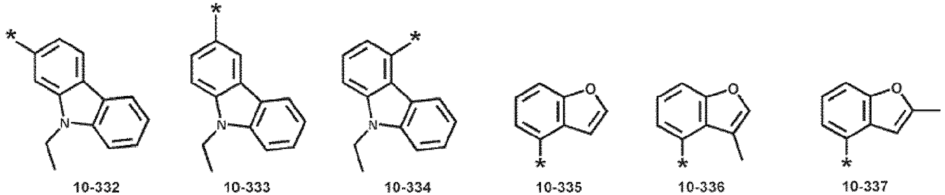 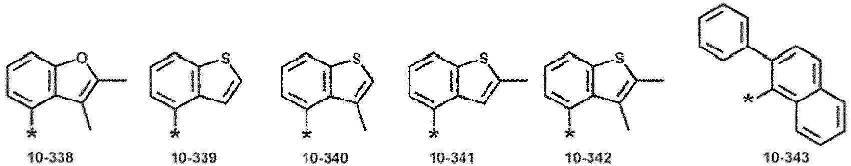 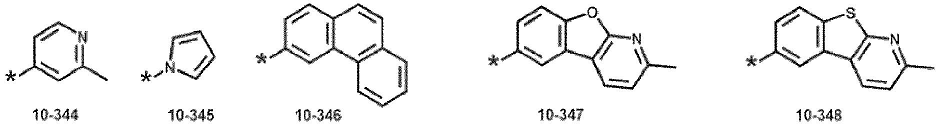 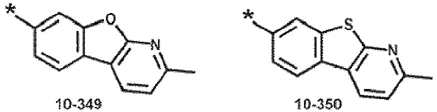
* in Formula 9-1 to 9-61, 9-201 to 9-237, 10-1 to 10-129, and 10-201 to 10-350 indicates a binding site to a neighboring atom, Ph is a phenyl group, TMS is a trimethylsilyl group, and TMG is a trimethylgermyl group.

In an embodiment, Q₁ to Q₉, Q₁₁ to Q₁₉, Q₂₁ to Q₂₉ and Q₃₁ to Q₃₉ described herein may each independently be:
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, or - CD₂CDH₂;
an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group; or
an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group, each substituted with deuterium, a C₁-C₁₀ alkyl group, a phenyl group, or a combination thereof.

In an embodiment, CY₁ in Formula 1-1 may be represented by one of Formulae 1-1 to 1-16: 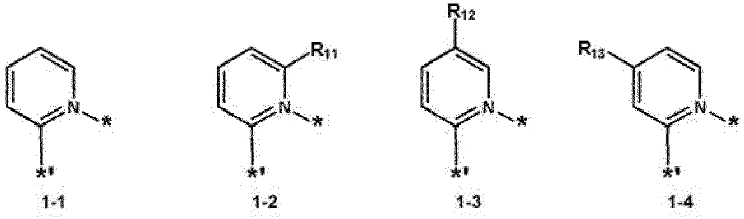 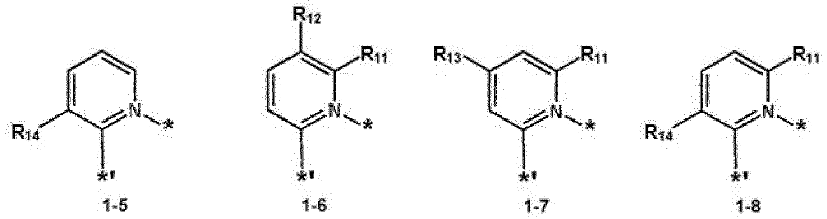 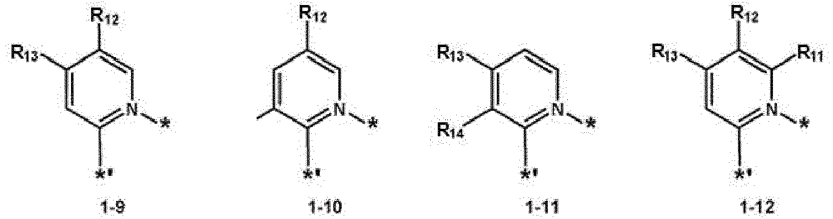 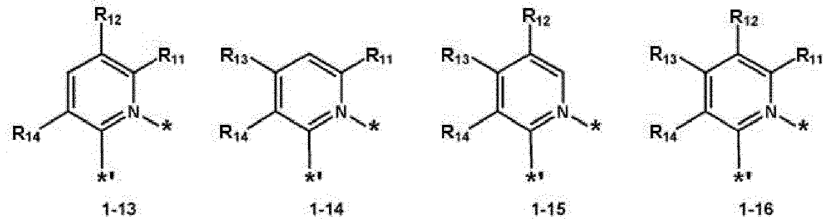 wherein, in Formulae 1-1 to 1-16,
R₁₁ to R₁₄ may each independently be deuterium, -F, -Cl, -Br, -I, -CD₃, -CD₂H, -CDH₂, -CF₃, -CF₂H, -CFH₂, a methyl group, an ethyl group, an n-propyl group, an isopropyl group, an n-butyl group, an isobutyl group, a sec-butyl group, a tert-butyl group, an n-pentyl group, an isopentyl group, a 2-methylbutyl group, a sec-pentyl group, a tert-pentyl group, a neo-pentyl group, 3-pentyl group, 3-methyl-2-butyl group, a phenyl group, a biphenyl group, a naphthyl group, - Si(Q₁)(Q₂)(Q₃), or -Ge(Q₁)(Q₂)(Q₃),
Q₁ to Q₃ may each independently be:
   -CH₃, -CD₃, -CD₂H, -CDH₂, -CH₂CH₃, -CH₂CD₃, -CH₂CD₂H, -CH₂CDH₂, - CHDCH₃, -CHDCD₂H, -CHDCDH₂, -CHDCD₃, -CD₂CD₃, -CD₂CD₂H, or - CD₂CDH₂;
   an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group; or an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group, each substituted with deuterium, a C₁-C₁₀ alkyl group, a phenyl group, or a combination thereof, and
   * and *' each indicate a binding site to a neighboring atom.

In an embodiment, CY₂ in Formula 1-1 may be represented by one of Formulae 2-1 to 2-16: 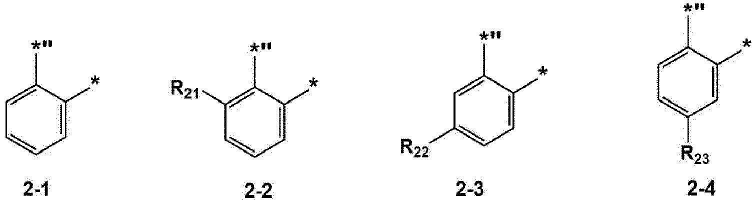 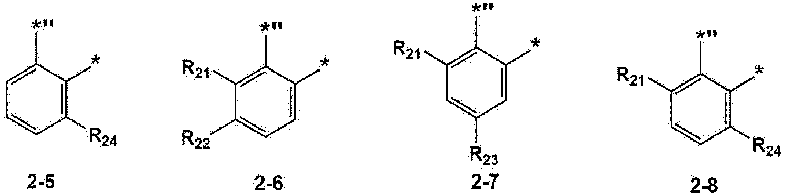 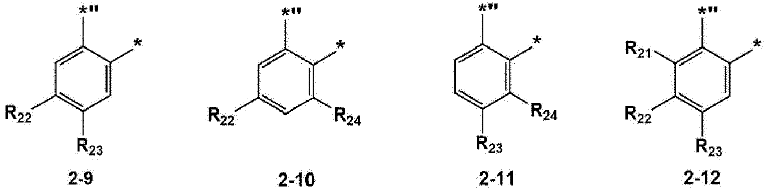 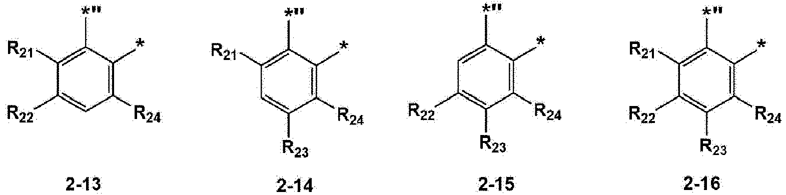 wherein, in Formulae 2-1 to 2-16,
R₂₁ to R₂₄ may each independently be deuterium, -F, -Cl, -Br, -I, -CD₃, -CD₂H, -CDH₂, -CF₃, -CF₂H, -CFH₂, a methyl group, an ethyl group, an n-propyl group, an isopropyl group, an n-butyl group, an isobutyl group, a sec-butyl group, a tert-butyl group, an n-pentyl group, an isopentyl group, a 2-methylbutyl group, a sec-pentyl group, a tert-pentyl group, a neo-pentyl group, 3-pentyl group, 3-methyl-2-butyl group, a phenyl group, a biphenyl group, a naphthyl group, -Si(Q₁)(Q₂)(Q₃), or -Ge(Q₁)(Q₂)(Q₃),
Q₁ to Q₃ may each independently be:
   -CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, or - CD₂CDH₂;
   an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group; or
   an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group, each substituted with deuterium, a C₁-C₁₀ alkyl group, a phenyl group, or a combination thereof, and
   * and *' each indicate a binding site to a neighboring atom.

In an embodiment, CY₃ in Formula 1-2 may be represented by one of Formulae 3-1 to 3-40: 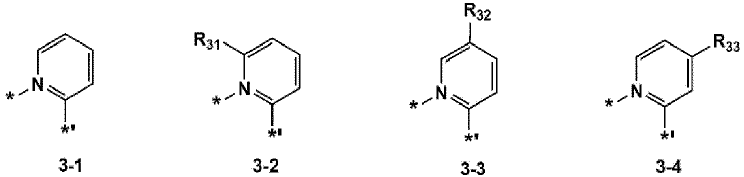 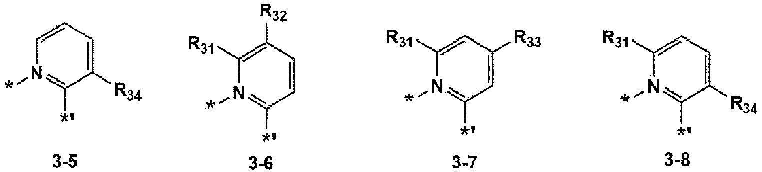 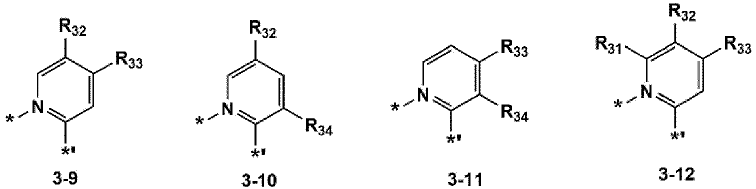 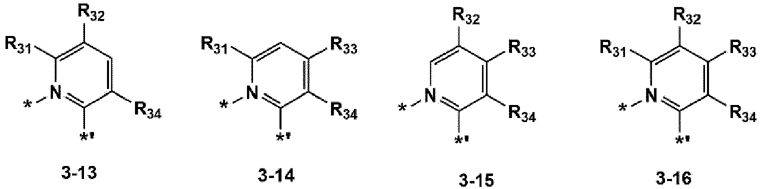 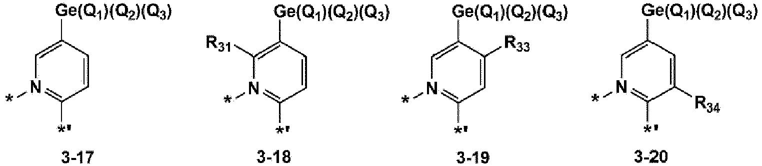 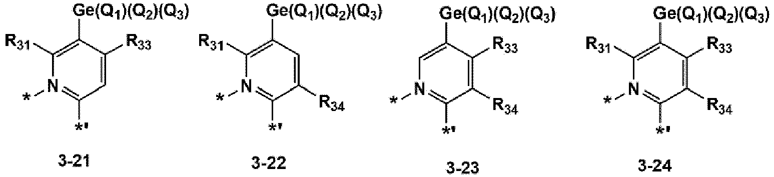 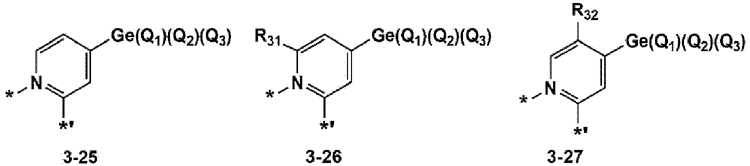 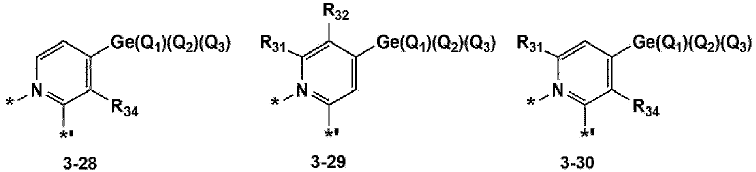 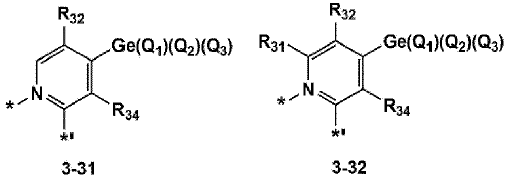 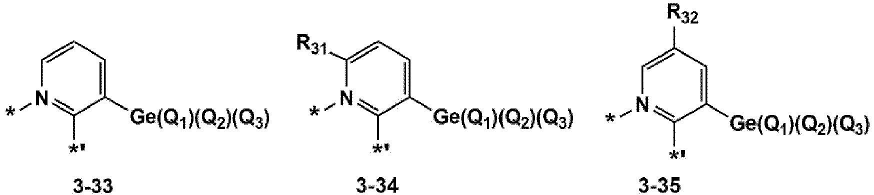 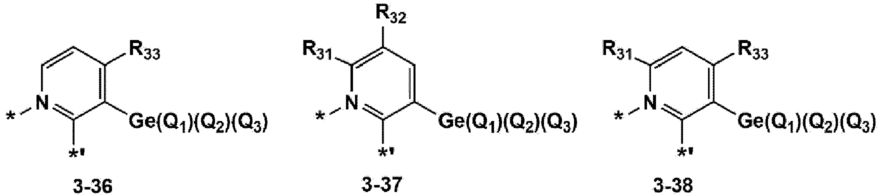 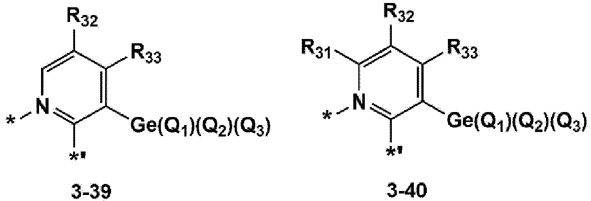 wherein, in Formulae 3-1 to 3-40,
R₃₁ to R₃₄ may each independently be deuterium, -F, -Cl, -Br, -I, -CD₃, -CD₂H, -CDH₂, -CF₃, -CF₂H, -CFH₂, a methyl group, an ethyl group, an n-propyl group, an isopropyl group, an n-butyl group, an isobutyl group, a sec-butyl group, a tert-butyl group, an n-pentyl group, an isopentyl group, a 2-methylbutyl group, a sec-pentyl group, a tert-pentyl group, a neo-pentyl group, 3-pentyl group, 3-methyl-2-butyl group, a phenyl group, a biphenyl group, a naphthyl group, -Si(Q₁)(Q₂)(Q₃), or -Ge(Q₁)(Q₂)(Q₃),
Q₁ to Q₃ may each independently be:
   -CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, or - CD₂CDH₂;
   an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group; or
   an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group, each substituted with deuterium, a C₁-C₁₀ alkyl group, a phenyl group, or a combination thereof, and
   * and *' each indicate a binding site to a neighboring atom.

In an embodiment, the organometallic compound may be a compound represented by Formula 11-1 below:

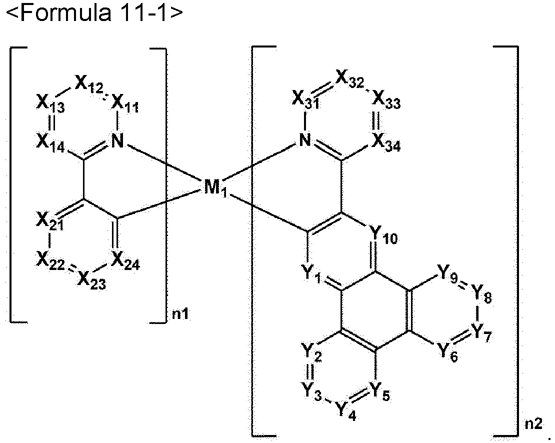

M₁, n1, n2, and Y₁ to Y₁₀ are each the same as described in the present specification,
X₁₁ may be C(R₁₁) or N, X₁₂ may be C(R₁₂) or N, X₁₃ may be C(R₁₃) or N, X₁₄ may be C(R₁₄) or N,
X₂₁ may be C(R₂₁) or N, X₂₂ may be C(R₂₂) or N, X₂₃ may be C(R₂₃) or N, X₂₄ may be C(R₂₄) or N,
X₃₁ may be C(R₃₁) or N, X₃₂ may be C(R₃₂) or N, X₃₃ may be C(R₃₃) or N, X₃₄ may be C(R₃₄) or N,
R₁₁ to R₁₄ are each independently the same as described in connection with R₁₀,
R₂₁ to R₂₄ are each independently the same as described in connection with R₂₀,
R₃₁ to R₃₄ are each independently the same as described in connection with R₃₀,
two or more of R₁₁ to R₁₄ may optionally be linked to each other to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more of R₂₁ to R₂₄ may optionally be linked to each other to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more of R₃₁ to R₃₄ may optionally be linked to each other to form a C₅-C₃₀ carbocyclic group that is unsubstituted or substituted with at least one R₁₀ₐ or a C₁-C₃₀ heterocyclic group that is unsubstituted or substituted with at least one R₁₀ₐ, and
R₁₀ₐ is the same as described in connection with R₁₀.

In an embodiment, at least one of R₁₁ to R₁₄, R₂₁ to R₂₄, R₃₁ to R₃₄ and R₄₁ to R₅₀ in Formula 11-1 may be -Ge(Q₁)(Q₂)(Q₃).

For example, at least one of R₃₁ to R₃₄ and R₄₁ to R₅₀ in Formula 11-1 may be -Ge(Q₁)(Q₂)(Q₃).

In an embodiment, at least one of R₁₁ to R₁₄ and R₂₁ to R₂₄ in Formula 11-1 may be -Ge(Q₁)(Q₂)(Q₃).

In an embodiment, examples of the "C₅-C₃₀ carbocyclic group that is unsubstituted or substituted with at least one R₁₀ₐ or a C₁-C₃₀ heterocyclic group that is unsubstituted or substituted with at least one R₁₀ₐ" include a benzene group, a naphthalene group, a cyclopentane group, a cyclopentadiene group, a cyclohexane group, a cycloheptane group, a bicyclo[2.2.1]heptane group, a furan group, a thiophene group, a pyrrole group, a silole group, an indene group, a benzofuran group, a benzothiophene group, an indole group, or a benzosilole group, each substituted or unsubstituted with at least one R₁₀ₐ. R₁₀ₐ is the same as described in connection with R₁₀. The C₅-C₃₀ carbocyclic group and the C₁-C₃₀ heterocyclic group are each the same as described in the present specification.

In an embodiment, the organometallic compound may be a compound represented by one of Formulae 12-1 to 12-7: 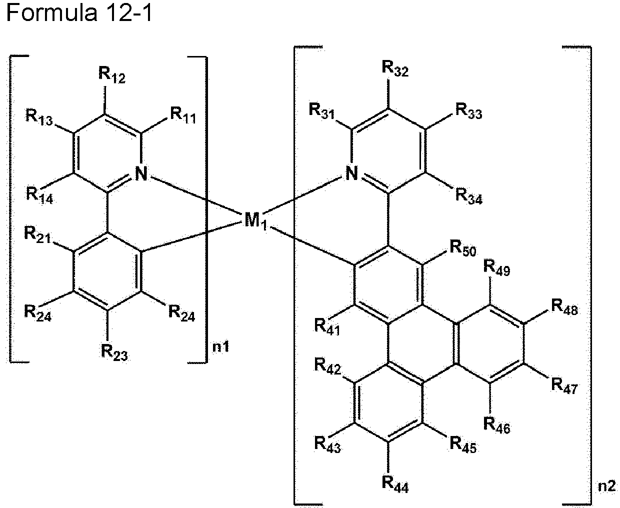 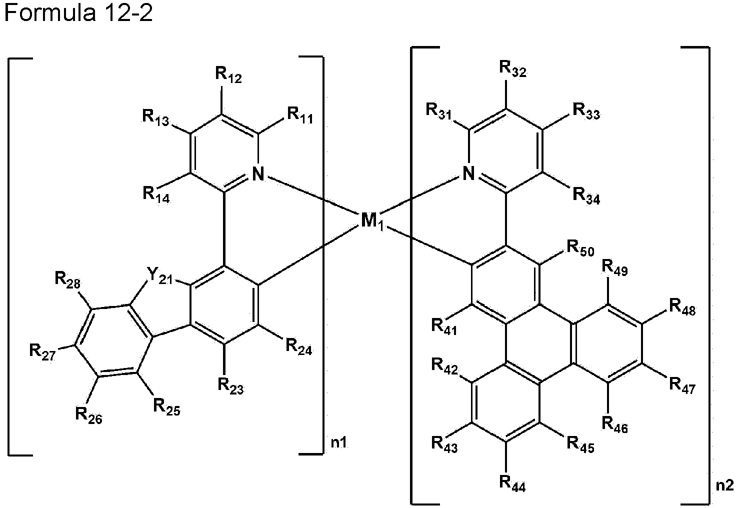 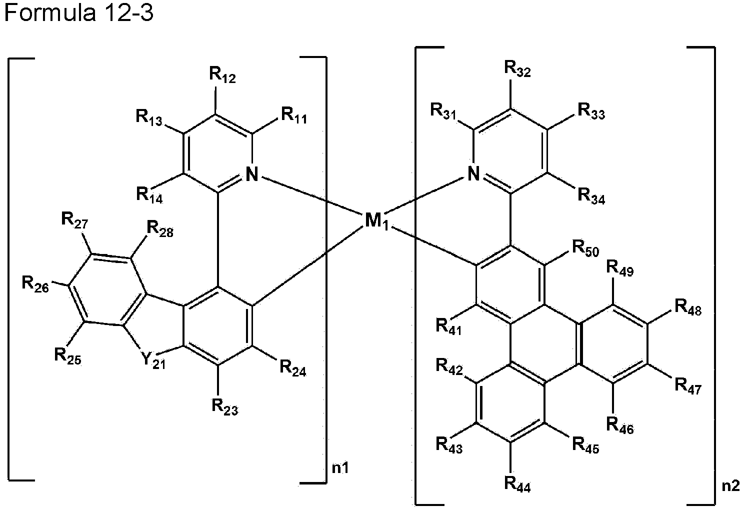 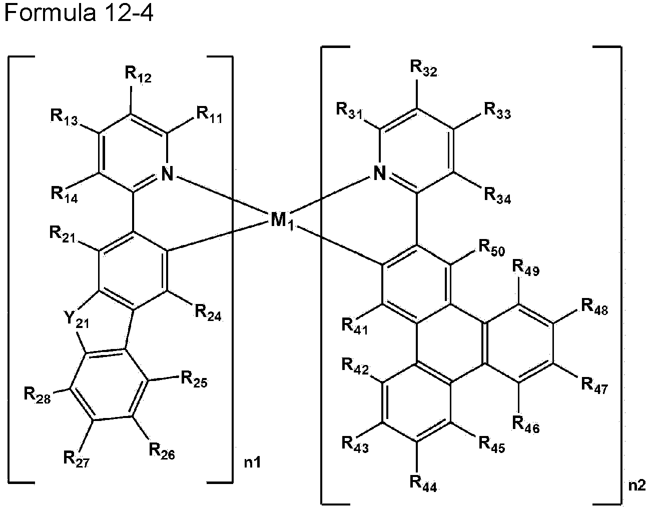 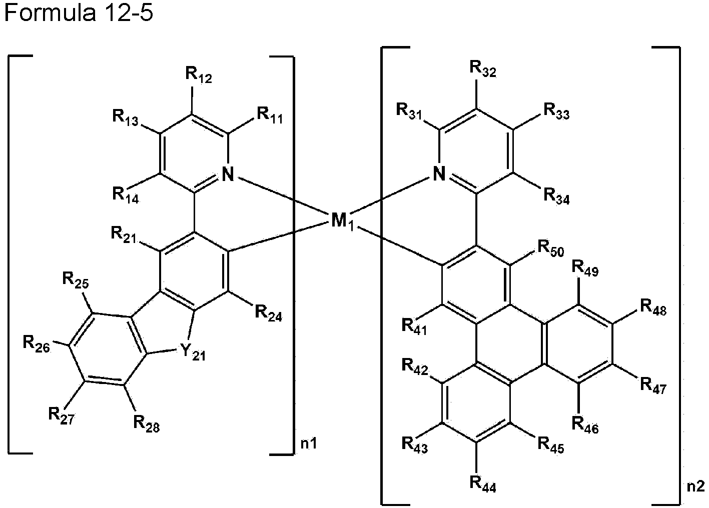 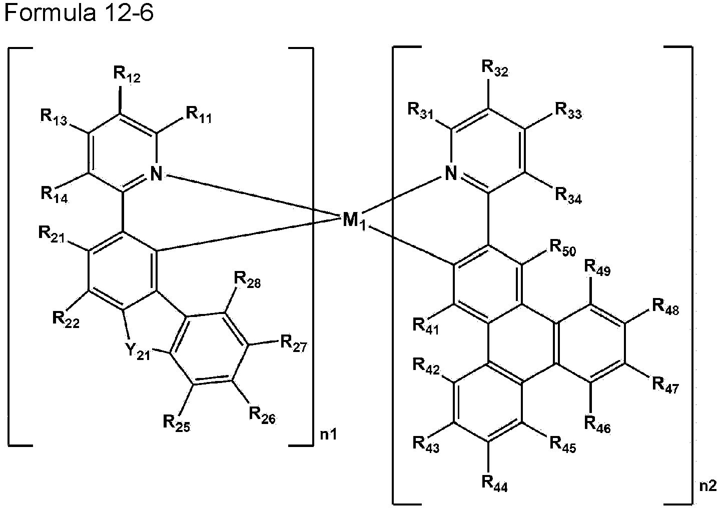 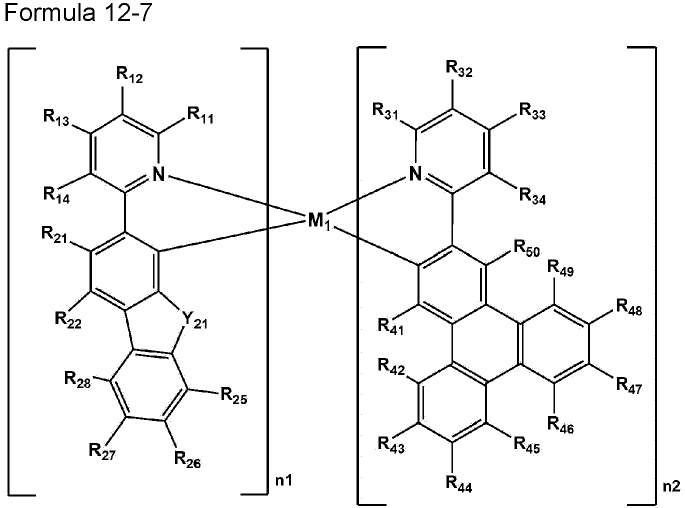 wherein, in Formulae 12-1 to 12-7,
M₁, n1, n2, and R₄₁ to R₅₀ are the same as described in the present specification,
Y₂₁ may be O, S, N(R_{29A}), C(R_{29A})(R_{29B}), or Si(R_{29A})(R_{29B}),
R₁₁ to R₁₄ are each independently the same as described in connection with R₁₀,
R₂₁ to R₂₈, R_{29A}, and R_{29B} are each independently the same as described in connection with R₂₀, and
R₃₁ to R₃₄ are each independently the same as described in connection with R₃₀.

In an embodiment, in Formulae 12-1 to 12-7,
two or more of R₁₁ to R₁₄ may optionally be linked to each other to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more of R₂₁ to R₂₈, R_{29A}, and R_{29B} may optionally be linked to each other to form a C₅-C₃₀ carbocyclic group that is unsubstituted or substituted with at least one R₁₀ₐ or a C₁-C₃₀ heterocyclic group that is unsubstituted or substituted with at least one R₁₀ₐ, and
two or more of R₃₁ to R₃₄ may optionally be linked to each other to form a C₅-C₃₀ carbocyclic group that is unsubstituted or substituted with at least one R₁₀ₐ or a C₁-C₃₀ heterocyclic group that is unsubstituted or substituted with at least one R₁₀ₐ

R₁₀ₐ is the same as described in the present specification.

Two or more neighboring groups among R₁₁ to R₁₄, R₂₁ to R₂₈, R_{29A}, R_{29B}, and R₃₁ to R₃₄ may optionally be linked together to form a benzene group or a naphthalene group.

In an embodiment, at least one of R₃₀(s) in the number of b30 may be - Ge(Q₁)(Q₂)(Q₃).

In an embodiment, at least one of R₄₁ to R₅₀ may be -Ge(Q₁)(Q₂)(Q₃).

In an embodiment, at least one of R₁₀ (s) in the number of b10, R₂₀(s) in the number of b20, and R₃₀(s) in the number of b30, and R₄₁ to R₅₀ may be a substituted or unsubstituted C₁-C₆₀ alkyl group, a substituted or unsubstituted C₃-C₁₀ cycloalkyl group, -Si(Q₁)(Q₂)(Q₃), or -Ge(Q₁)(Q₂)(Q₃).

In an embodiment, at least one of R₁₀ (s) in the number of b10, R₂₀(s) in the number of b20, and R₃₀(s) in the number of b30, and R₄₁ to R₅₀ may be a methyl group, an ethyl group, an n-propyl group, an isopropyl group, an n-butyl group, an isobutyl group, a sec-butyl group, a tert-butyl group, an n-pentyl, an isopentyl, a 2-methylbutyl group, a sec-pentyl, a tert-pentyl, a neo-pentyl, a 3-pentyl, a 3-methyl-2-butyl group, a cyclopropyl group, a cyclobutyl group, a cyclopentyl, a cyclohexyl group, a cycloheptyl group, a cyclooctyl group, a phenyl group, a biphenyl group, a C₁-C₂₀ alkylphenyl group, a naphthyl group, -Si(Q₁)(Q₂)(Q₃), or -Ge(Q₁)(Q₂)(Q₃), each unsubstituted or substituted with deuterium.

In an embodiment, at least one of R₃₁ to R₃₄ and R₄₁ to R₅₀ in Formulae 11-1 or Formula 12-1 to 12-7 may be a substituted or unsubstituted C₁-C₆₀ alkyl group, a substituted or unsubstituted C₃-C₁₀ cycloalkyl group, -Si(Q₁)(Q₂)(Q₃), or - Ge(Q₁)(Q₂)(Q₃).

In an embodiment, at least one of R₃₁ to R₃₄ and R₄₁ to R₅₀ in Formulae 11-1 or Formula 12-1 to 12-7 may be a methyl group, an ethyl group, an n-propyl group, an isopropyl group, an n-butyl group, an isobutyl group, a sec-butyl group, a tert-butyl group, an n-pentyl, an isopentyl, a 2-methylbutyl group, a sec-pentyl, a tert-pentyl, a neo-pentyl, a 3-pentyl, a 3-methyl-2-butyl group, a cyclopropyl group, a cyclobutyl group, a cyclopentyl, a cyclohexyl group, a cycloheptyl group, a cyclooctyl group, a phenyl group, a biphenyl group, a C₁-C₂₀ alkylphenyl group, a naphthyl group, - Si(Q₁)(Q₂)(Q₃), or -Ge(Q₁)(Q₂)(Q₃).

In an embodiment, at least one of R₃₁ to R₃₄ in Formulae 3-1 to 3-40 may be a substituted or unsubstituted C₁-C₆₀ alkyl group, a substituted or unsubstituted C₃-C₁₀ cycloalkyl group, -Si(Q₁)(Q₂)(Q₃), or -Ge(Q₁)(Q₂)(Q₃).

In an embodiment, at least one of R₃₁ to R₃₄ in Formulae 3-1 to 3-40 may be a methyl group, an ethyl group, an n-propyl group, an isopropyl group, an n-butyl group, an isobutyl group, a sec-butyl group, a tert-butyl group, an n-pentyl group, an isopentyl group, a 2-methylbutyl group, a sec-pentyl group, a tert-pentyl group, a neo-pentyl group, a 3-pentyl group, a 3-methyl-2-butyl group, a phenyl group, a biphenyl group, a C₁-C₂₀ alkylphenyl group, a naphthyl group, -Si(Q₁)(Q₂)(Q₃), or -Ge(Q₁)(Q₂)(Q₃).

In an embodiment, the organometallic compound may include one germanyl group or two, three, or four germanyl groups. For example, the organometallic compound may include one to four -Ge(Q₁)(Q₂)(Q₃).

For example, the organometallic compound may include one germanyl group or two germanyl groups. For example, the organometallic compound may include one or two -Ge(Q₁)(Q₂)(Q₃).

In an embodiment, the organometallic compound may be one of Compounds 1 to 36:

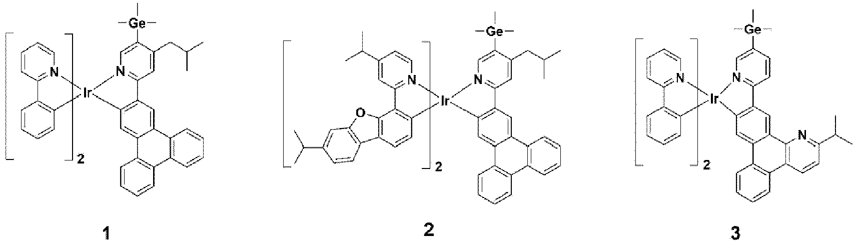

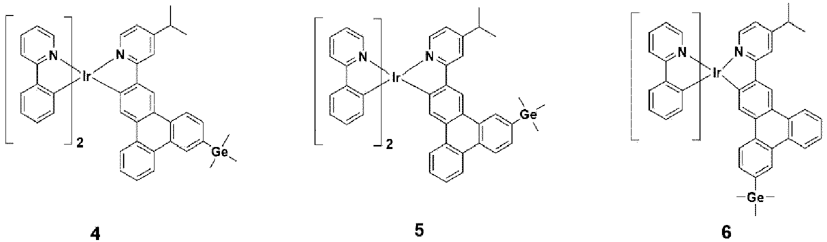

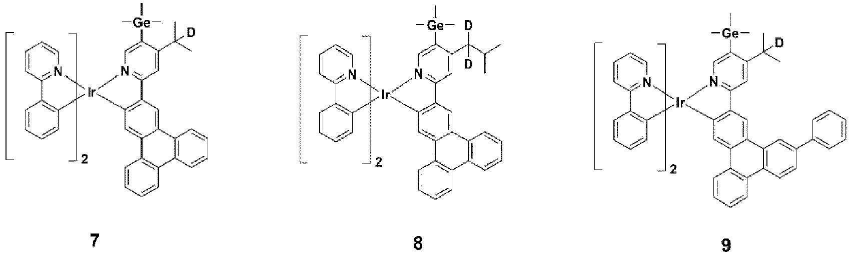

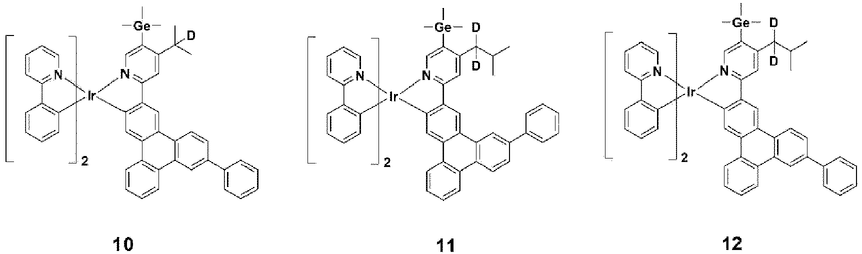

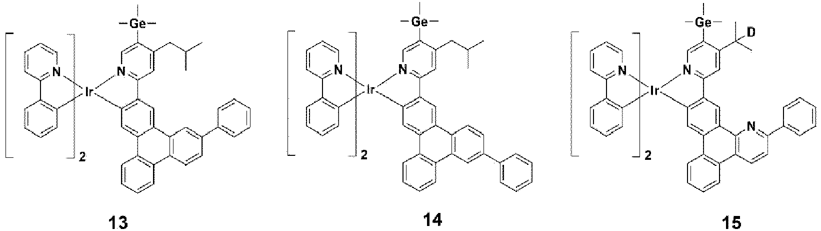

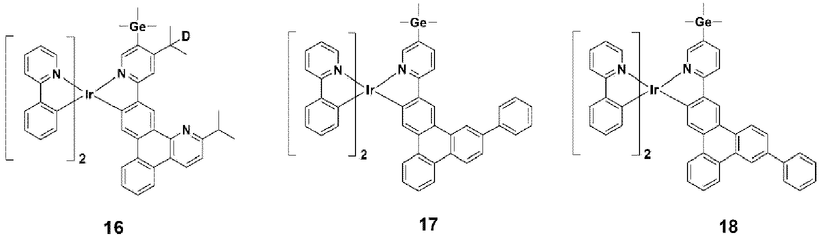

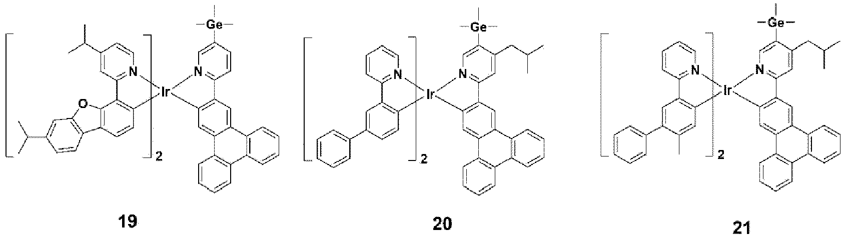

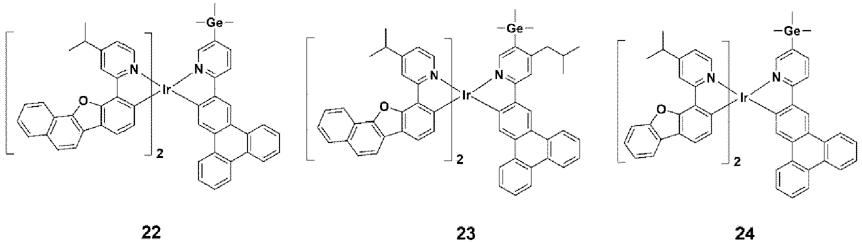

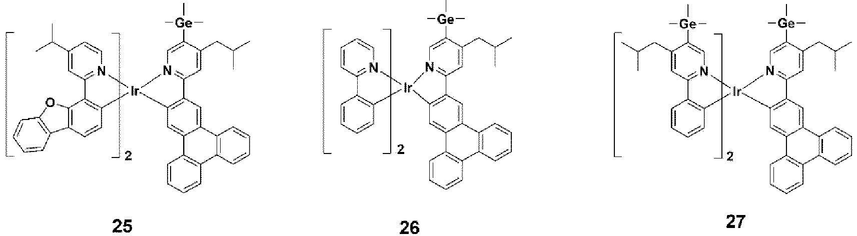

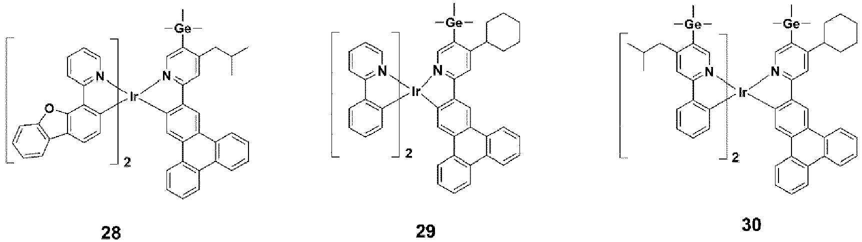

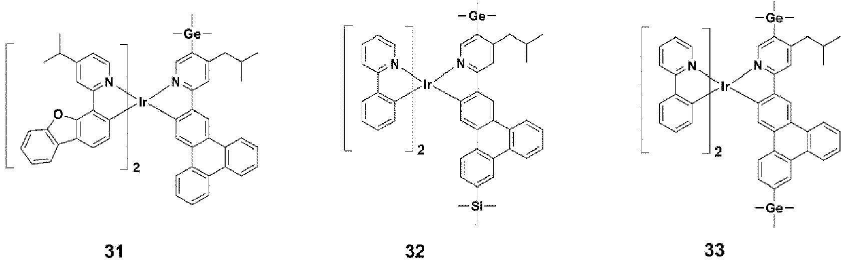

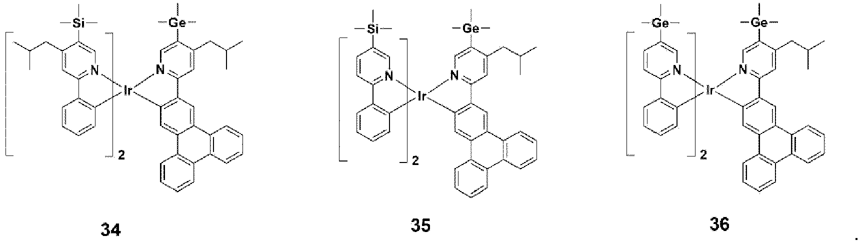

In an embodiment, the organometallic compound may be electrically neutral.

The organometallic compound represented by Formula 1 satisfies the structure of Formula 1 and has at least one germanyl group which is substituted in a bidentate ligand including triphenylene. Due to this structure, the organometallic compound represented by Formula 1 has excellent luminescence characteristics, and has such characteristics suitable for use as a luminescent material with high color purity by controlling the emission wavelength range.

In addition, the organometallic compound represented by Formula 1 has excellent electrical mobility, and thus, electronic devices including the organometallic compound, for example, organic light-emitting devices including the organometallic compound may show low driving voltage, high efficiency, a long lifespan, and reduced roll-off phenomenon.

In addition, the photochemically stability of the organometallic compound represented by Formula 1 is improved, and thus, electronic devices including the organometallic compound, for example, organic light-emitting devices including the organometallic compound may show high luminescence efficiency, long lifespan, and high color purity.

The highest occupied molecular orbital (HOMO) energy level, lowest unoccupied molecular orbital (LUMO) energy level, energy gap, S₁ energy level, and T₁ energy level of some compounds of the organometallic compound represented by Formula 1 were evaluated using the Gaussian 09 program with the molecular structure optimization obtained by B3LYP-based density functional theory (DFT), and results thereof are shown in Table 1.

**Table 1**

| Compound No. | HOMO (eV) | LUMO (eV) | Energy gap (eV) | S₁ (eV) | T₁ (eV) |
|---|---|---|---|---|---|
| Compound 1 | -4.794 | -1.345 | 3.449 | 2.794 | 2.497 |
| Compound 2 | -4.758 | -1.356 | 3.402 | 2.771 | 2.500 |
| Compound 3 | -4.827 | -1.340 | 3.487 | 2.818 | 2.522 |
| Compound A | -4.805 | -1.388 | 3.417 | 2.763 | 2.477 |
| Compound B | -4.776 | -1.290 | 3.485 | 2.840 | 2.520 |
| Compound C | -4.810 | -1.350 | 3.461 | 2.798 | 2.496 |

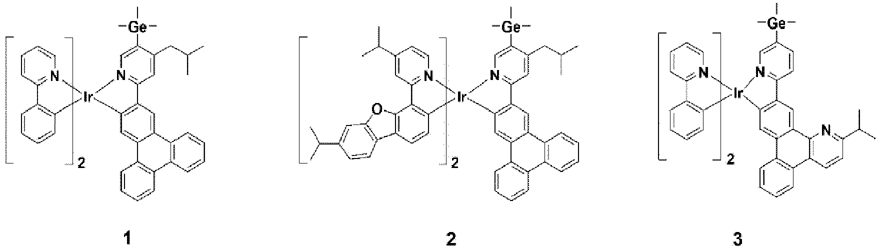

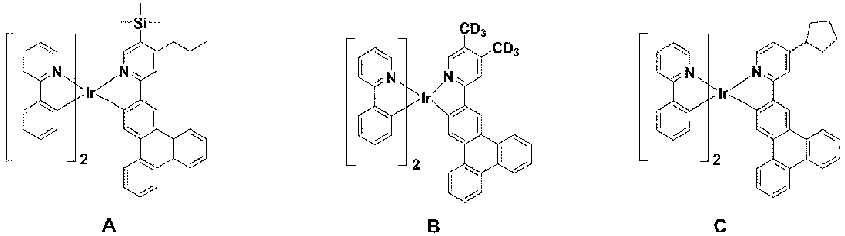

From Table 1, it is confirmed that the organometallic compound represented by Formula 1 has such electric characteristics that are suitable for use as a dopant for an electric device, for example, an organic light-emitting device.

In an embodiment, the full width at half maximum (FWHM) of the emission peak of the emission spectrum or the electroluminescence spectrum of the organometallic compound may be about 70 nm or less. For example, the FWHM of the emission peak of the emission spectrum or the electroluminescence spectrum of the organometallic compound may be from about 30 nm to about 65 nm, from about 40 nm to about 63 nm, or from about 45 nm to about 62 nm.

In an embodiment, the maximum emission wavelength (emission peak wavelength, λₘₐₓ) of the emission peak of the emission spectrum or electroluminescence spectrum of the organometallic compound may be from about 500 nm to about 600 nm.

Synthesis methods of the organometallic compound represented by Formula 1 may be recognizable by one of ordinary skill in the art by referring to Synthesis Examples provided below.

The organometallic compound represented by Formula 1 is suitable for use in an organic layer of an organic light-emitting device, for example, for use as a dopant in an emission layer of the organic layer. Thus, another aspect provides an organic light-emitting device that includes: a first electrode; a second electrode; and an organic layer that is located between the first electrode and the second electrode and includes an organic layer including an emission layer and at least one of the organometallic compound represented by Formula 1.

As described above, due to the inclusion of the organic layer including the organometallic compound represented by Formula 1, the organic light-emitting device may have excellent characteristics in terms of driving voltage, current efficiency, power efficiency, external quantum efficiency, lifespan, and/or color purity. Also, such an organic light-emitting device may have a reduced roll-off phenomenon and a relatively narrow electroluminescent (EL) spectrum emission peak FWHM.

The organometallic compound of Formula 1 may be used between a pair of electrodes of an organic light-emitting device. For example, the organometallic compound represented by Formula 1 may be included in the emission layer. In this regard, the organometallic compound may act as a dopant, and the emission layer may further include a host (that is, an amount of the organometallic compound represented by Formula 1 in the emission layer is smaller than an amount of the host).

In an embodiment, the emission layer may emit green light. For example, the emission layer may emit green light having a maximum emission wavelength of about 500 nm to about 600 nm.

The expression "(an organic layer) includes at least one organometallic compounds" used herein may include a case in which "(an organic layer) includes identical organometallic compounds represented by Formula 1" and a case in which "(an organic layer) includes two or more different organometallic compounds represented by Formula 1."

For example, the organic layer may include, as the organometallic compound, only Compound 1. In this embodiment, Compound 1 may be included in the emission layer of the organic light-emitting device. In one or more embodiments, the organic layer may include, as the organometallic compound, Compound 1 and Compound 2. In this regard, Compound 1 and Compound 2 may exist in an identical layer (for example, Compound 1 and Compound 2 all may exist in an emission layer).

The first electrode may be an anode, which is a hole injection electrode, and the second electrode may be a cathode, which is an electron injection electrode; or the first electrode may be a cathode, which is an electron injection electrode, and the second electrode may be an anode, which is a hole injection electrode.

In an embodiment, in the organic light-emitting device, the first electrode is an anode, and the second electrode is a cathode, and the organic layer may further include a hole transport region located between the first electrode and the emission layer and an electron transport region located between the emission layer and the second electrode, and the hole transport region may include a hole injection layer, a hole transport layer, an electron blocking layer, a buffer layer, or a combination thereof, and the electron transport region may include a hole blocking layer, an electron transport layer, an electron injection layer, or a combination thereof.

The term "organic layer" used herein refers to a single layer and/or a plurality of layers located between the first electrode and the second electrode of the organic light-emitting device. The "organic layer" may include, in addition to an organic compound, an organometallic complex including metal.

FIGURE is a schematic cross-sectional view of an organic light-emitting device 10 according to an embodiment. Hereinafter, the structure of an organic light-emitting device according to an embodiment of the present disclosure and a method of manufacturing an organic light-emitting device according to an embodiment of the present disclosure will be described in connection with FIGURE. The organic light-emitting device 10 includes a first electrode 11, an organic layer 15, and a second electrode 19, which are sequentially stacked.

A substrate may be additionally located under the first electrode 11 or above the second electrode 19. For use as the substrate, any substrate that is used in organic light-emitting devices available in the art may be used, and the substrate may be a glass substrate or a transparent plastic substrate, each having excellent mechanical strength, thermal stability, transparency, surface smoothness, ease of handling, and water resistance.

In one or more embodiments, the first electrode 11 may be formed by depositing or sputtering a material for forming the first electrode 11 on the substrate. The first electrode 11 may be an anode. The material for forming the first electrode 11 may be selected from materials with a high work function to facilitate hole injection. The first electrode 11 may be a reflective electrode, a semi-transmissive electrode, or a transmissive electrode. The material for forming the first electrode 11 may be indium tin oxide (ITO), indium zinc oxide (IZO), tin oxide (SnO₂), or zinc oxide (ZnO). In one or more embodiments, the material for forming the first electrode 11 may be metal, such as magnesium (Mg), aluminum (Al), aluminum-lithium (Al-Li), calcium (Ca), magnesium-indium (Mg-In), or magnesium-silver (Mg-Ag).

The first electrode 11 may have a single-layered structure or a multi-layered structure including two or more layers. For example, the first electrode 11 may have a three-layered structure of ITO/Ag/ITO, but the structure of the first electrode 11 is not limited thereto.

The organic layer 15 is located on the first electrode 11.

The organic layer 15 may include a hole transport region, an emission layer, and an electron transport region.

The hole transport region may be between the first electrode 11 and the emission layer.

The hole transport region may include a hole injection layer, a hole transport layer, an electron blocking layer, a buffer layer, or a combination thereof.

The hole transport region may include only either a hole injection layer or a hole transport layer. In one or more embodiments, the hole transport region may have a hole injection layer/hole transport layer structure or a hole injection layer/hole transport layer/electron blocking layer structure, wherein, for each structure, each layer is sequentially stacked in this stated order from the first electrode 11.

When the hole transport region includes a hole injection layer (HIL), the hole injection layer may be formed on the first electrode 11 by using one or more suitable methods, for example, vacuum deposition, spin coating, casting, and/or Langmuir-Blodgett (LB) deposition.

When a hole injection layer is formed by vacuum deposition, the deposition conditions may vary according to a material that is used to form the hole injection layer, and the structure and thermal characteristics of the hole injection layer. For example, the deposition conditions may include a deposition temperature of about 100 °C to about 500 °C, a vacuum pressure of about 10⁻⁸ torr to about 10⁻³ torr, and a deposition rate of about 0.01 Å/sec to about 100 Å/sec. However, the deposition conditions are not limited thereto.

When the hole injection layer is formed using spin coating, coating conditions may vary according to the material used to form the hole injection layer, and the structure and thermal properties of the hole injection layer. For example, a coating speed may be from about 2,000 rpm to about 5,000 rpm, and a temperature at which a heat treatment is performed to remove a solvent after coating may be from about 80 °C to about 200 °C. However, the coating conditions are not limited thereto.

Conditions for forming a hole transport layer and an electron blocking layer may be understood by referring to conditions for forming the hole injection layer.

The hole transport region may include at least one of m-MTDATA, TDATA, 2-TNATA, NPB, β-NPB, TPD, Spiro-TPD, Spiro-NPB, methylated NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), a compound represented by Formula 201 below, a compound represented by Formula 202 below, or a combination thereof:

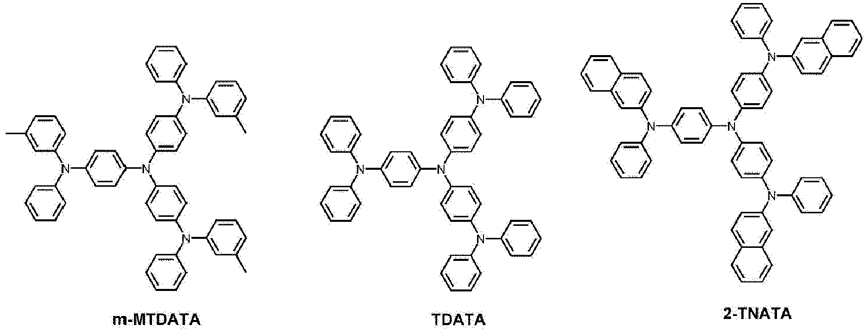

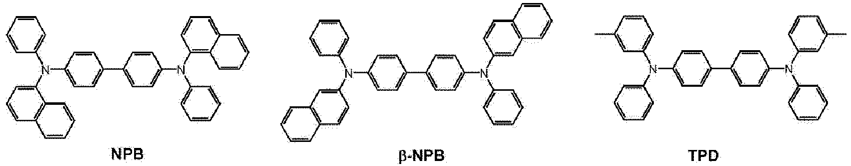

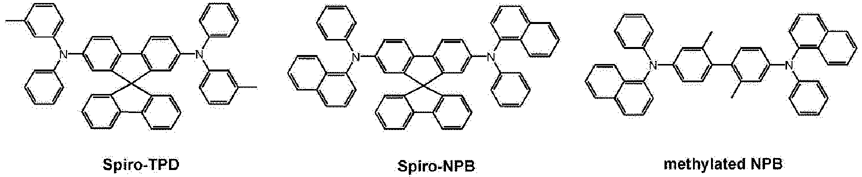

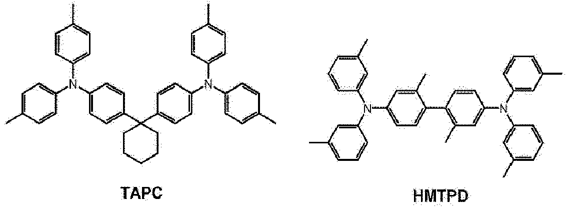

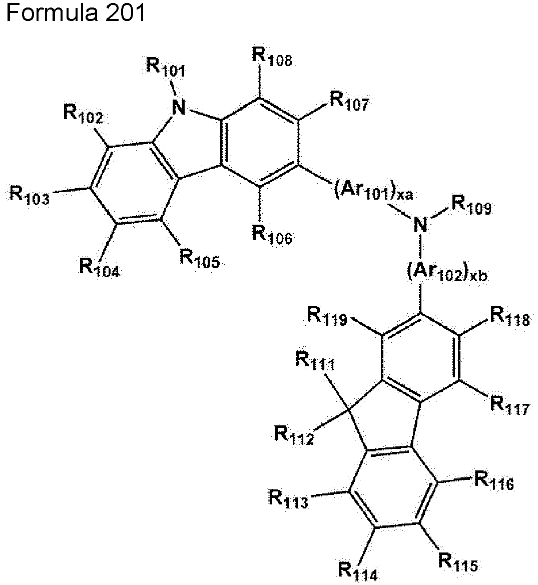

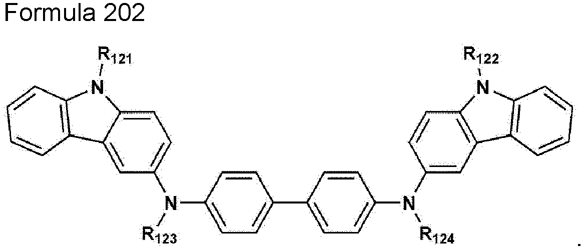

Ar₁₀₁ and Ar₁₀₂ in Formula 201 may each independently be:
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, or a pentacenylene group; or
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, or a pentacenylene group, each substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ cycloalkenyl group, a C₁-C₁₀ heterocycloalkyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, or a combination thereof.

xa and xb in Formula 201 may each independently be an integer from 0 to 5, or 0, 1, or 2. For example, xa may be 1 and xb may be 0, but xa and xb are not limited thereto.

R₁₀₁ to R₁₀₈, R₁₁₁ to R₁₁₉ and R₁₂₁ to R₁₂₄ in Formulae 201 and 202 may each independently be:
hydrogen, deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₁₀ alkyl group (for example, a methyl group, an ethyl group, a propyl group, a butyl group, pentyl group, a hexyl group, or the like) or a C₁-C₁₀ alkoxy group (for example, a methoxy group, an ethoxy group, a propoxy group, a butoxy group, a pentoxy group, or the like);
a C₁-C₁₀ alkyl group or a C₁-C₁₀ alkoxy group, each substituted with deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, or a combination thereof;
a phenyl group, a naphthyl group, an anthracenyl group, a fluorenyl group, or a pyrenyl group; or
a phenyl group, a naphthyl group, an anthracenyl group, a fluorenyl group, or a pyrenyl group, each substituted with deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₁₀ alkyl group, a C₁-C₁₀ alkoxy group or a combination thereof, but embodiments of the present disclosure are not limited thereto.

R₁₀₉ in Formula 201 may be:
a phenyl group, a naphthyl group, an anthracenyl group, or a pyridinyl group; or
a phenyl group, a naphthyl group, an anthracenyl group, or a pyridinyl group, each substituted with deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₂₀ alkyl group, a C₁-C₂₀ alkoxy group, a phenyl group, a naphthyl group, an anthracenyl group, a pyridinyl group, or a combination thereof.

According to an embodiment, the compound represented by Formula 201 may be represented by Formula 201A below, but embodiments of the present disclosure are not limited thereto:

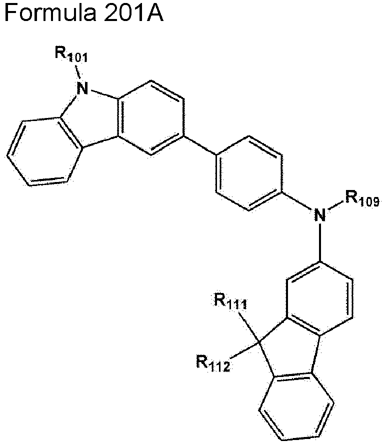

R₁₀₁, R₁₁₁, R₁₁₂, and R₁₀₉ in Formula 201A may be understood by referring to the description provided herein.

For example, the compound represented by Formula 201, and the compound represented by Formula 202 may include compounds HT1 to HT20 illustrated below, but are not limited thereto:

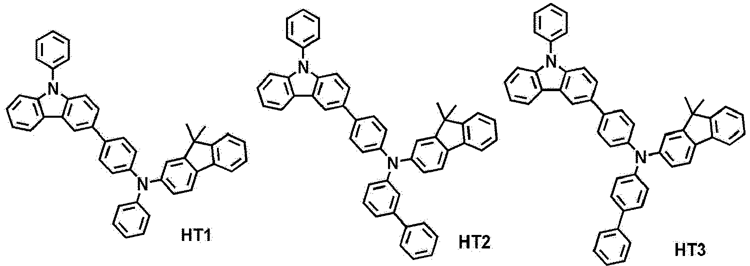

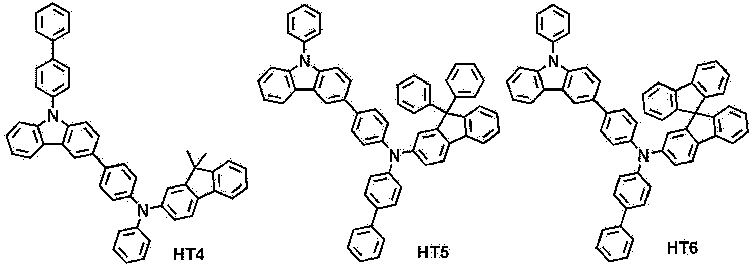

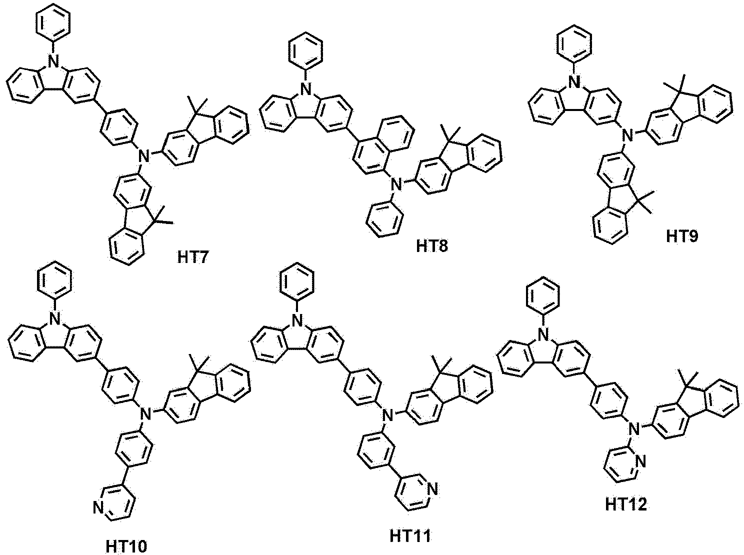

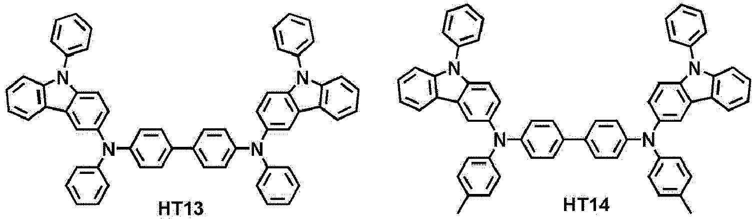

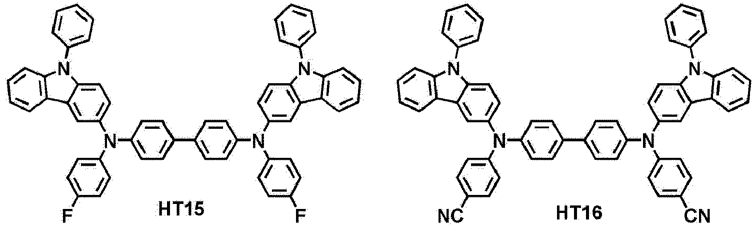

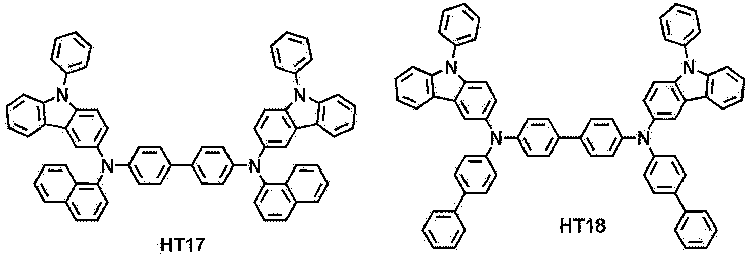

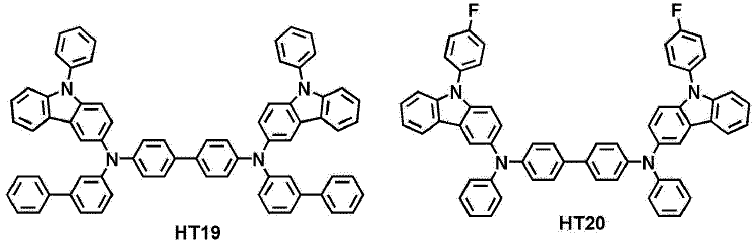

A thickness of the hole transport region may be in the range of about 100 Å to about 10,000 Å, for example, about 100 Å to about 1,000 Å. When the hole transport region includes at least one of a hole injection layer and a hole transport layer, a thickness of the hole injection layer may be in a range of about 100 Å to about 10,000 Å, for example, about 100 Å to about 1,000 Å, and a thickness of the hole transport layer may be in a range of about 50 Å to about 2,000 Å, for example, about 100 Å to about 1,500 Å. When the thicknesses of the hole transport region, the hole injection layer and the hole transport layer are within these ranges, satisfactory hole transporting characteristics may be obtained without a substantial increase in driving voltage.

The hole transport region may further include, in addition to these materials, a charge-generation material for the improvement of conductive properties. The charge-generation material may be homogeneously or non-homogeneously dispersed in the hole transport region.

The charge-generation material may be, for example, a p-dopant. The p-dopant may be one of a quinone derivative, a metal oxide, a cyano group-containing compound, or a combination thereof, but embodiments of the present disclosure are not limited thereto. Non-limiting examples of the p-dopant are a quinone derivative, such as tetracyanoquinonedimethane (TCNQ) or 2,3,5,6-tetrafluoro-tetracyano-1,4-benzoquinonedimethane (F4-TCNQ); a metal oxide, such as a tungsten oxide or a molybdenum oxide; and a cyano group-containing compound, such as Compound HT-D1 or F12, but are not limited thereto.

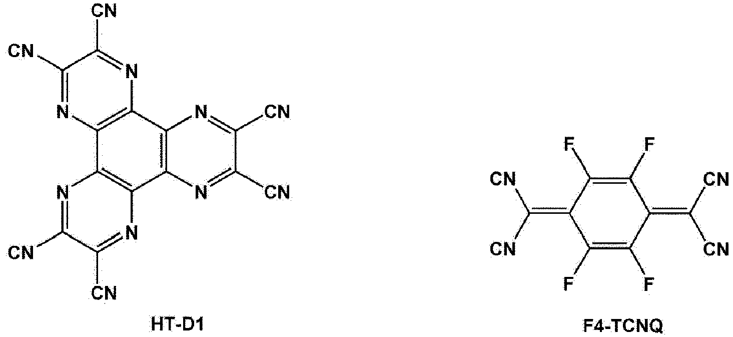

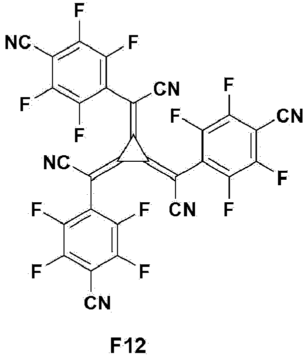

The hole transport region may include a buffer layer.

Also, the buffer layer may compensate for an optical resonance distance according to a wavelength of light emitted from the emission layer, and thus, the efficiency of a formed organic light-emitting device may be improved.

Then, an emission layer (EML) may be formed on the hole transport region by vacuum deposition, spin coating, casting, LB deposition, or the like. When the emission layer is formed by vacuum deposition or spin coating, the deposition or coating conditions may be similar to those applied in forming the hole injection layer although the deposition or coating conditions may vary according to a material that is used to form the emission layer.

Meanwhile, when the hole transport region includes an electron blocking layer, a material for the electron blocking layer may be selected from materials for the hole transport region described above and materials for a host to be explained later. However, the material for the electron blocking layer is not limited thereto. For example, when the hole transport region includes an electron blocking layer, a material for the electron blocking layer may be mCP, which will be explained later.

The emission layer may include a host and a dopant, and the dopant may include the organometallic compound represented by Formula 1.

The host may include at least one of TPBi, TBADN, ADN (also referred to as "DNA"), CBP, CDBP, TCP, mCP, Compound H50, Compound H51, or a combination thereof:

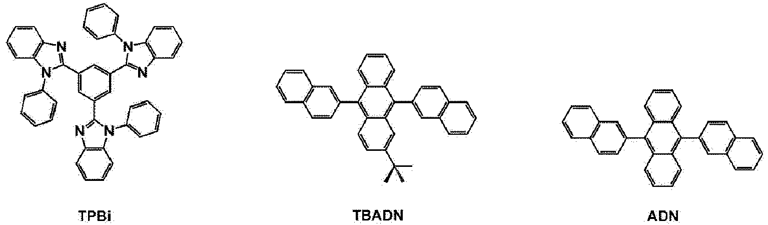

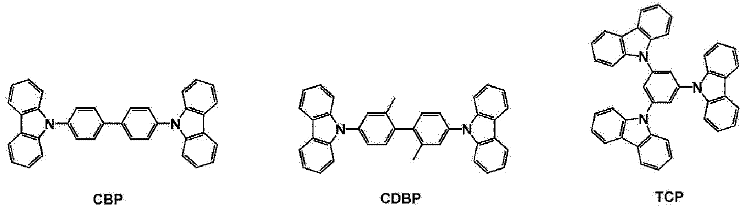

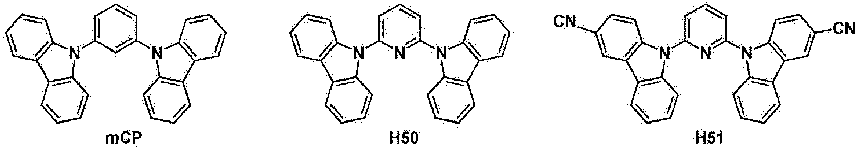

In one or more embodiments, the host may further include a compound represented by Formula 301:

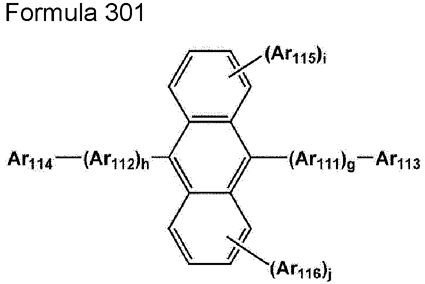

Ar₁₁₁ and Ar₁₁₂ in Formula 301 may each independently be:
a phenylene group, a naphthylene group, a phenanthrenylene group, or a pyrenylene group; or
a phenylene group, a naphthylene group, a phenanthrenylene group, or a pyrenylene group, each substituted with a phenyl group, a naphthyl group, an anthracenyl group, or a combination thereof.

Ar₁₁₃ to Ar₁₁₆ in Formula 301 may each independently be:
a C₁-C₁₀ alkyl group, a phenyl group, a naphthyl group, a phenanthrenyl group, or a pyrenyl group; or
a phenyl group, a naphthyl group, a phenanthrenyl group or a pyrenyl group, each substituted with a phenyl group, a naphthyl group, an anthracenyl group, or a combination thereof.

g, h, i, and j in Formula 301 may each independently be an integer from 0 to 4, and may be, for example, 0, 1, or 2.

Ar₁₁₃ and Ar₁₁₆ in Formula 301 may each independently be:
a C₁-C₁₀ alkyl group which is substituted with a phenyl group, a naphthyl group, an anthracenyl group, or a combination thereof;
a phenyl group, a naphthyl group, an anthracenyl group, a pyrenyl group, a phenanthrenyl group, or a fluorenyl group;
a phenyl group, a naphthyl group, an anthracenyl group, a pyrenyl group, a phenanthrenyl group, or a fluorenyl group, each substituted with deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a C₁-C₆₀ alkyl group, a C₂-C₆₀ alkenyl group, a C₂-C₆₀ alkynyl group, a C₁-C₆₀ alkoxy group, a phenyl group, a naphthyl group, an anthracenyl group, a pyrenyl group, a phenanthrenyl group, a fluorenyl group, or a combination thereof; or

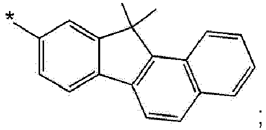

In one or more embodiments, the host may include a compound represented by Formula 302 below:

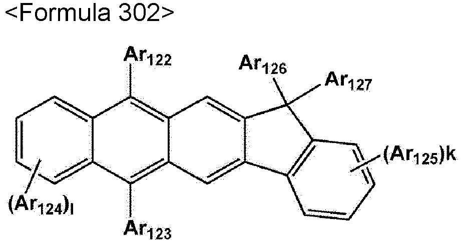

Ar₁₂₂ to Ar₁₂₅ in Formula 302 are the same as described in detail in connection with Ar₁₁₃ in Formula 301.

Ar₁₂₆ and Ar₁₂₇ in Formula 302 may each independently be a C₁-C₁₀ alkyl group (for example, a methyl group, an ethyl group, or a propyl group).

k and I in Formula 302 may each independently be an integer from 0 to 4. For example, k and I may be 0, 1, or 2.

When the organic light-emitting device is a full-color organic light-emitting device, the emission layer may be patterned into a red emission layer, a green emission layer, and a blue emission layer. In one or more embodiments, due to a stacked structure including a red emission layer, a green emission layer, and/or a blue emission layer, the emission layer may emit white light.

When the emission layer includes a host and a dopant, an amount of the dopant may be in a range of about 0.01 parts by weight to about 15 parts by weight based on 100 parts by weight of the host, but embodiments of the present disclosure are not limited thereto.

A thickness of the emission layer may be in a range of about 100 Å to about 1,000 Å, for example, about 200 Å to about 600 Å. When the thickness of the emission layer is within these ranges, excellent light-emission characteristics may be obtained without a substantial increase in driving voltage.

Then, an electron transport region may be located on the emission layer.

The electron transport region may include a hole blocking layer, an electron transport layer, an electron injection layer, or a combination thereof.

For example, the electron transport region may have a hole blocking layer/electron transport layer/electron injection layer structure or an electron transport layer/electron injection layer structure, and the structure of the electron transport region is not limited thereto. The electron transport layer may have a single-layered structure or a multi-layered structure including two or more different materials.

Conditions for forming the hole blocking layer, the electron transport layer, and the electron injection layer which constitute the electron transport region may be understood by referring to the conditions for forming the hole injection layer.

When the electron transport region includes a hole blocking layer, the hole blocking layer may include, for example, at least one of BCP, Bphen, BAlq, or a combination thereof, but embodiments of the present disclosure are not limited thereto.

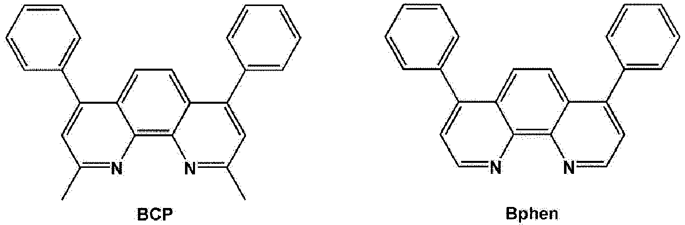

A thickness of the hole blocking layer may be in a range of about 20 Å to about 1,000 Å, for example, about 30 Å to about 300 Å. When the thickness of the hole blocking layer is within these ranges, the hole blocking layer may have excellent hole blocking characteristics without a substantial increase in driving voltage.

The electron transport layer may further include at least one of BCP, Bphen, Alq₃, BAlq, TAZ, NTAZ, or a combination thereof.

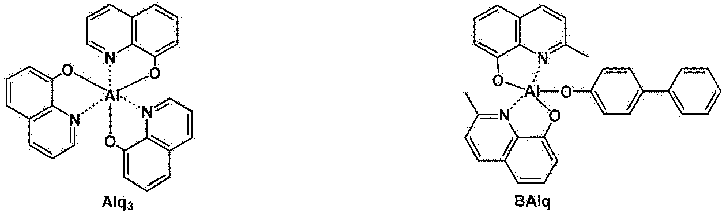

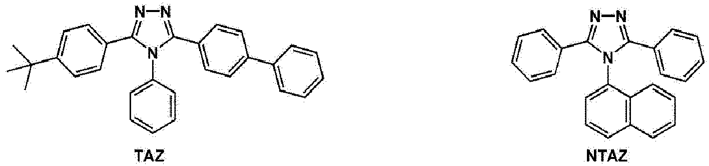

In one or more embodiments, the electron transport layer may include at least one of ET1 to ET25, but are not limited thereto:

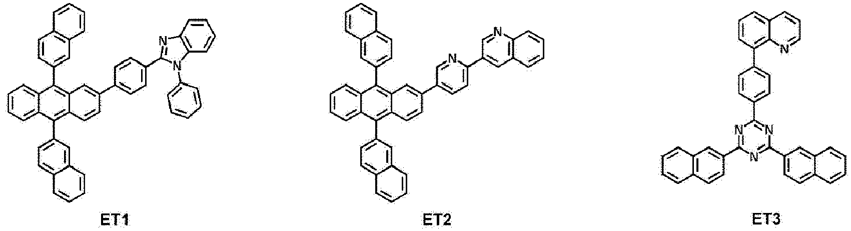

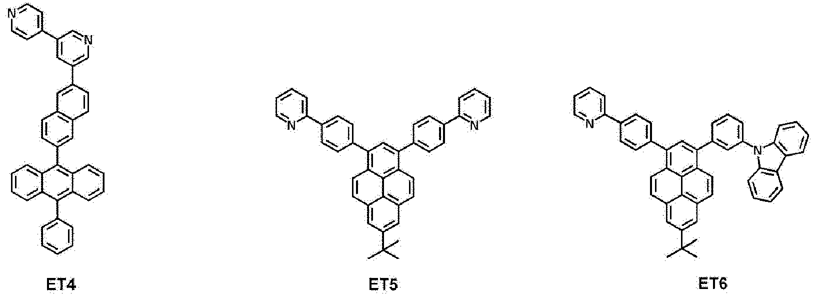

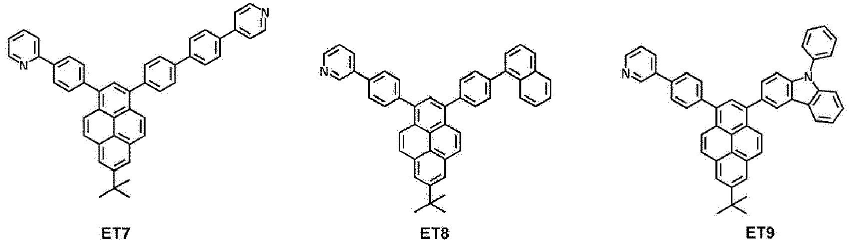

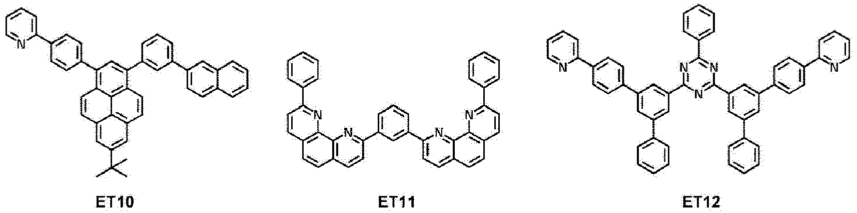

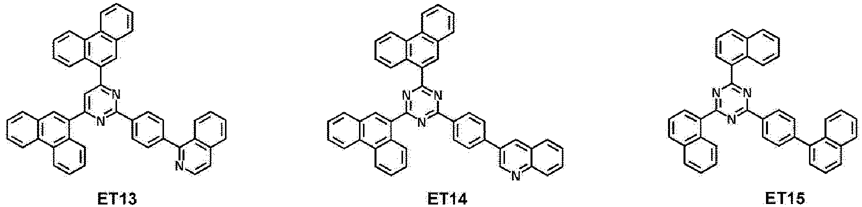

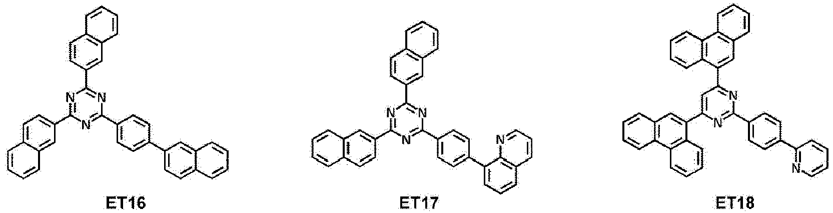

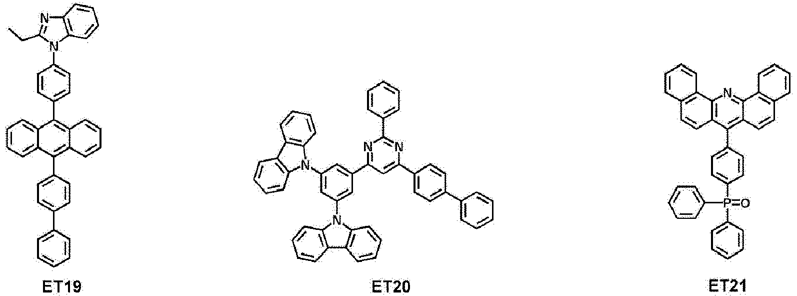

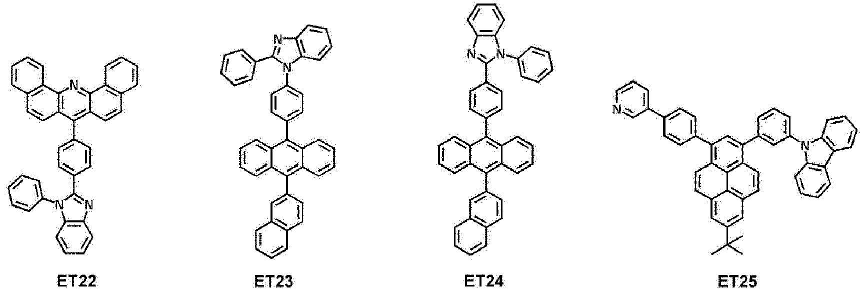

A thickness of the electron transport layer may be in the range of about 100 Å to about 1,000 Å, for example, about 150 Å to about 500 Å. When the thickness of the electron transport layer is within the range described above, the electron transport layer may have satisfactory electron transport characteristics without a substantial increase in driving voltage.

Also, the electron transport layer may further include, in addition to the materials described above, a metal-containing material.

The metal-containing material may include a Li complex. The Li complex may include, for example, Compound ET-D1 (lithium quinolate, LiQ) or ET-D2:

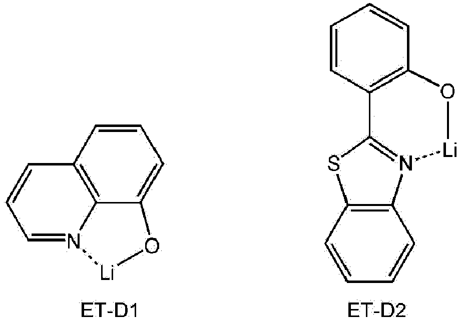

The electron transport region may include an electron injection layer (EIL) that promotes the flow of electrons from the second electrode 19 thereinto.

The electron injection layer may include LiF, NaCl, CsF, Li₂O, BaO, or a combination thereof.

A thickness of the electron injection layer may be in a range of about 1 Å to about 100 Å, and, for example, about 3 Å to about 90 Å. When the thickness of the electron injection layer is within the range described above, the electron injection layer may have satisfactory electron injection characteristics without a substantial increase in driving voltage.

The second electrode 19 is located on the organic layer 15. The second electrode 19 may be a cathode. A material for forming the second electrode 19 may be metal, an alloy, an electrically conductive compound, or a combination thereof, which have a relatively low work function. For example, lithium (Li), magnesium (Mg), aluminum (Al), aluminum-lithium (Al-Li), calcium (Ca), magnesium-indium (Mg-In), or magnesium-silver (Mg-Ag) may be used as the material for forming the second electrode 19. In one or more embodiments, to manufacture a top-emission type light-emitting device, a transmissive electrode formed using ITO or IZO may be used as the second electrode 19.

Hereinbefore, the organic light-emitting device has been described with reference to FIGURE, but embodiments of the present disclosure are not limited thereto.

Another aspect provides a diagnostic composition including at least one organometallic compound represented by Formula 1.

The organometallic compound represented by Formula 1 provides high luminescent efficiency. Accordingly, a diagnostic composition including the organometallic compound may have high diagnostic efficiency.

The diagnostic composition may be used in various applications including a diagnostic kit, a diagnostic reagent, a biosensor, and a biomarker.

The term "C₁-C₆₀ alkyl group" as used herein refers to a linear or branched saturated aliphatic hydrocarbon monovalent group having 1 to 60 carbon atoms, and non-limiting examples thereof include a methyl group, an ethyl group, a propyl group, an isobutyl group, a sec-butyl group, a tert-butyl group, a pentyl group, an isoamyl group, and a hexyl group. The term "C₁-C₆₀ alkylene group" as used herein refers to a divalent group having the same structure as the C₁-C₆₀ alkyl group.

The term "C₁-C₆₀ alkoxy group" used herein refers to a monovalent group represented by -OA₁₀₁ (wherein A₁₀₁ is the C₁-C₆₀ alkyl group), and examples thereof include a methoxy group, an ethoxy group, and an isopropyloxy group.

The term "C₂-C₆₀ alkenyl group" as used herein refers to a hydrocarbon group formed by substituting at least one carbon-carbon double bond in the middle or at the terminus of the C₂-C₆₀ alkyl group, and examples thereof include an ethenyl group, a propenyl group, and a butenyl group. The term "C₂-C₆₀ alkenylene group" as used herein refers to a divalent group having the same structure as the C₂-C₆₀ alkenyl group.

The term "C₂-C₆₀ alkynyl group" as used herein refers to a hydrocarbon group formed by substituting at least one carbon-carbon triple bond in the middle or at the terminus of the C₂-C₆₀ alkyl group, and examples thereof include an ethynyl group, and a propynyl group. The term "C₁-C₆₀ alkynylene group" as used herein refers to a divalent group having the same structure as the C₁-C₆₀ alkynyl group.

The term "C₃-C₁₀ cycloalkyl group" as used herein refers to a monovalent saturated hydrocarbon monocyclic group having 3 to 10 carbon atoms, and examples thereof include a cyclopropyl group, a cyclobutyl group, a cyclopentyl group, a cyclohexyl group, and a cycloheptyl group. The term "C₃-C₁₀ cycloalkylene group" as used herein refers to a divalent group having the same structure as the C₃-C₁₀ cycloalkyl group.

The term "C₁-C₁₀ heterocycloalkyl group" as used herein refers to a monovalent saturated monocyclic group having at least one heteroatom selected from N, O, P, Si, S, Se, B, Te, Ge, or a combination thereof as a ring-forming atom and 1 to 10 carbon atoms, and non-limiting examples thereof include a tetrahydrofuranyl group, and a tetrahydrothiophenyl group. The term "C₁-C₁₀ heterocycloalkylene group" as used herein refers to a divalent group having the same structure as the C₁-C₁₀ heterocycloalkyl group.

The term "C₃-C₁₀ cycloalkenyl group" as used herein refers to a monovalent monocyclic group that has 3 to 10 carbon atoms and at least one carbon-carbon double bond in the ring thereof and no aromaticity, and non-limiting examples thereof include a cyclopentenyl group, a cyclohexenyl group, and a cycloheptenyl group. The term "C₃-C₁₀ cycloalkenylene group" as used herein refers to a divalent group having the same structure as the C₃-C₁₀ cycloalkenyl group.

The term "C₂-C₁₀ heterocycloalkenyl group" as used herein refers to a monovalent monocyclic group that has at least one hetero atom selected from N, O, P, Si, S, Se, B, Te, Ge, or a combination thereof as a ring-forming atom, 2 to 10 carbon atoms, and at least one carbon-carbon double bond in its ring. Examples of the C₂-C₁₀ heterocycloalkenyl group are a 2,3-dihydrofuranyl group, and a 2,3-dihydrothiophenyl group. The term "C₂-C₁₀ heterocycloalkenylene group" as used herein refers to a divalent group having the same structure as the C₂-C₁₀ heterocycloalkenyl group.

The term "C₆-C₆₀ aryl group" as used herein refers to a monovalent group having a carbocyclic aromatic system having 6 to 60 carbon atoms, and the term "C₆-C₆₀ arylene group" as used herein refers to a divalent group having a carbocyclic aromatic system having 6 to 60 carbon atoms. Examples of the C₆-C₆₀ aryl group include a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, the rings may be fused to each other. The C₇₋C₆₀ alkylaryl group refers to a C₆-C₆₀ aryl group substituted with at least one C₁-C₆₀ alkyl group.

The term "C₁-C₆₀ heteroaryl group" as used herein refers to a monovalent group having a cyclic aromatic system that has at least one heteroatom of N, O, P, Si, S, Se, B, Te, Ge, or a combination thereof as a ring-forming atom, and 1 to 60 carbon atoms. The term "C₁-C₆₀ heteroarylene group" as used herein refers to a divalent group having a carbocyclic aromatic system that has at least one heteroatom of N, O, P, S, Se, B, Te, Ge, or a combination thereof as a ring-forming atom, and 1 to 60 carbon atoms. Examples of the C₁-C₆₀ heteroaryl group include a pyridinyl group, a pyrimidinyl group, a pyrazinyl group, a pyridazinyl group, a triazinyl group, a quinolinyl group, and an isoquinolinyl group. When the C₆-C₆₀ heteroaryl group and the C₆-C₆₀ heteroarylene group each include two or more rings, the rings may be fused to each other. The C₂-C₆₀ alkylheteroaryl group refers to a C₁-C₆₀ heteroaryl group substituted with at least one C₁-C₆₀ alkyl group.

The term "C₆-C₆₀ aryloxy group" as used herein indicates -OA₁₀₂ (wherein A₁₀₂ is the C₆-C₆₀ aryl group), and the term "C₆-C₆₀ arylthio group" as used herein indicates -SA₁₀₃ (wherein A₁₀₃ is the C₆-C₆₀ aryl group).

The C₁-C₆₀ heteroaryloxy group used herein indicates -OA₁₀₄ (wherein A₁₀₄ is a C₁-C₆₀ heteroaryl group), and the C₁-C₆₀ heteroarylthio group indicates -SA₁₀₅ (wherein A₁₀₅ is the C₁-C₆₀ heteroaryl group).

The term "monovalent non-aromatic condensed polycyclic group" as used herein refers to a monovalent group (for example, having 8 to 60 carbon atoms) having two or more rings condensed to each other, only carbon atoms as ring-forming atoms, and no aromaticity in its entire molecular structure. Examples of the monovalent non-aromatic condensed polycyclic group include a fluorenyl group. The term "divalent non-aromatic condensed polycyclic group" as used herein refers to a divalent group having the same structure as a monovalent non-aromatic condensed polycyclic group.

The term "monovalent non-aromatic condensed heteropolycyclic group" as used herein refers to a monovalent group (for example, having 2 to 60 carbon atoms) having two or more rings condensed to each other, a heteroatom selected from N, O, P, Si, S, Se, B, Te, Ge, or a combination thereof, other than carbon atoms, as a ring-forming atom, and no aromaticity in its entire molecular structure. Examples of the monovalent non-aromatic condensed heteropolycyclic group include a carbazolyl group. The term "divalent non-aromatic heterocondensed polycyclic group" as used herein refers to a divalent group having the same structure as a monovalent non-aromatic heterocondensed polycyclic group.

The term "C₅-C₃₀ carbocyclic group" as used herein refers to a saturated or unsaturated cyclic group having, as a ring-forming atom, 5 to 30 carbon atoms only. The C₅-C₃₀ carbocyclic group may be a monocyclic group or a polycyclic group.

The term "C₁-C₃₀ heterocyclic group" as used herein refers to a saturated or unsaturated cyclic group having, as a ring-forming atom, at least one heteroatom selected from N, O, Si, P, S, Se, B, Te, Ge, or a combination thereof other than 1 to 30 carbon atoms. The C₁-C₃₀ heterocyclic group may be a monocyclic group or a polycyclic group.

In the present specification, TMS represents * -Si(CH₃)₃, and TMG represents * -Ge(CH₃)₃.

At least one substituent of the substituted C₅-C₃₀ carbocyclic group, the substituted C₁-C₃₀ heterocyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₂-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted C₁-C₆₀ heteroaryloxy group, the substituted C₁-C₆₀ hetero arylthio group, the substituted monovalent non-aromatic condensed polycyclic group, and the substituted monovalent non-aromatic condensed heteropolycyclic group may be:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, or a combination thereof;
a C₁-C₆₀ alkyl group, a C₂-C₆₀ alkenyl group, a C₂-C₆₀ alkynyl group, a C₁-C₆₀ alkoxy group, or a combination thereof, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a C₁-C₆₀ heteroaryloxy group, a C₁-C₆₀ heteroarylthio group, a monovalent non-aromatic condensed polycyclic group, a monovalent non-aromatic condensed heteropolycyclic group, -Si(Q₁₁)(Q₁₂)(Q₁₃), -Ge(Q₁₁)(Q₁₂)(Q₁₃), -N(Q₁₄)(Q₁₅), -B(Q₁₆)(Q₁₇), - P(=0)(Q₁₈)(Q₁₉), or a combination thereof;
a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a C₁-C₆₀ heteroaryloxy group, a C₁-C₆₀ heteroarylthio group, a monovalent non-aromatic condensed polycyclic group, a monovalent non-aromatic condensed heteropolycyclic group, or a combination thereof;
a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, or a combination thereof, each substituted with deuterium, -F, -Cl, -Br, -I, -CD₃, -CD₂H, -CDH₂, -CF₃, -CF₂H, - CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a C₁-C₆₀ heteroaryloxy group, a C₁-C₆₀ heteroarylthio group, a monovalent non-aromatic condensed polycyclic group, a monovalent non-aromatic condensed heteropolycyclic group, - Si(Q₂₁)(Q₂₂)(Q₂₃), -Ge(Q₂₁)(Q₂₂)(Q₂₃), -N(Q₂₄)(Q₂₅), -B(Q₂₆)(Q₂₇), - P(=O)(Q₂₈)(Q₂₉), or a combination thereof; or
-Si(Q₃₁)(Q₃₂)(Q₃₃), -Ge(Q₃₁)(Q₃₂)(Q₃₃), -N(Q₃₄)(Q₃₅), -B(Q₃₆)(Q₃₇), or - P(=O)(Q₃₈)(Q₃₉), or a combination thereof,
wherein Q₁ to Q₉, Q₁₁ to Q₁₉, Q₂₁ to Q₂₉, and Q₃₁ to Q₃₉ may each independently be hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a C₁-C₆₀ heteroaryloxy group, a C₁-C₆₀ heteroarylthio group, a substituted or unsubstituted monovalent non-aromatic condensed polycyclic group, or a substituted or unsubstituted monovalent non-aromatic condensed heteropolycyclic group.

Hereinafter, a compound and an organic light-emitting device according to embodiments are described in detail with reference to Synthesis Example and Examples. However, the organic light-emitting device is not limited thereto. The wording "'B' was used instead of 'A‴ used in describing Synthesis Examples means that an amount of 'A' used was identical to an amount of 'B' used, in terms of a molar equivalent.

### Examples

### Synthesis Example 1: Synthesis of Compound 1

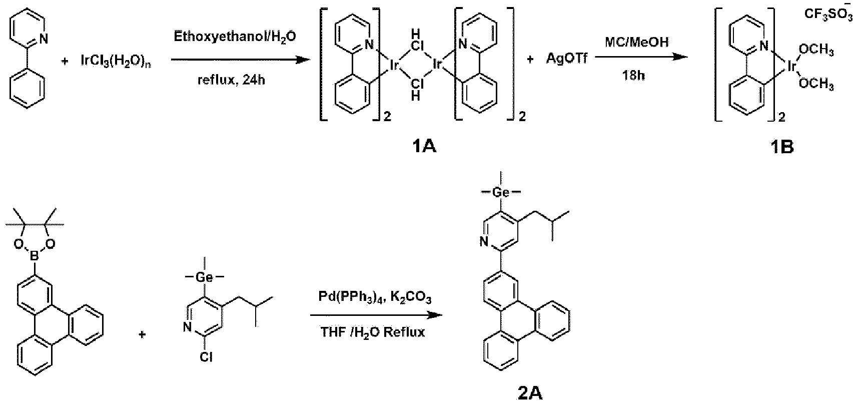

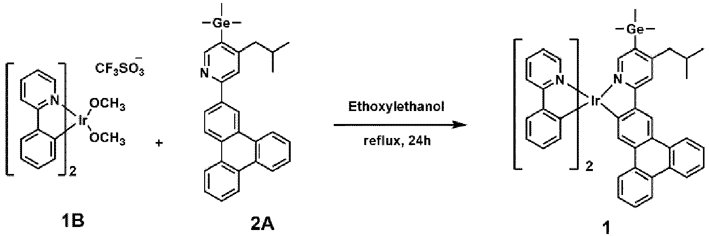

### (1) Synthesis of Compound 1A

2-phenyl-pyridine (5.0 g, 31.9 mmol) and iridium chloride (4.9 g, 14.2 mmol) were mixed with 120 mL of ethoxyethanol and 40 mL of deionized (DI) water, and then stirred for 24 hours while refluxing. The resultant mixture was cooled to room temperature. The resulting solid was separated by filtration, washed sufficiently with water, methanol, and hexane, in this stated order, and then dried in a vacuum oven to obtain 7.9 g (yield of 89%) of Compound 1A. Compound 1A obtained was used in the next reaction without an additional purification process.

### (2) Synthesis of Compound 1B

Compound 1A (1.6 g, 1.5 mmol) and 45 mL of methylene chloride were mixed, and then, AgOTf (0.8 g, 3.1 mmol) was added thereto after being mixed with 15 mL of methanol. Thereafter, the mixture was stirred for 18 hours at room temperature while light was blocked with aluminum foil, and then filtered through Celite to remove the resulting solid, and the filtrate was concentrated under reduced pressure to obtain a solid (Compound 1B). Compound 1B was used in the next reaction without an additional purification process.

### (3) Synthesis of Compound 2A

Under nitrogen environment, 4,4,5,5-tetramethyl-2-(triphenylene-2-yl)-1,3,2-dioxoborolane (3.0 g, 8.4 mmol) and 2-chloro-4-isobutyl-5-(trimethylgermyl)pyridine (2.0 g, 7.0 mmol) were dissolved in 140 ml of tetrahydrofuran. Then, potassium carbonate (K₂CO₃) (2.2 g, 21.0 mmol) was dissolved in 35 mL of DI water, and then, the resultant solution was added to a reaction mixture, and a palladium catalyst (Pd(PPh₃)₄) (0.81g, 0.70 mmol) was added thereto. Then, the reaction mixture was stirred while refluxing at 100 °C. After extraction, the obtained solid was subjected to column chromatography (eluent: MC (methylene chloride) and hexane) to obtain 4.0 g (yield of 91%) of 4-isobutyl-5-(trimethylgermyl)-2-(triphenylene-2-yl)pyridine. The obtained compound was identified by HRMS and HPLC analysis.
HRMS(MALDI) calcd for C30H31GeN: m/z: 479.17 Found: 480.21

### (4) Synthesis of Compound 1

Compound 1B (1.5 g, 2.1 mmol) and Compound 2A (1.1 g, 2.3 mmol) were mixed with 100 mL of 2-ethoxyethanol, and stirred while refluxing for 24 hours and then, the result was cooled. An extraction process was performed thereon with methylene chloride and water, and then, the water layer was removed therefrom. The resulting organic layer was treated with anhydrous magnesium sulfate, followed by filtration and concentration under reduced pressure. The obtained solid was subjected to column chromatography (eluent: methylene chloride (MC) and hexane) to obtain 0.7 g (yield of 35%) of Compound 1. The obtained compound was identified by Mass and HPLC analysis.
HRMS(MALDI) calcd for C52H46GelrN3: m/z: 979.25 Found: 980.81

### Synthesis Example 2: Synthesis of Compound 2

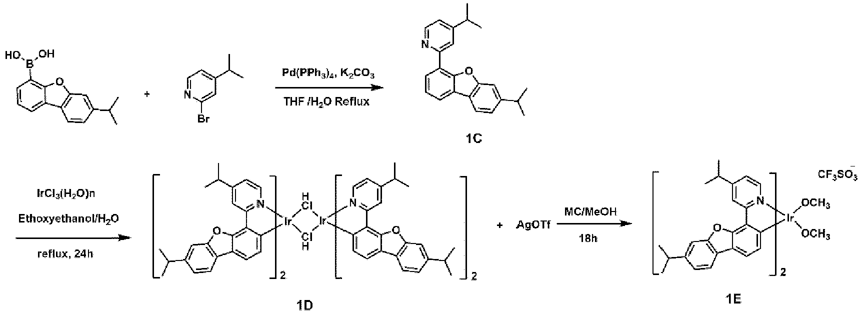

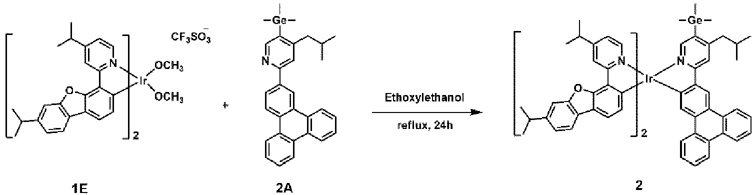

### (1) Synthesis of Compound 1C

2.3 g (yield of 93%) of 4-isopropyl-2-(7-isopropyldibenzo[b,d]furan-4-yl)pyridine was obtained in the same manner as used to synthesize Compound 2A, except that (7-isopropyldibenzo[b,d]furan-4-yl)boronic acid (2.3 g, 9.0 mmol) was used instead of 4,4,5,5-tetramethyl-2-(triphenylen-2-yl)-1,3,2-dioxoborolane, and 2-bromo-4-isopropylpyridine (1.5 g, 7.5 mmol) was used instead of 2-chloro-4-isobutyl-5-(trimethylgermyl group)pyridine. The obtained compound was identified by Mass and HPLC analysis.
HRMS(MALDI) calcd for C23H23NO: m/z: 329.18 Found: 330.44

### (2) Synthesis of Compound 1D

2.2 g (yield of 84%) was obtained in the same manner as used to synthesize Compound 1A, except that 4-isopropyl-2-(7-isopropyldibenzo[b,d]furan-4-yl)pyridine (2.0 g, 6.0 mmol) was used instead of 2-phenyl-pyridine. Compound 1D obtained was used in the next reaction without an additional purification process.

### (3) Synthesis of compound 1E

Compound 1E was obtained in the same manner as used to synthesize Compound 1B, except that Compound 1D (1.4 g, 0.8 mmol) was used instead of Compound 1A. Compound 1E obtained was used in the next reaction without an additional purification process.

### (4) Synthesis of Compound 2

0.73 g (yield of 35%) of Compound 2 was obtained in the same manner as used to synthesize Compound 1, except that Compound 1E (1.5 g, 1.4 mmol) and Compound 2A (0.8 g, 1.6 mmol) were used. The obtained compound was identified by Mass and HPLC analysis.
HRMS(MALDI) calcd for C76H74GeIrN3O2: m/z: 1327.46 Found: 1327.35 Synthesis Example 3: Synthesis of Compound 3

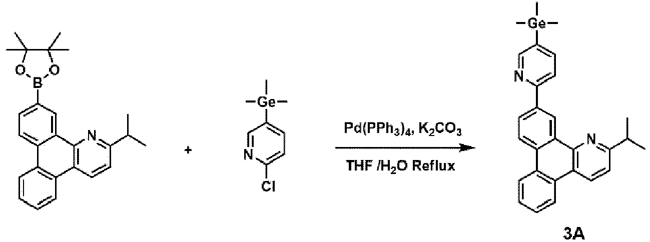

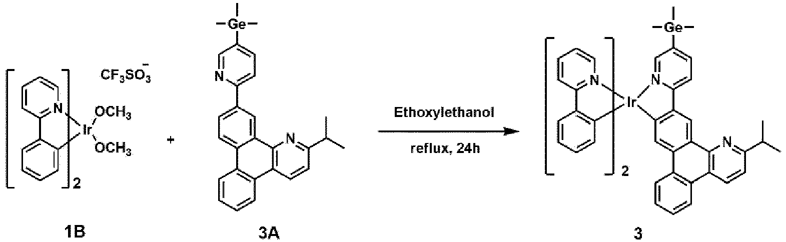

### (1) Synthesis of Compound 3A

1.1 g (yield of 91%) of 2-isopropyl-11-(5-(trimethylgermyl group)pyridin-2-yl)dibenzo[f,h]quinoline was obtained in the same manner as used to synthesize Compound 2A, except that 2-isopropyl-11-(4,4,5,5-tetramethyl-1,3,2-dioxoborolan-2-yl)dibenzo[f,h]quinoline (1.1 g, 2.9 mmol) and 2-chloro-5-(trimethylgermyl group)pyridine (0.6 g, 2.6 mmol) were used instead of 4,4,5,5-tetramethyl-2-(triphenylen-2-yl)-1,3,2-dioxoborolane. The obtained compound was identified by Mass and HPLC analysis.
HRMS(MALDI) calcd for C28H28GeN2: m/z: 466.15 Found: 467.28

### (2) Synthesis of Compound 3

0.70 g (yield of 39%) of Compound 3 was obtained in the same manner as used to synthesize Compound 1, except that Compound 1B (1.2 g, 1.7 mmol) and Compound 3A (0.9 g, 1.9 mmol) were used. The obtained compound was identified by Mass and HPLC analysis.
HRMS(MALDI) calcd for C50H43GeIrN4 : m/z: 964.77 Found: 965.31

### Example 1

As an anode, an ITO-patterned glass substrate was cut to a size of 50 mm x 50 mm x 0.5 mm, sonicated with isopropyl alcohol and pure water, each for 5 minutes, and then cleaned by exposure to ultraviolet rays and ozone for 30 minutes. The resultant glass substrate was loaded onto a vacuum deposition apparatus.

Compounds HT3 and F12(p-dopant) were vacuum-codeposited on the anode at the weight ratio of 98 : 2 to form a hole injection layer having a thickness of 100 Å, and Compound HT3 was vacuum-deposited on the hole injection layer to form a hole transport layer having a thickness of 1,650 Å.

Then, GH3 (host) and Compound 1(dopant) were co-deposited at a weight ratio of 92:8 on the hole transport layer to form an emission layer having a thickness of 400 Å.

Then, Compound ET3 and LiQ (n-dopant) were co-deposited on the emission layer at the volume ratio of 50:50 to form an electron transport layer having a thickness of 350 Å, LiQ was vacuum-deposited on the electron transport layer to form an electron injection layer having a thickness of 10 Å, and AI was vacuum-deposited on the electron injection layer to form a cathode having a thickness of 1000 Å, thereby completing the manufacture of an organic light-emitting device.

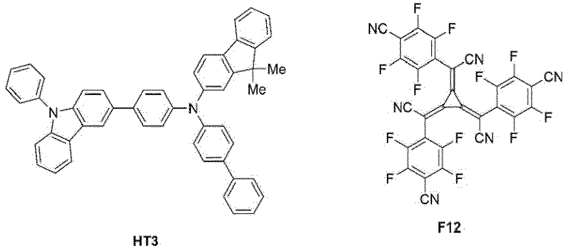

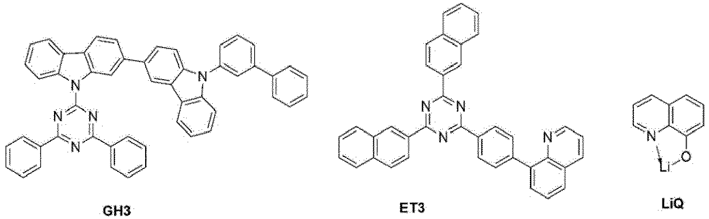

### Examples 2 to 3 and Comparative Examples 1 to 3

Organic light-emitting devices were manufactured in the same manner as in Example 1, except that Compounds shown in Table 2 were each used instead of Compound 1 as a dopant in forming an emission layer.

The driving voltage, external quantum efficiency, maximum emission wavelength (λₘₐₓ), FWHM, and lifespan (T₉₇) of each of the organic light-emitting devices manufactured according to Examples 1 to 3 and Comparative Examples 1 to 3 were evaluated. Results thereof are shown in Table 2. A current-voltage meter (Keithley 2400) and a luminance meter (Minolta Cs-1,000A) were used as an apparatus for evaluation, and the lifespan (T₉₇) (at 18,000 nit, wherein 1nit= 1cd/m2) was obtained by measuring the amount of time that elapsed until luminance was reduced to 97% of the initial brightness of 100%, and the results are expressed as a relative value.

**Table 2**

| | Dopant in emission layer | Driving voltage (V) | External quantum efficiency (%) | Maximum emission wavelength (nm) | FWHM (nm) | LT₉₇(%) |
|---|---|---|---|---|---|---|
| Example 1 | Compound 1 | 4.2 | 23.0 | 528 | 68 | 120% |
| Example 2 | Compound 2 | 4.15 | 23.5 | 528 | 67 | 125% |
| Example 3 | Compound 3 | 4.2 | 22.5 | 529 | 72 | 105% |
| Comparative Example 1 | Compound A | 4.4 | 21.5 | 535 | 74 | 100 % |
| Comparative Example 2 | Compound B | 4.3 | 22.0 | 520 | 70 | 20% |
| Comparative Example 3 | Compound C | 4.3 | 20.0 | 527 | 73 | 90% |

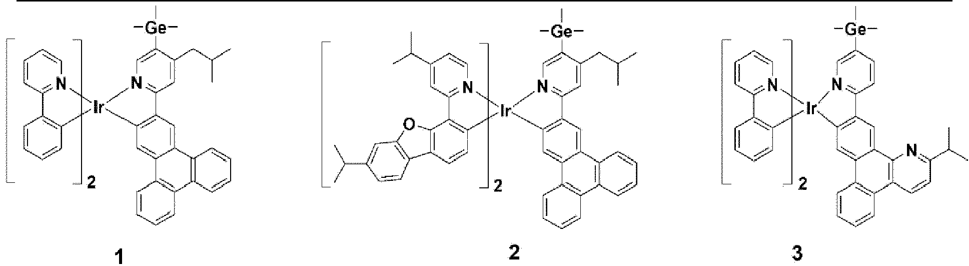

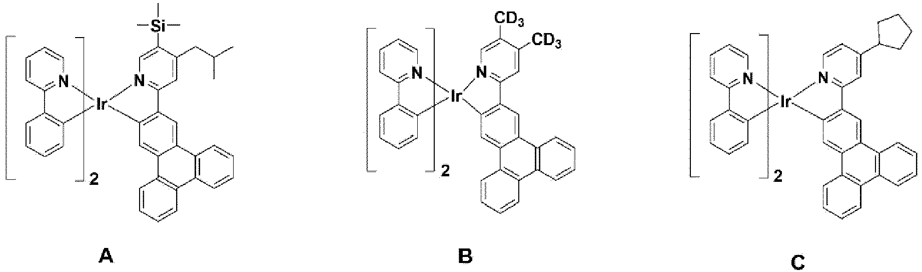

Referring to Table 2, it can be seen that the organic light-emitting devices of Examples 1 to 3 have low driving voltage, narrow FWHM, and excellent characteristics in terms of current efficiency, external quantum efficiency, and lifespan. In addition, it can be seen that the organic light-emitting devices of Example 1 to 3 have a lower driving voltage, a similar level of or narrower FWHM, higher current efficiency, higher external quantum efficiency, and longer lifespan characteristics than the organic light-emitting devices of Comparative Example 1 to 3.

The organometallic compounds have excellent electrical characteristics and thermal stability. The organometallic compounds have a high glass transition temperature so that crystallization thereof can be prevented, and electric mobility thereof can be improved. Accordingly, an electronic device using the organometallic compounds, for example, an organic light-emitting device using the organometallic compounds, has a low driving voltage, high efficiency, a long lifespan, reduced roll-off ratio, and a relatively narrow EL spectrum emission peak FWHM.

Thus, due to the use of the organometallic compounds, a high-quality organic light-emitting device may be embodied. Such organometallic compounds have excellent phosphorescent luminescent characteristics, and thus, when used, a diagnostic composition having a high diagnostic efficiency may be provided.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the scope as defined by the following claims.

## Claims

1. An organometallic compound represented by Formula 1:
Formula 1 M₁(Ln₁)ₙ₁(Ln₂)ₙ₂
wherein, in Formula 1,
M₁ is a transition metal,
Ln₁ is a ligand represented by Formula 1-1,
Ln₂ is a ligand represented by Formula 1-2,
n1 is 0, 1, or 2,
n2 is 1, 2, or 3, 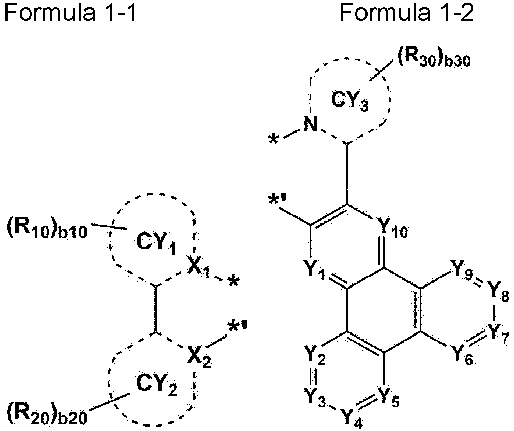
wherein, in Formulae 1-1 and 1-2,
X₁ is C or N, and X₂ is C or N,
Y₁ is C(R₄₁) or N, Y₂ is C(R₄₂) or N, Y₃ is C(R₄₃) or N, Y₄ is C(R₄₄) or N, Y₅ is C(R₄₅) or N, Y₆ is C(R₄₆) or N, Y₇ is C(R₄₇) or N, Y₈ is C(R₄₈) or N, Y₉ is C(R₄₈) or N, and Y₁₀ is C(R₅₀) or N,
CY₁ and CY₂ are each independently a C₅-C₃₀ carbocyclic group or a C₁-C₃₀ heterocyclic group,
CY₃ is a N-containing C₁-C₃₀ heterocyclic group,
R₁₀, R₂₀, R₃₀, and R₄₁ to R₅₀ are each independently hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted monovalent non-aromatic condensed polycyclic group, a substituted or unsubstituted monovalent non-aromatic condensed heteropolycyclic group, -Si(Q₁)(Q₂)(Q₃), -Ge(Q₁)(Q₂)(Q₃), -N(Q₄)(Q₅), - B(Q₆)(Q₇), or -P(=O)(Q₈)(Q₉),
at least one of the ligands represented by Formulae 1-1 and 1-2 comprises at least one -Ge(Q₁)(Q₂)(Q₃),
two or more of a plurality of R₁₀ are optionally bonded together to form a substituted or unsubstituted C₅-C₃₀ carbocyclic group or a substituted or unsubstituted C₁-C₃₀ heterocyclic group,
two or more of a plurality of R₂₀ are optionally bonded together to form a substituted or unsubstituted C₅-C₃₀ carbocyclic group or a substituted or unsubstituted C₁-C₃₀ heterocyclic group,
two or more of a plurality of R₃₀ are optionally bonded together to form a substituted or unsubstituted C₅-C₃₀ carbocyclic group or a substituted or unsubstituted C₁-C₃₀ heterocyclic group,
two or more neighboring groups of R₁₀, R₂₀, R₃₀, and R₄₁ to R₅₀ are optionally bonded together to form a substituted or unsubstituted C₅-C₃₀ carbocyclic group or a substituted or unsubstituted C₁-C₃₀ heterocyclic group,
b10, b20, and b30 are each independently 1, 2, 3, 4, 5, 6, 7, 8, 9, or 10,
at least one substituent of the substituted C₅-C₃₀ carbocyclic group, the substituted C₁-C₃₀ heterocyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₂-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted C₁-C₆₀ heteroaryloxy group, the substituted C₁-C₆₀ hetero arylthio group, the substituted monovalent non-aromatic condensed polycyclic group, and the substituted monovalent non-aromatic condensed heteropolycyclic group is:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, or a C₁-C₆₀ alkoxy group;
a C₁-C₆₀ alkyl group, a C₂-C₆₀ alkenyl group, a C₂-C₆₀ alkynyl group, or a C₁-C₆₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a C₁-C₆₀ heteroaryloxy group, a C₁-C₆₀ heteroarylthio group, a monovalent non-aromatic condensed polycyclic group, a monovalent non-aromatic condensed heteropolycyclic group, -Si(Q₁₁)(Q₁₂)(Q₁₃), -Ge(Q₁₁)(Q₁₂)(Q₁₃), -N(Q₁₄)(Q₁₅), -B(Q₁₆)(Q₁₇), - P(=O)(Q₁₈)(Q₁₉), or a combination thereof;
a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a C₁-C₆₀ heteroaryloxy group, a C₁-C₆₀ heteroarylthio group, a monovalent non-aromatic condensed polycyclic group, or a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a C₁-C₆₀ heteroaryloxy group, a C₁-C₆₀ heteroarylthio group, a monovalent non-aromatic condensed polycyclic group, a monovalent non-aromatic condensed heteropolycyclic group, -Si(Q₂₁)(Q₂₂)(Q₂₃), -Ge(Q₂₁)(Q₂₂)(Q₂₃), -N(Q₂₄)(Q₂₅), -B(Q₂₆)(Q₂₇), - P(=O)(Q₂₈)(Q₂₉), or a combination thereof; or
-Si(Q₃₁)(Q₃₂)(Q₃₃), -Ge(Q₃₁)(Q₃₂)(Q₃₃), -N(Q₃₄)(Q₃₅), -B(Q₃₆)(Q₃₇), or - P(=O)(Q₃₈)(Q₃₉),
wherein Q₁ to Q₉, Q₁₁ to Q₁₉, Q₂₁ to Q₂₉, and Q₃₁ to Q₃₉ are each independently hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a C₁-C₆₀ heteroaryloxy group, a C₁-C₆₀ heteroarylthio group, a substituted or unsubstituted monovalent non-aromatic condensed polycyclic group, or a substituted or unsubstituted monovalent non-aromatic condensed heteropolycyclic group.

2. The organometallic compound of claim 1, wherein M₁ is iridium (Ir), platinum (Pt), osmium (Os), titanium (Ti), zirconium (Zr), hafnium (Hf), europium (Eu), terbium (Tb), thulium (Tm), or rhodium (Rh);
preferably wherein M₁ is Ir, and the sum of n1 and n2 is 3.

3. The organometallic compound of claims 1 or 2, wherein CY₁ and CY₂ are each independently a benzene group, a naphthalene group, 1,2,3,4-tetrahydronaphthalene group, a phenanthrene group, a pyridine group, a pyrimidine group, a pyrazine group, a pyridazine group, a triazine group, a quinoline group, an isoquinoline group, a quinoxaline group, a quinazoline group, a phenanthroline group, a benzofuran group, a benzothiophene group, a fluorene group, a carbazole group, a dibenzofuran group, a dibenzothiophene group, a dibenzosilole group, an azafluorene group, an azacarbazole group, an azadibenzofuran group, an azadibenzothiophene group, or an azadibenzosilole group; and/or
wherein CY₃ is a pyridine group, a pyrimidine group, a pyridazine group, a pyrazine group, a triazine group, a quinoline group, an isoquinoline group, a quinoxaline group, or a quinazoline group.

4. The organometallic compound of any of claims 1-3, wherein R₁₀, R₂₀, R₃₀, and R₄₁ to R₅₀ are each independently:
hydrogen, deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a C₁-C₂₀ alkyl group, or a C₁-C₂₀ alkoxy group;
a C₁-C₂₀ alkyl group or a C₁-C₂₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, or a combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, or a combination thereof; or
-Si(Q₁)(Q₂)(Q₃), -Ge(Q₁)(Q₂)(Q₃), -N(Q₄)(Q₅), -B(Q₆)(Q₇), or -P(=O)(Q₈)(Q₉),
wherein Q₁ to Q₉ are each independently:
-CH₃, -CD₃, -CD₂H, -CDH₂, -CH₂CH₃, -CH₂CD₃, -CH₂CD₂H, -CH₂CDH₂, - CHDCH₃, -CHDCD₂H, -CHDCDH₂, -CHDCD₃, -CD₂CD₃, -CD₂CD₂H, or -CD₂CDH₂;
an n-propyl group, an isopropyl group, an n-butyl group, an isobutyl group, a see-butyl group, a tert-butyl group, an n-pentyl group, an isopentyl group, a sec-pentyl group, a tert-pentyl group, a phenyl group, or a naphthyl group; or
an n-propyl group, an isopropyl group, an n-butyl group, an isobutyl group, a see-butyl group, a tert-butyl group, an n-pentyl group, an isopentyl group, a sec-pentyl group, a tert-pentyl group, a phenyl group, or a naphthyl group, each substituted with deuterium, a C₁-C₁₀ alkyl group, a phenyl group, or a combination thereof.

5. The organometallic compound of any of claims 1-4, wherein R₁₀, R₂₀, R₃₀, and R₄₁ to R₅₀ are each independently:
hydrogen, deuterium, -F, -Cl, -Br, -I, -CD₃, -CD₂H, -CDH₂, -CF₃, -CF₂H, -CFH₂, a C₁-C₆₀ alkyl group, a C₂-C₆₀ alkenyl group, a C₂-C₆₀ alkynyl group, a C₁-C₆₀ alkoxy group, -Si(Q₁)(Q₂)(Q₃), or -Ge(Q₁)(Q₂)(Q₃); or
a group represented by one of Formulae 9-1 to 9-61, 9-201 to 9-237, 10-1 to 10-129, or 10-201 to 10-350: 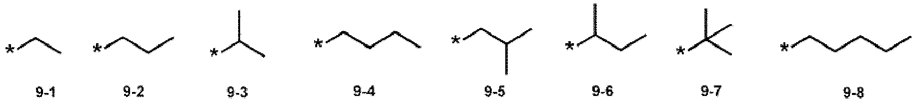 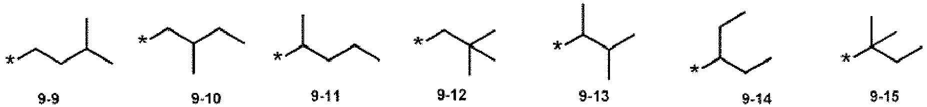 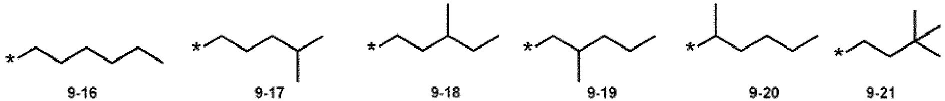 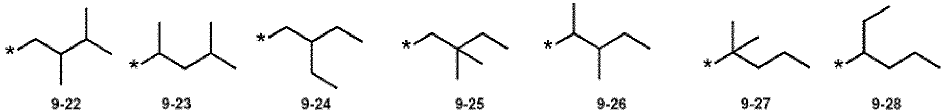 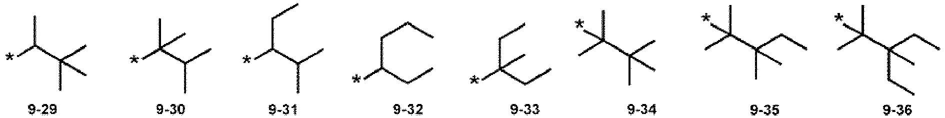 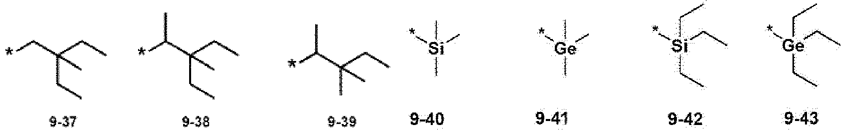 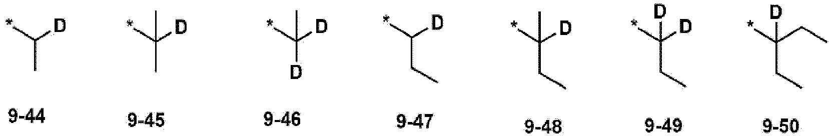 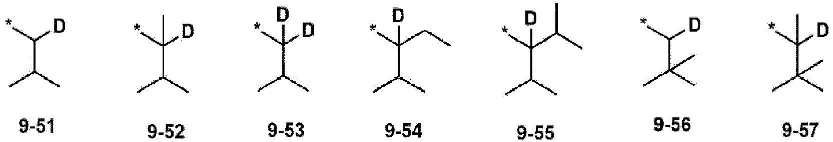 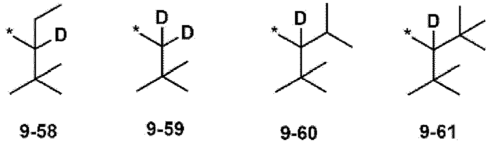 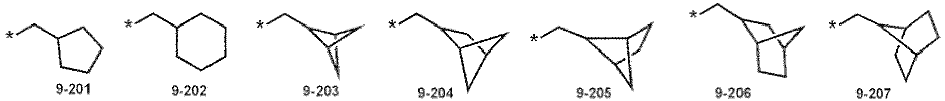 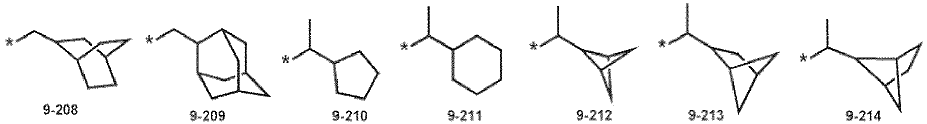 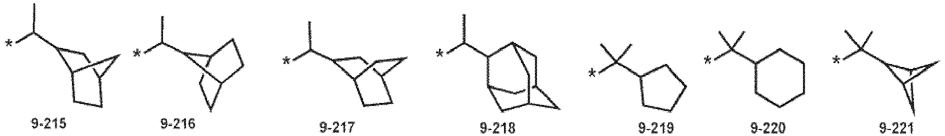 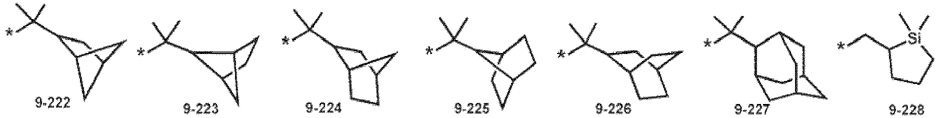 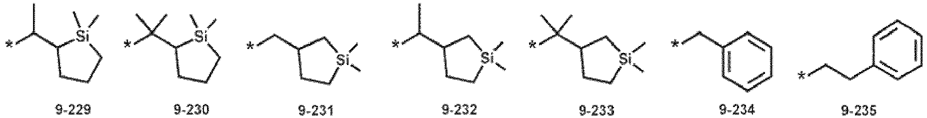 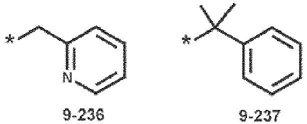 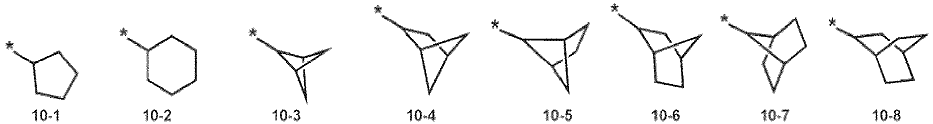 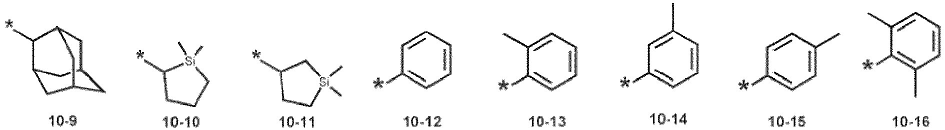 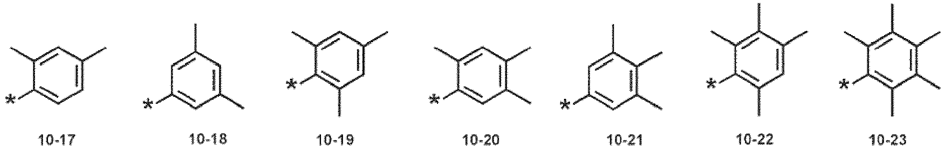 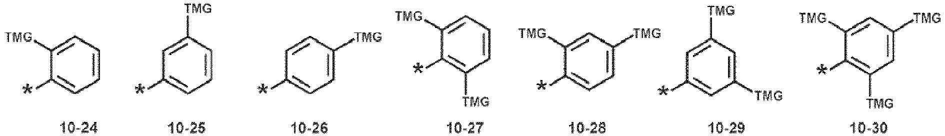 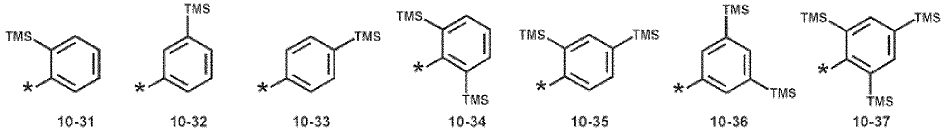 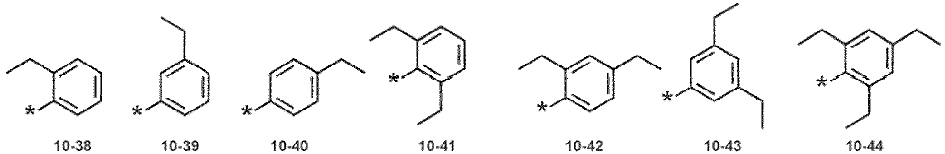 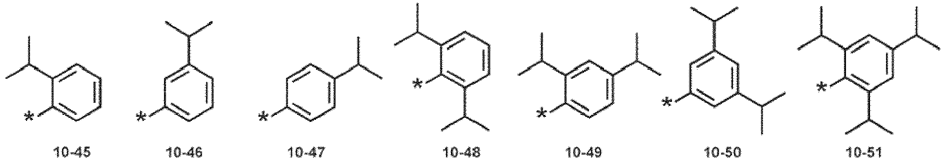 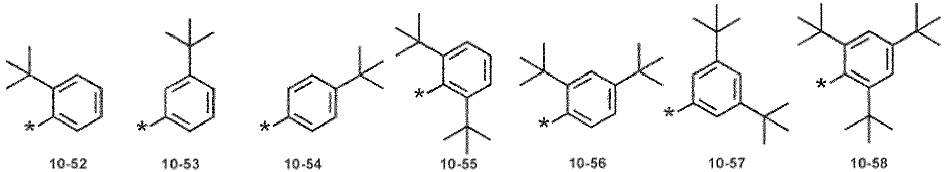 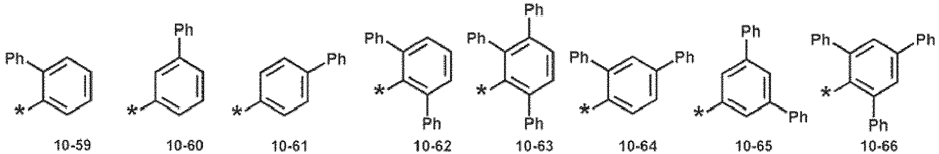 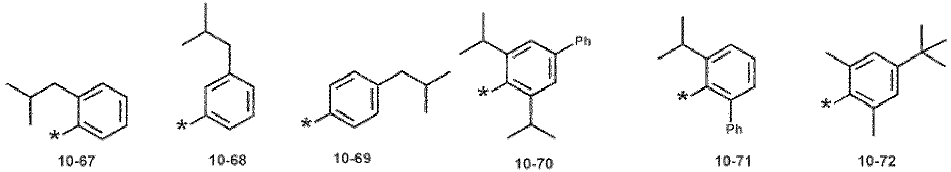 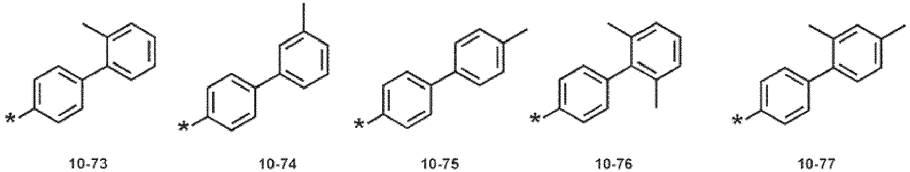 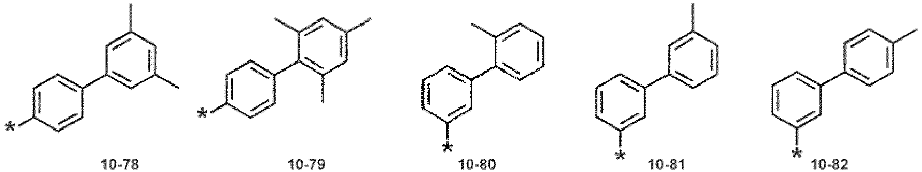 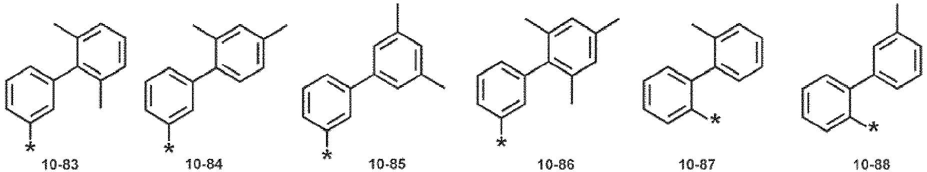 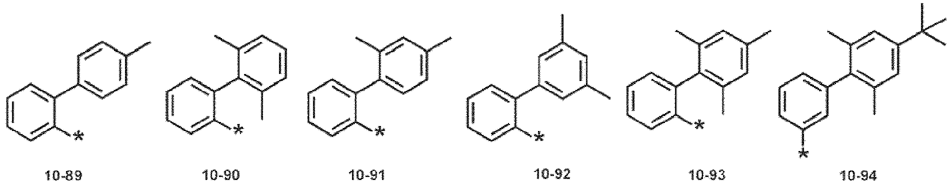 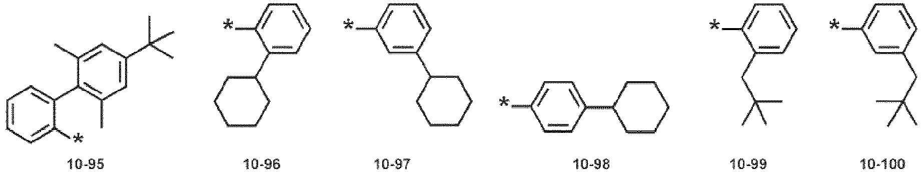 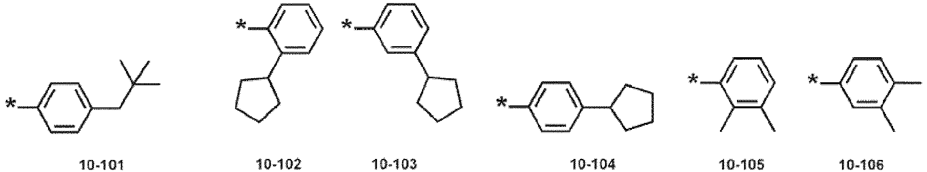 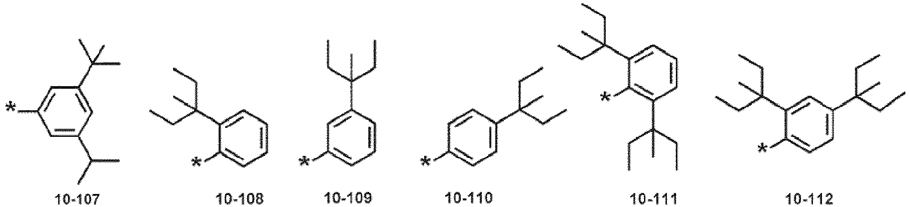 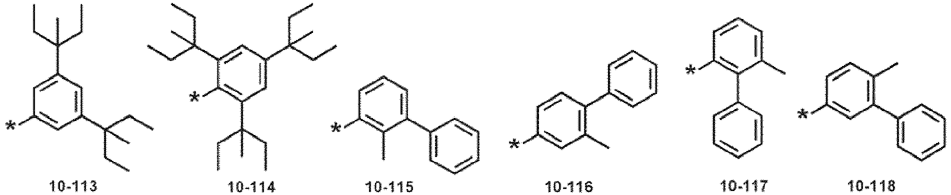 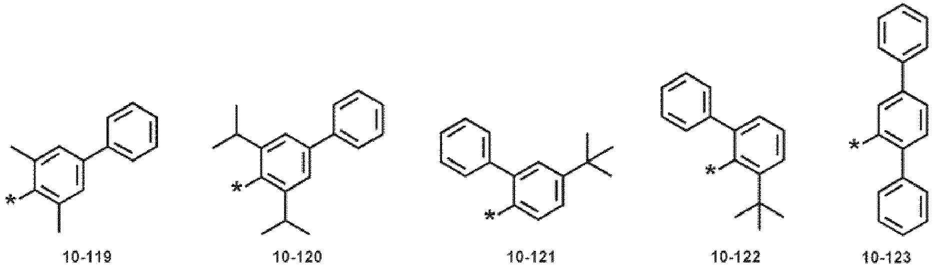 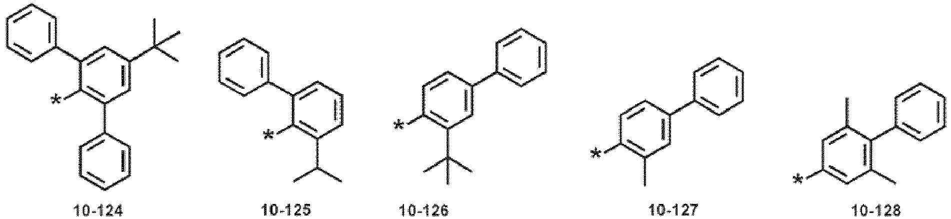 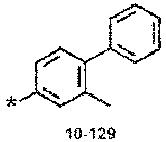 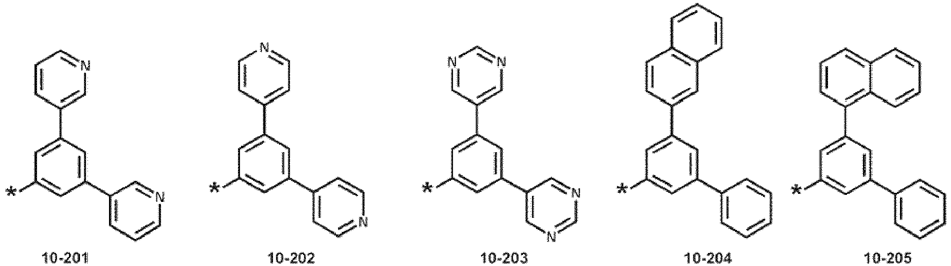 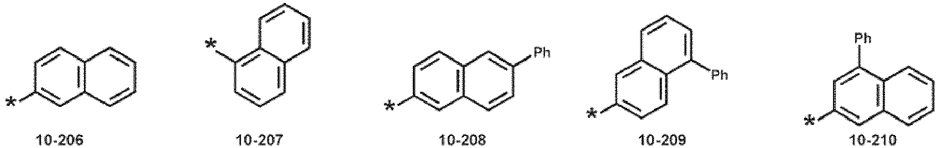 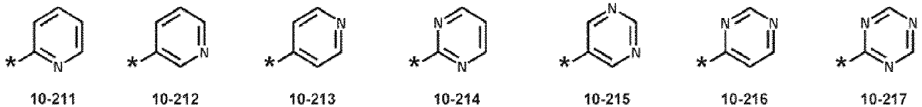 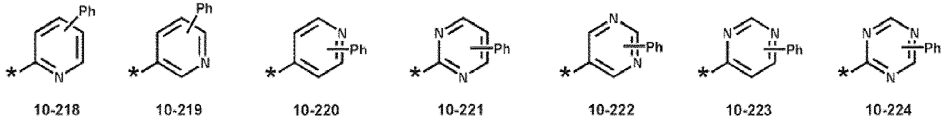 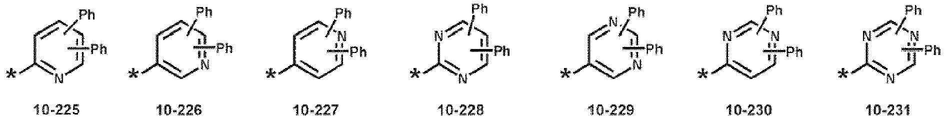 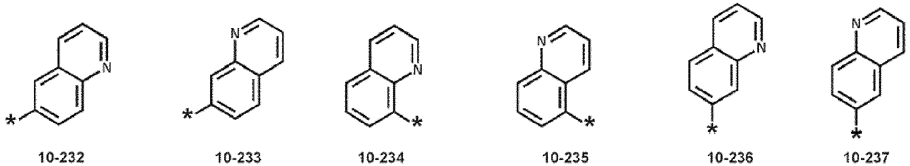 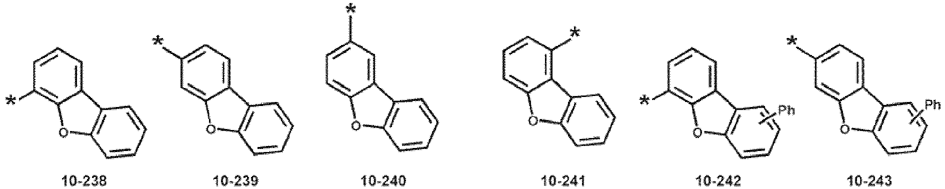 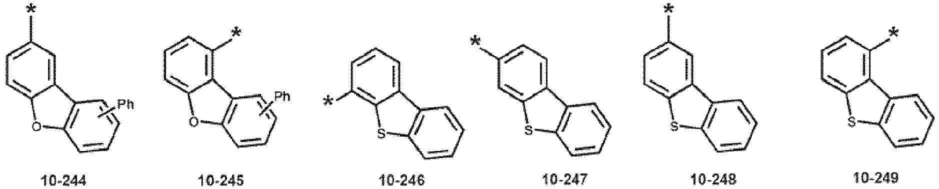 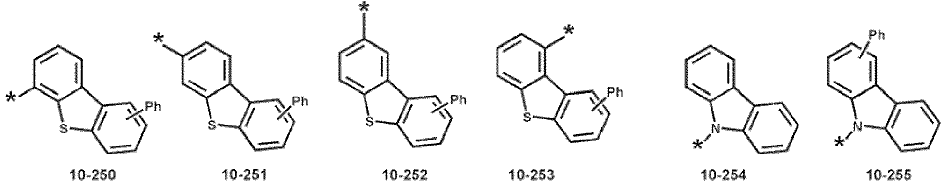 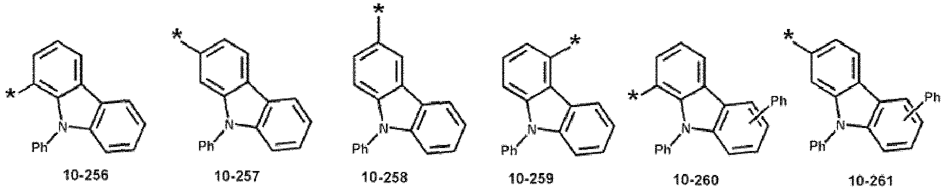 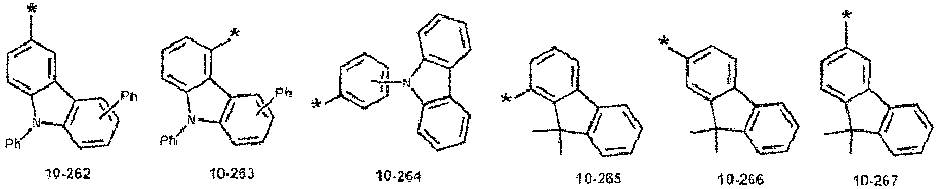 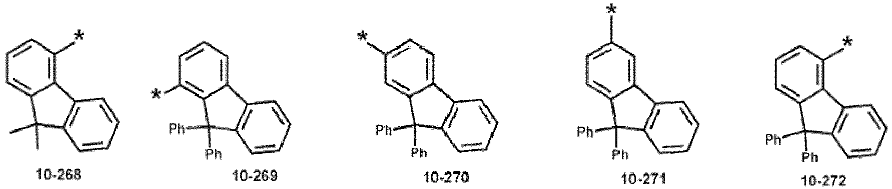 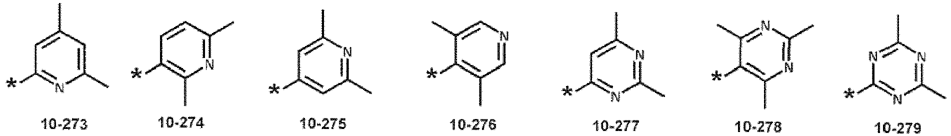 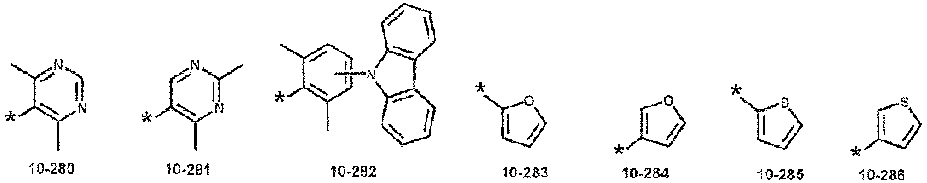 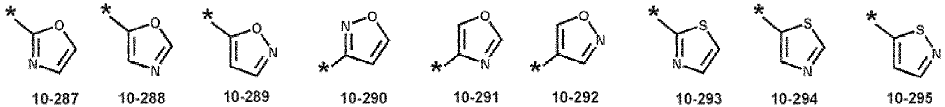 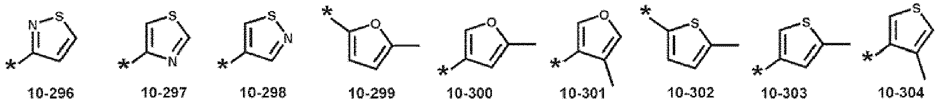 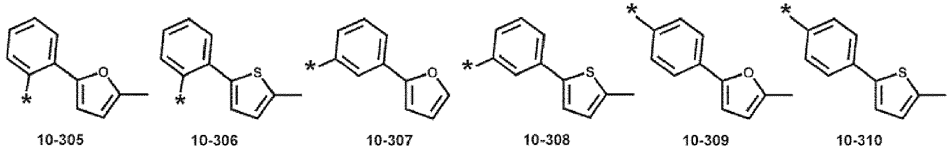 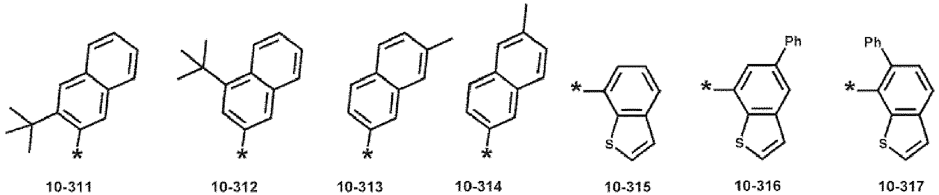 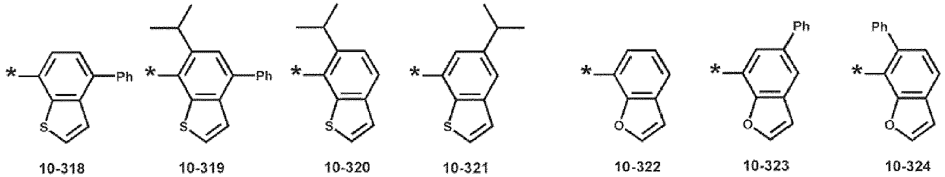 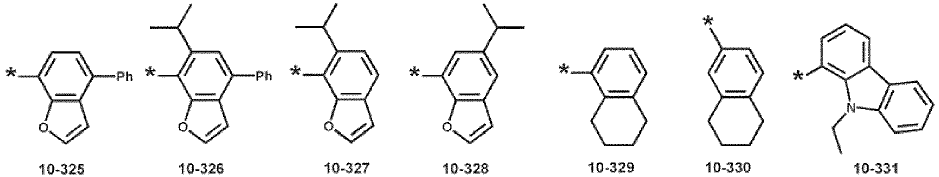 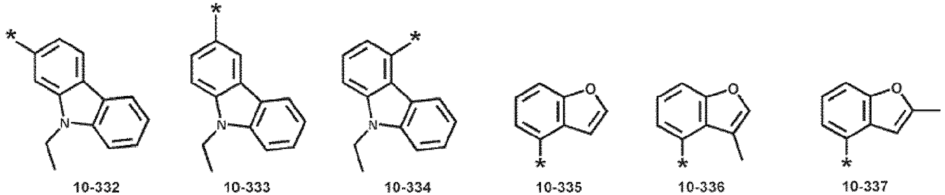 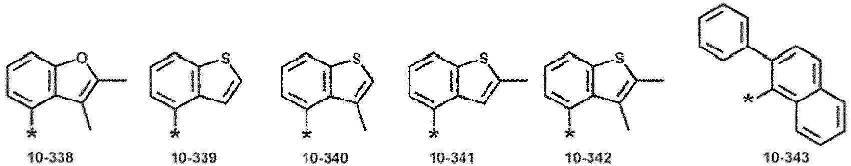 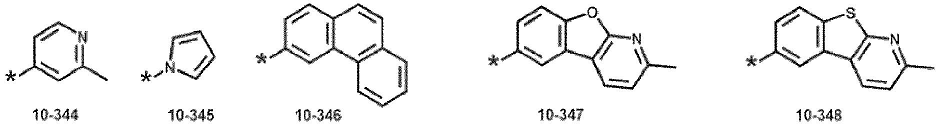 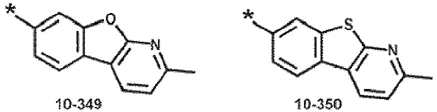
* in Formula 9-1 to 9-61, 9-201 to 9-237, 10-1 to 10-129, and 10-201 to 10-350 indicates a binding site to a neighboring atom, Ph is a phenyl group, TMS is a trimethylsilyl group, and TMG is a trimethylgermyl group.

6. The organometallic compound of any of claims 1-5, wherein the organometallic compound comprises one, two, three, or four germanyl groups; and/or
wherein at least one of R₃₀(s) in the number of b30 is -Ge(Q₁)(Q₂)(Q₃).

7. The organometallic compound of any of claims 1-6, wherein CY₁ is represented by one of Formulae 1-1 to 1-16: 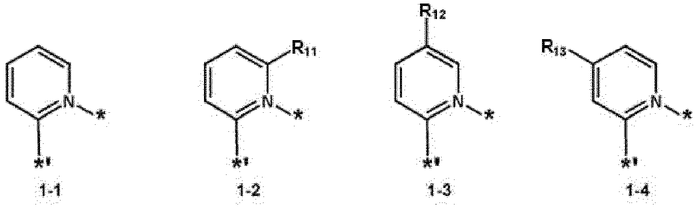 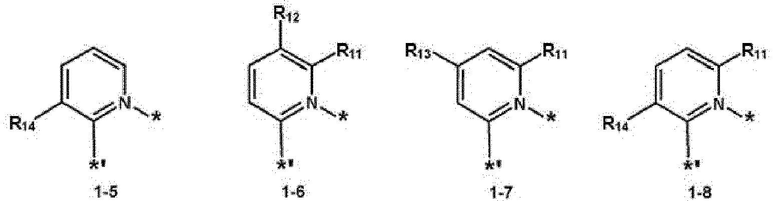 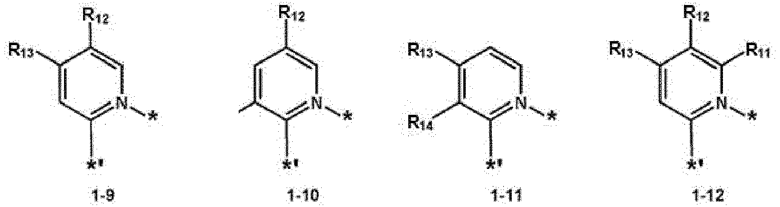 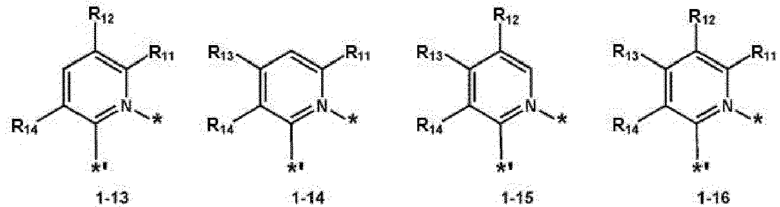 wherein, in Formulae 1-1 to 1-16,
R₁₁ to R₁₄ are each independently deuterium, -F, -Cl, -Br, -I, -CD₃, -CD₂H, - CDH₂, -CF₃, -CF₂H, -CFH₂, a methyl group, an ethyl group, an n-propyl group, an isopropyl group, an n-butyl group, an isobutyl group, a see-butyl group, a tert-butyl group, an n-pentyl group, an isopentyl group, a 2-methylbutyl group, a sec-pentyl group, a tert-pentyl group, a neo-pentyl group, 3-pentyl group, 3-methyl-2-butyl group, a phenyl group, a biphenyl group, a naphthyl group, -Si(Q₁)(Q₂)(Q₃), or - Ge(Q₁)(Q₂)(Q₃),
Q₁ to Q₃ are each independently:
-CH₃, -CD₃, -CD₂H, -CDH₂, -CH₂CH₃, -CH₂CD₃, -CH₂CD₂H, -CH₂CDH₂, - CHDCH₃, -CHDCD₂H, -CHDCDH₂, -CHDCD₃, -CD₂CD₃, -CD₂CD₂H, or -CD₂CDH₂;
an n-propyl group, an isopropyl group, an n-butyl group, an isobutyl group, a see-butyl group, a tert-butyl group, an n-pentyl group, an isopentyl group, a sec-pentyl group, a tert-pentyl group, a phenyl group, or a naphthyl group; or
an n-propyl group, an isopropyl group, an n-butyl group, an isobutyl group, a see-butyl group, a tert-butyl group, an n-pentyl group, an isopentyl group, a sec-pentyl group, a tert-pentyl group, a phenyl group, or a naphthyl group, each substituted with deuterium, a C₁-C₁₀ alkyl group, a phenyl group, or a combination thereof, and
* and *' each indicate a binding site to a neighboring atom; and/or
wherein CY₂ is represented by one of Formulae 2-1 to 2-16: 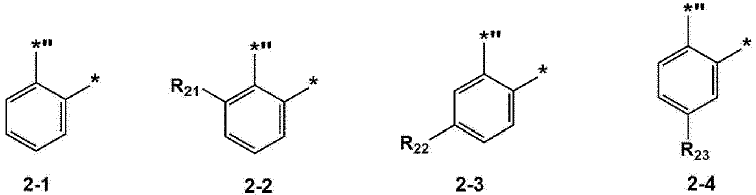 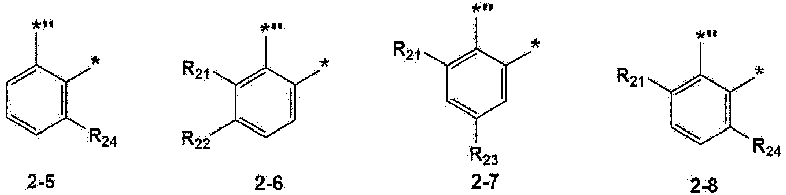 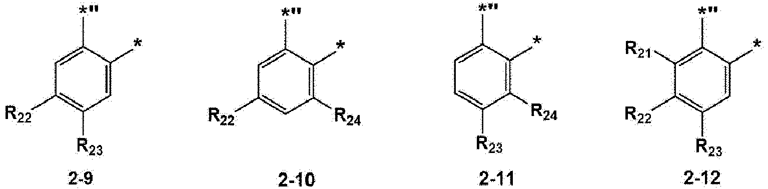 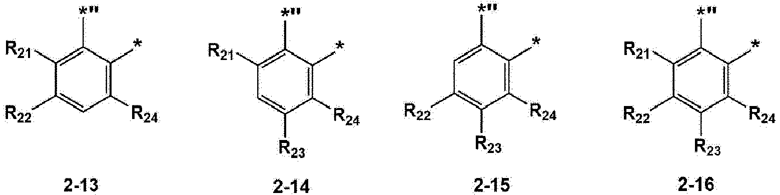
wherein, in Formulae 2-1 to 2-16,
R₂₁ to R₂₄ are each independently deuterium, -F, -Cl, -Br, -I, -CD₃, -CD₂H, - CDH₂, -CF₃, -CF₂H, -CFH₂, a methyl group, an ethyl group, an n-propyl group, an isopropyl group, an n-butyl group, an isobutyl group, a see-butyl group, a tert-butyl group, an n-pentyl group, an isopentyl group, a 2-methylbutyl group, a sec-pentyl group, a tert-pentyl group, a neo-pentyl group, 3-pentyl group, 3-methyl-2-butyl group, a phenyl group, a biphenyl group, a naphthyl group, -Si(Q₁)(Q₂)(Q₃), or - Ge(Q₁)(Q₂)(Q₃),
Q₁ to Q₃ are each independently:
-CH₃, -CD₃, -CD₂H, -CDH₂, -CH₂CH₃, -CH₂CD₃, -CH₂CD₂H, -CH₂CDH₂, - CHDCH₃, -CHDCD₂H, -CHDCDH₂, -CHDCD₃, -CD₂CD₃, -CD₂CD₂H, or -CD₂CDH₂;
an n-propyl group, an isopropyl group, an n-butyl group, an isobutyl group, a see-butyl group, a tert-butyl group, an n-pentyl group, an isopentyl group, a sec-pentyl group, a tert-pentyl group, a phenyl group, or a naphthyl group; or
an n-propyl group, an isopropyl group, an n-butyl group, an isobutyl group, a see-butyl group, a tert-butyl group, an n-pentyl group, an isopentyl group, a sec-pentyl group, a tert-pentyl group, a phenyl group, or a naphthyl group, each substituted with deuterium, a C₁-C₁₀ alkyl group, a phenyl group, or a combination thereof, and
* and *' each indicate a binding site to a neighboring atom.

8. The organometallic compound of any of claims 1-7, wherein CY₃ is represented by one of Formulae 3-1 to 3-40: 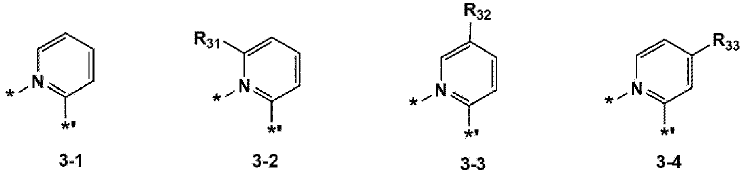 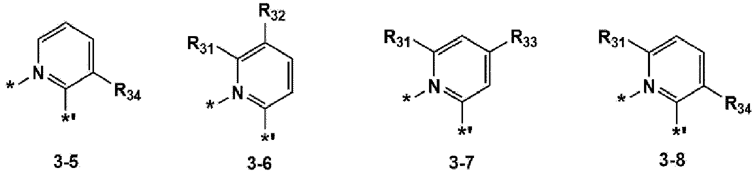 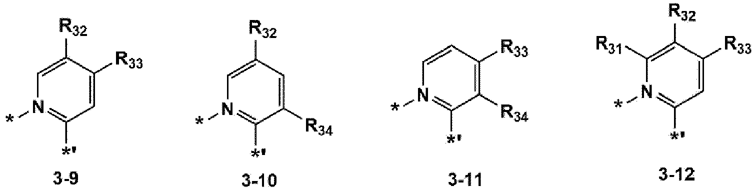 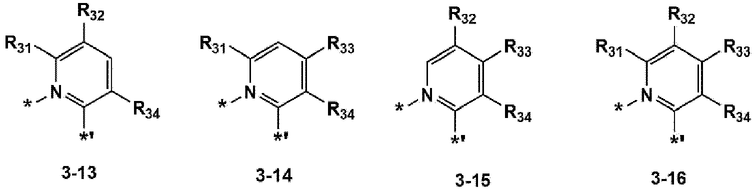 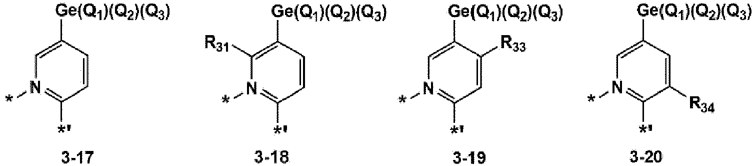 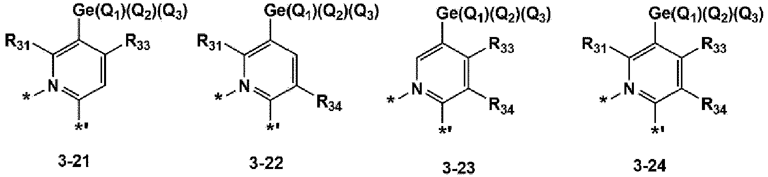 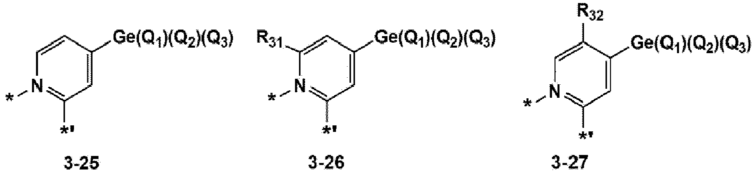 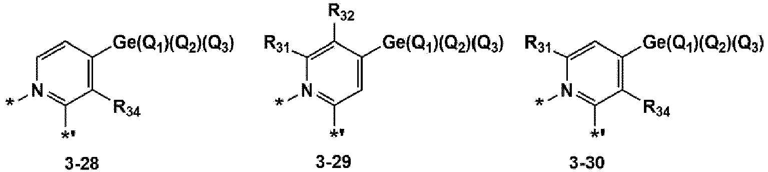 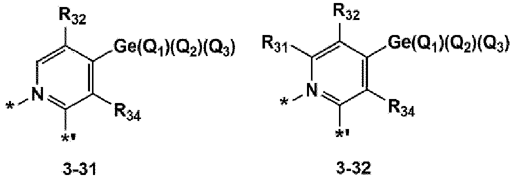 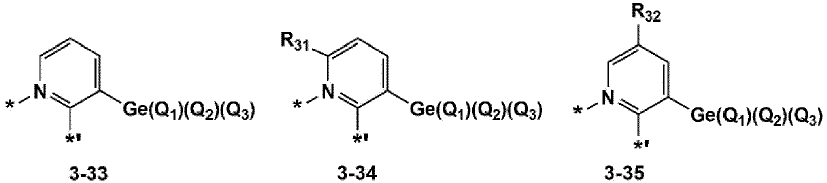 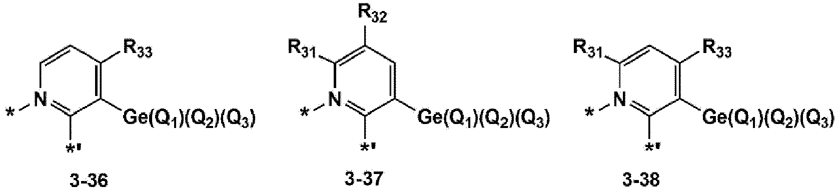 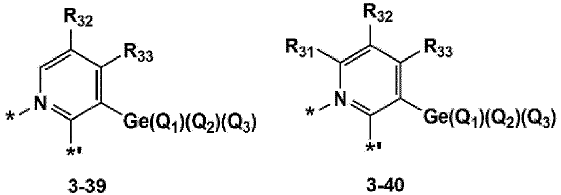 , wherein, in Formulae 3-1 to 3-40,
R₃₁ to R₃₄ are each independently deuterium, -F, -Cl, -Br, -I, -CD₃, -CD₂H, - CDH₂, -CF₃, -CF₂H, -CFH₂, a methyl group, an ethyl group, an n-propyl group, an isopropyl group, an n-butyl group, an isobutyl group, a see-butyl group, a tert-butyl group, an n-pentyl group, an isopentyl group, a 2-methylbutyl group, a sec-pentyl group, a tert-pentyl group, a neo-pentyl group, 3-pentyl group, 3-methyl-2-butyl group, a phenyl group, a biphenyl group, a naphthyl group, -Si(Q₁)(Q₂)(Q₃), or - Ge(Q₁)(Q₂)(Q₃),
Q₁ to Q₃ are each independently:
-CH₃, -CD₃, -CD₂H, -CDH₂, -CH₂CH₃, -CH₂CD₃, -CH₂CD₂H, -CH₂CDH₂, - CHDCH₃, -CHDCD₂H, -CHDCDH₂, -CHDCD₃, -CD₂CD₃, -CD₂CD₂H, or -CD₂CDH₂;
an n-propyl group, an isopropyl group, an n-butyl group, an isobutyl group, a see-butyl group, a tert-butyl group, an n-pentyl group, an isopentyl group, a sec-pentyl group, a tert-pentyl group, a phenyl group, or a naphthyl group; or
an n-propyl group, an isopropyl group, an n-butyl group, an isobutyl group, a see-butyl group, a tert-butyl group, an n-pentyl group, an isopentyl group, a sec-pentyl group, a tert-pentyl group, a phenyl group, or a naphthyl group, each substituted with deuterium, a C₁-C₁₀ alkyl group, a phenyl group, or a combination thereof, and
* and *' each indicate a binding site to a neighboring atom.

9. The organometallic compound of any of claims 1-8, wherein the organometallic compound is represented by Formula 11-1: 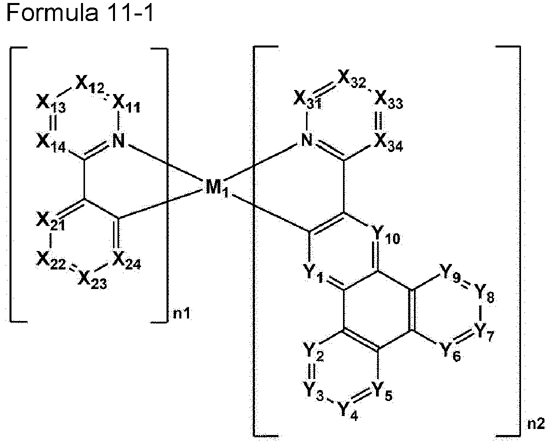 wherein, in Formula 11-1,
M₁, n1, n2, and Y₁ to Y₁₀ are each the same as described in claim 1,
X₁₁ is C(R₁₁) or N, X₁₂ is C(R₁₂) or N, X₁₃ is C(R₁₃) or N, and X₁₄ is C(R₁₄) or N,
X₂₁ is C(R₂₁) or N, X₂₂ is C(R₂₂) or N, X₂₃ is C(R₂₃) or N, and X₂₄ is C(R₂₄) or N,
X₃₁ is C(R₃₁) or N, X₃₂ is C(R₃₂) or N, X₃₃ is C(R₃₃) or N, and X₃₄ is C(R₃₄) or N,
R₁₁ to R₁₄ are each independently the same as described in connection with R₁₀ in claim 1,
R₂₁ to R₂₄ are each independently the same as described in connection with R₂₀ in claim 1,
R₃₁ to R₃₄ are each independently the same as described in connection with R₃₀ in claim 1,
two or more of R₁₁ to R₁₄ are optionally linked to each other to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more of R₂₁ to R₂₄ are optionally linked to each other to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more of R₃₁ to R₃₄ are optionally linked to each other to form a C₅-C₃₀ carbocyclic group that is unsubstituted or substituted with at least one R₁₀ₐ or a C₁-C₃₀ heterocyclic group that is unsubstituted or substituted with at least one R₁₀ₐ, and
R₁₀ₐ is the same as described in connection with R₁₀.

10. The organometallic compound of claim 1, wherein the organometallic compound is one of Compounds 1 to 36:

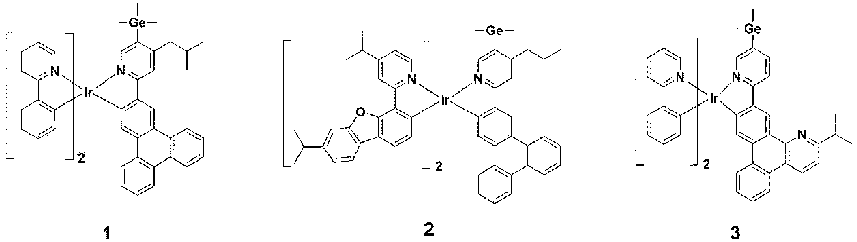

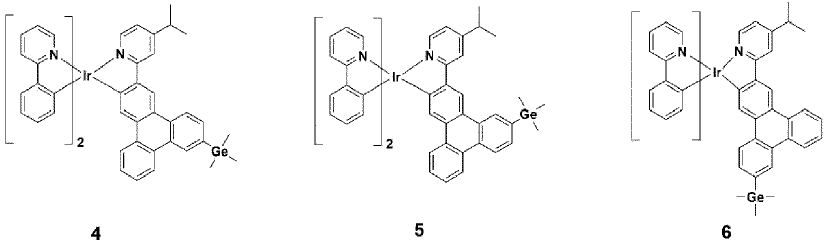

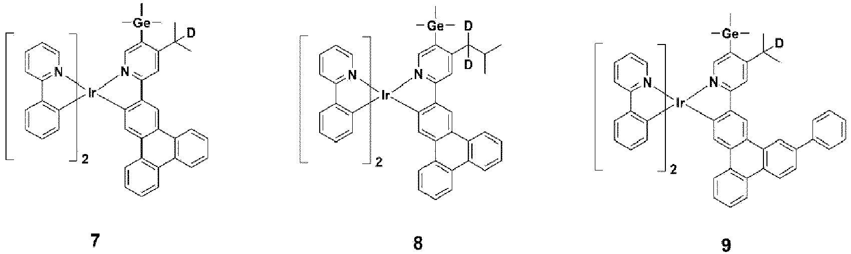

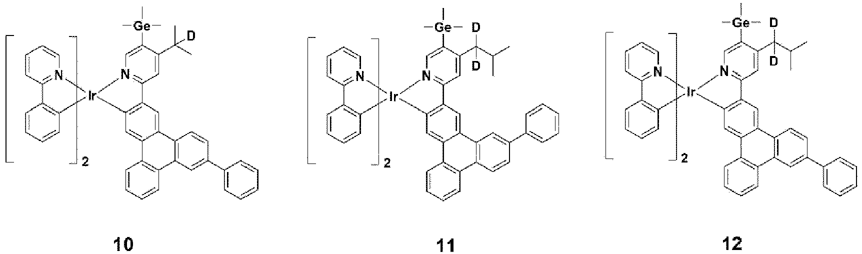

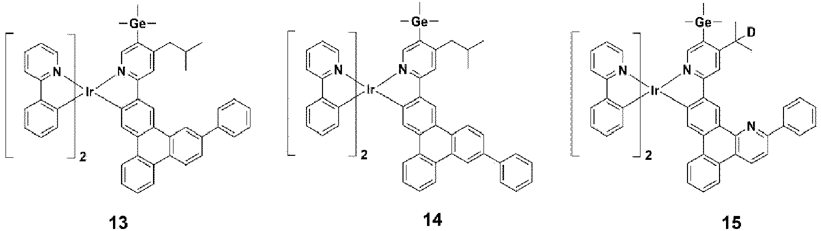

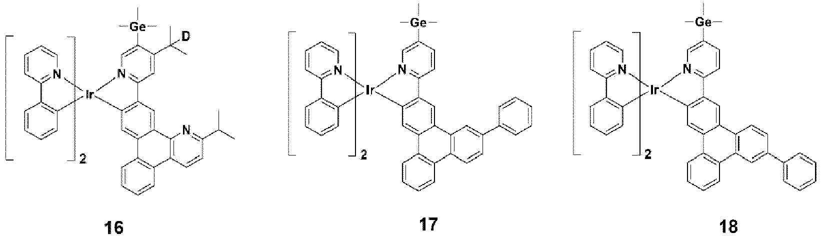

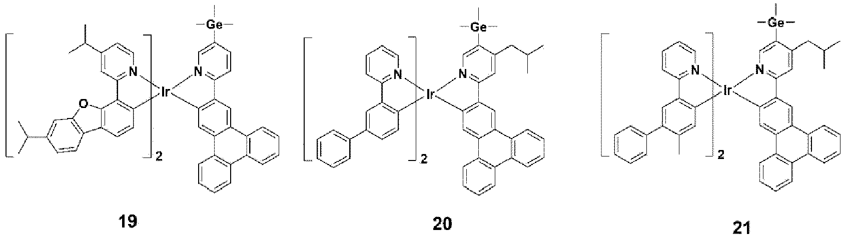

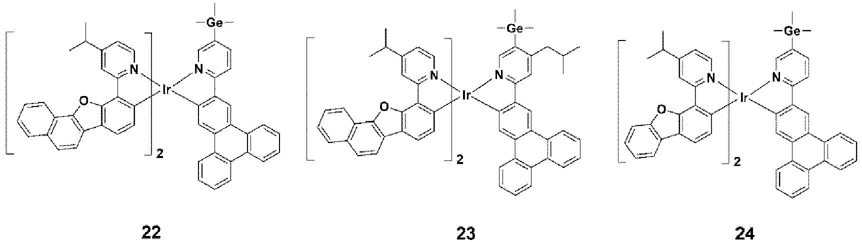

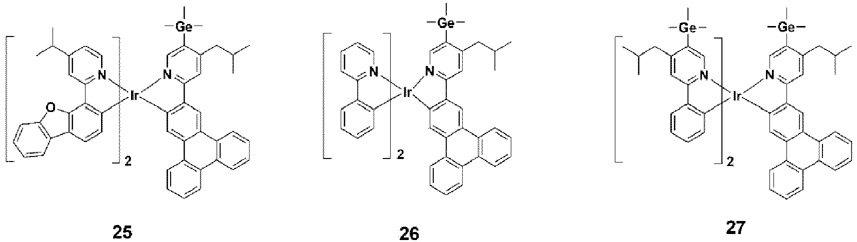

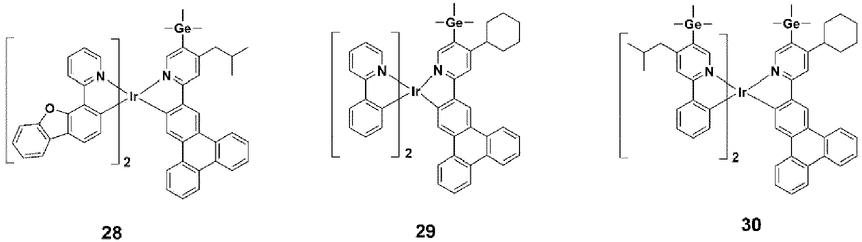

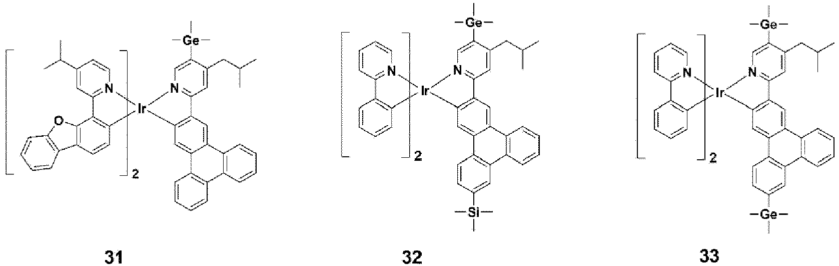

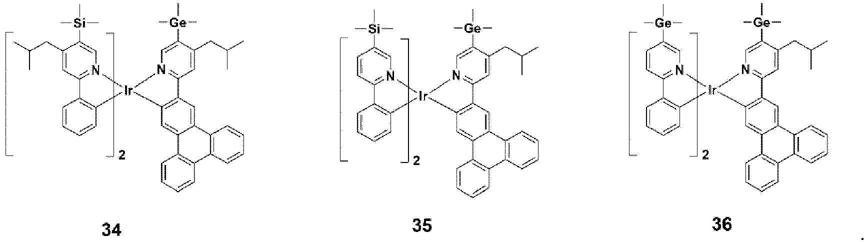

11. An organic light-emitting device comprising:
a first electrode;
a second electrode; and
an organic layer located between the first electrode and the second electrode and comprising an emission layer,
wherein the organic layer comprises the organometallic compound of any of claims 1-10.

12. The organic light-emitting device of claim 11, wherein the organometallic compound is included in the emission layer.

13. The organic light-emitting device of claim 12, wherein the emission layer further comprises a host and the amount of the host is greater than the amount of the organometallic compound; and/or
wherein the emission layer emits green light having a maximum emission wavelength of 500 nm to 600 nm.

14. The organic light-emitting device of claims 12 or 13, wherein the first electrode is an anode,
the second electrode is a cathode,
the organic layer further comprises a hole transport region located between the first electrode and the emission layer and an electron transport region located between the emission layer and the second electrode, wherein
the hole transport region comprises a hole injection layer, a hole transport layer, an electron blocking layer, a buffer layer, or a combination thereof, and
the electron transport region comprises a hole blocking layer, an electron transport layer, an electron injection layer, or a combination thereof.

15. A diagnostic composition comprising the organometallic compound of any of claims 1-10.

## Patentansprüche

1. Organometallverbindung, dargestellt durch Formel 1:
Formel 1 M₁(Ln₁)ₙ₁(Ln₂)ₙ₂
wobei in Formel 1
M₁ ein Übergangsmetall ist,
Ln₁ ein Ligand ist, dargestellt durch Formel 1-1,
Ln₂ ein Ligand ist, dargestellt durch Formel 1-2,
n1 0, 1 oder 2 ist,
n2 1, 2 oder 3 ist, 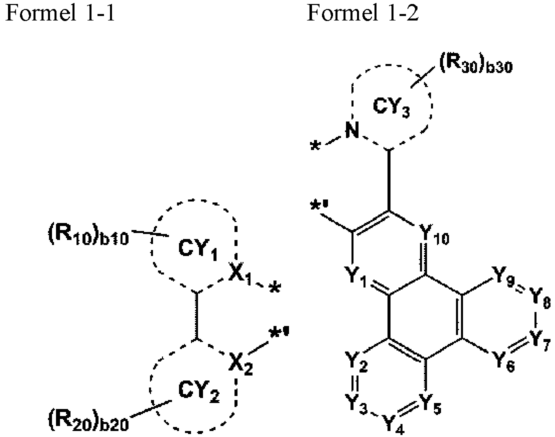
wobei in Formeln 1-1 und 1-2,
X₁ C oder N ist, und X₂ C oder N ist,
Y₁ C(R₄₁) oder N ist, Y₂ C(R₄₂) oder N ist, Y₃ C(R₄₃) oder N ist, Y₄ C(R₄₄) oder N ist, Y₅ C(R₄₅) oder N ist, Y₆ C(R₄₆) oder N ist, Y₇ C(R₄₇) oder N ist, Y₈ C(R₄₈) oder N ist, Y₉ C(R₄₉) oder N ist und Y₁₀ C(R₅₀) oder N ist,
CY₁ and CY₂ jeweils unabhängig voneinander eine carbocyclische C₅-C₃₀-Gruppe oder eine heterocyclische C₁-C₃₀-Gruppe sind,
CY₃ eine N-haltige heterocyclische C₁-C₃₀-Gruppe ist,
R₁₀, R₂₀, R₃₀ und R₄₁ bis R₅₀ jeweils unabhängig Wasserstoff, Deuterium, -F, -Cl, -Br, -I, - SF₅, eine Hydroxygruppe, eine Cyangruppe, eine Nitrogruppe, eine Aminogruppe, eine Amidingruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine substituierte oder unsubstituierte C₁-C₆₀-Alkylgruppe, eine substituierte oder unsubstituierte C₂-C₆₀-Alkenylgruppe, eine substituierte oder unsubstituierte C₂-C₆₀-Alkinylgruppe, eine substituierte oder unsubstituierte C₁-C₆₀-Alkoxygruppe, eine substituierte oder unsubstituierte C₃-C₁₀-Cycloalkylgruppe, eine substituierte oder unsubstituierte C₁-C₁₀-Heterocycloalkylgruppe, eine substituierte oder unsubstituierte C₃-C₁₀-Cycloalkenylgruppe, eine substituierte oder unsubstituierte C₂-C₁₀-Heterocycloalkenylgruppe, eine substituierte oder unsubstituierte C₆-C₆₀-Arylgruppe, eine substituierte oder unsubstituierte C₆-C₆₀-Aryloxygruppe, eine substituierte oder unsubstituierte C₆-C₆₀-Arylthiogruppe, eine substituierte oder unsubstituierte C₁-C₆₀-Heteroarylgruppe, eine substituierte oder unsubstituierte C₁-C₆₀-Heteroaryloxygruppe, eine substituierte oder unsubstituierte C₁-C₆₀-Heteroarylthiogruppe, eine substituierte oder unsubstituierte einwertige nicht-aromatische kondensierte polycyclische Gruppe, eine substituierte oder unsubstituierte einwertige nicht-aromatische kondensierte heteropolycyclische Gruppe, -Si(Q₁)(Q₂)(Q₃), -Ge(Q₁)(Q₂)(Q₃), -N(Q₄)(Q₅), - B(Q₆)(Q₇) oder - P(=O)(Q₈)(Q₉) sind,
mindestens einer der durch die Formeln 1-1 und 1-2 dargestellten Liganden mindestens eines von -Ge(Q₁)(Q₂)(Q₃) umfasst,
zwei oder mehrere aus einer Vielzahl von R₁₀ gegebenenfalls miteinander verbunden sind, um eine substituierte oder unsubstituierte carbocyclische C₅-C₃₀-Gruppe oder eine substituierte oder unsubstituierte heterocyclische C₁-C₃₀-Gruppe zu bilden,
zwei oder mehrere aus einer Vielzahl von R₂₀ gegebenenfalls miteinander verbunden sind, um eine substituierte oder unsubstituierte carbocyclische C₅-C₃₀-Gruppe oder eine substituierte oder unsubstituierte heterocyclische C₁-C₃₀-Gruppe zu bilden,
zwei oder mehrere aus einer Vielzahl von R₃₀ gegebenenfalls miteinander verbunden sind, um eine substituierte oder unsubstituierte carbocyclische C₅-C₃₀-Gruppe oder eine substituierte oder unsubstituierte heterocyclische C₁-C₃₀-Gruppe zu bilden,
zwei oder mehr benachbarte Gruppen von R₁₀, R₂₀, R₃₀ und R₄₁ bis R₅₀ gegebenenfalls miteinander verbunden sind, um eine substituierte oder unsubstituierte carbocyclische C₅-C₃₀-Gruppe oder eine substituierte oder unsubstituierte heterocyclische C₁-C₃₀-Gruppe zu bilden,
b10, b20 und b30 jeweils unabhängig voneinander 1, 2, 3, 4, 5, 6, 7, 8, 9 oder 10 sind,
mindestens ein Substituent der substituierten carbocyclischen C₅-C₃₀-Gruppe, der substituierten heterocyclischen C₁-C₃₀-Gruppe, der substituierten C₁-C₆₀-Alkyl-Gruppe, der substituierten C₂-C₆₀-Alkenyl-Gruppe, der substituierten C₂-C₆₀-Alkinyl-Gruppe, der substituierten C₁-C₆₀-Alkoxy-Gruppe, der substituierten C₃-C₁₀-Cycloalkyl-Gruppe, der substituierten C₁-C₁₀-Heterocycloalkyl-Gruppe, der substituierten C₃-C₁₀-Cycloalkenyl-Gruppe, der substituierten C₂-C₁₀-Heterocycloalkenylgruppe, der substituierten C₆-C₆₀-Arylgruppe, der substituierten C₆-C₆₀-Aryloxygruppe, der substituierten C₆-C₆₀-Arylthiogruppe, der substituierten C₁-C₆₀-Heteroarylgruppe, der substituierten C₁-C₆₀-Heteroaryloxygruppe, der substituierten C₁-C₆₀-Heteroarythiogruppe, der substituierten einwertigen nicht-aromatischen kondensierten polycyclischen Gruppe und der substituierten einwertigen nicht-aromatischen kondensierten heteropolycylischen Gruppe ist:
ein Deuterium, -F, -Cl, -Br, -I, -CD₃, -CD₂H, -CDH₂, -CF₃, -CF₂H, -CFH₂, eine Hydroxygruppe, eine Cyangruppe, eine Nitrogruppe, eine Aminogruppe, eine Amidingruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine C₁-C₆₀ Alkylgruppe, eine C₂-C₆₀ Alkenylgruppe, eine C₂-C₆₀ Alkinylgruppe oder eine C₁-C₆₀ Alkoxygruppe,
eine C₁-C₆₀ Alkylgruppe, eine C₂-C₆₀ Alkenylgruppe, eine C₂-C₆₀ Alkynylgruppe oder eine C₁- C₆₀ Alkoxygruppe, jeweils substituiert mit Deuterium, -F, - Cl, -Br, -I, -CD₃, -CD₂H, -CDH₂, -CF₃, -CF₂H, -CFH₂, einer Hydroxygruppe, einer Cyangruppe, einer Nitrogruppe, einer Aminogruppe, einer Amidingruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₃-C₁₀-Cycloalkylgruppe, einer C₁-C₁₀-Heterocycloalkylgruppe, einer C₃-C₁₀-Cycloalkenylgruppe, einer C₂-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer C₁-C₆₀-Heteroaryloxygruppe, einer C₁-C₆₀-Heteroarylthiogruppe, einer einwertigen nicht-aromatischen kondensierten polycyclischen Gruppe, einer einwertigen nicht-aromatischen kondensierten heteropolycyclischen Gruppe, - Si(Q₁₁)(Q₁₂)(Q₁₃), -Ge(Q₁₁)(Q₁₂)(Q₁₃), -N(Q₁₄)(Q₁₅), -B(Q₁₆)(Q₁₇), -P(=O)(Q₁₈)(Q₁₉) oder einer Kombination davon;
eine C₃-C₁₀-Cycloalkylgruppe, eine C₁-C₁₀-Heterocycloalkylgruppe, eine C₃-C₁₀-Cycloalkenylgruppe, eine C₂-C₁₀-Heterocycloalkenylgruppe, eine C₆-C₆₀-Arylgruppe, eine C₆-C₆₀-Aryloxygruppe, eine C₆-C₆₀-Arylthiogruppe, eine C₁-C₆₀-Heteroarylgruppe, eine C₁-C₆₀-Heteroaryloxygruppe, eine C₁-C₆₀-Heteroarylthiogruppe, eine einwertige nicht-aromatische kondensierte polycyclische Gruppe oder eine einwertige nicht-aromatische kondensierte heteropolycyclische Gruppe,
eine C₃-C₁₀-Cycloalkylgruppe, eine C₁-C₁₀-Heterocycloalkylgruppe, eine C₃-C₁₀-Cycloalkenylgruppe, eine C₂-C₁₀-Heterocycloalkenylgruppe, eine C₆-C₆₀-Arylgruppe, eine C₆-C₆₀-Aryloxygruppe, eine C₆-C₆₀-Arylthiogruppe, eine C₁-C₆₀-Heteroarylgruppe, eine einwertige nicht-aromatische kondensierte polycyclische Gruppe oder eine einwertige nicht-aromatische kondensierte heteropolycyclischen Gruppe, jeweils substituiert mit Deuterium, -F, -Cl, -Br, -I, - CD₃, -CD₂H, -CDH₂, -CF₃, -CF₂H, -CFH₂, eine Hydroxygruppe, eine Cyanogruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine C₁-C₆₀-Alkylgruppe, eine C₂-C₆₀-Alkenylgruppe, eine C₂-C₆₀-Alkinylgruppe, eine C₁-C₆₀-Alkoxygruppe, eine C₃-C₁₀-Cycloalkylgruppe, eine C₁-C₁₀-Heterocycloalkylgruppe, eine C₃-C₁₀-Cycloalkenylgruppe, eine C₂-C₁₀-Heterocycloalkenylgruppe, eine C₆-C₆₀-Arylgruppe, eine C₆-C₆₀-Aryloxygruppe, eine C₆-C₆₀-Arylthiogruppe, eine C₁-C₆₀-Heteroarylgruppe, eine C₁-C₆₀-Heteroaryloxygruppe, eine C₁-C₆₀-Heteroarylthiogruppe, eine einwertige nicht-aromatische kondensierte polycyclische Gruppe, eine einwertige nicht-aromatische kondensierte heteropolycyclische Gruppe, -Si(Q₂₁)(Q₂₂)(Q₂₃), - Ge(Q₂₁)(Q₂₂)(Q₂₃), -N(Q₂₄)(Q₂₅), -B(Q₂₆)(Q₂₇), -P(=O)(Q₂₈)(Q₂₉) oder eine Kombination davon, oder
-Si(Q₃₁)(Q₃₂)(Q₃₃), -Ge(Q₃₁)(Q₃₂)(Q₃₃), -N(Q₃₄)(Q₃₅), -B(Q₃₆)(Q₃₇) oder - P(=O)(Q₃₈)(Q₃₉),
wobei Q₁ bis Q₉, Q₁₁ bis Q₁₉, Q₂₁ bis Q₂₉, und Q₃₁ bis Q₃₉ jeweils unabhängig voneinander Wasserstoff, Deuterium, -F, -Cl, -Br, -I, eine Hydroxygruppe, eine Cyanogruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine substituierte oder unsubstituierte C₁-C₆₀-Alkylgruppe, eine substituierte oder unsubstituierte C₂-C₆₀-Alkenylgruppe, eine substituierte oder unsubstituierte C₂-C₆₀-Alkinylgruppe, eine substituierte oder unsubstituierte C₁-C₆₀-Alkoxygruppe, eine substituierte oder unsubstituierte C₃-C₁₀-Cycloalkylgruppe, eine substituierte oder unsubstituierte C₁-C₁₀-Heterocycloalkylgruppe, eine substituierte oder unsubstituierte C₃-C₁₀-Cycloalkenylgruppe, eine substituierte oder unsubstituierte C₂-C₁₀-Heterocycloalkenylgruppe, eine substituierte oder unsubstituierte C₆-C₆₀-Arylgruppe, eine substituierte oder unsubstituierte C₆-C₆₀-Aryloxygruppe, eine substituierte oder unsubstituierte C₆-C₆₀-Arylthiogruppe, eine substituierte oder unsubstituierte C₁- bisC₆₀-Heteroarylgruppe, eine C₁-bis C₆₀-Heteroaryloxygruppe, eine C₁-bis C₆₀-Heteroarylthiogruppe, eine substituierte oder unsubstituierte einwertige nicht-aromatische kondensierte polycyclische Gruppe oder eine substituierte oder unsubstituierte einwertige nicht-aromatische kondensierte heteropolycyclische Gruppe sind.

2. Organometallverbindung nach Anspruch 1, wobei M₁ Iridium (Ir), Platin (Pt), Osmium (Os), Titan (Ti), Zirkonium (Zr), Hafnium (Hf), Europium (Eu), Terbium (Tb), Thulium (Tm) oder Rhodium (Rh) ist;
wobei M₁ vorzugsweise Ir ist und die Summe von n1 und n2 3 ist.

3. Organometallverbindung nach Anspruch 1 oder 2, wobei CY₁ und CY₂ jeweils unabhängig eine Benzolgruppe, eine Naphthalingruppe, eine 1,2,3,4-Tetrahydronaphthalingruppe, eine Phenanthrengruppe, eine Pyridingruppe, eine Pyrimidingruppe, eine Pyrazingruppe, eine Pyridazingruppe, eine Triazingruppe, eine Chinolingruppe, eine Isochinolingruppe, eine Chinoxalingruppe, eine Chinazolin-Gruppe, eine Phenanthrolin-Gruppe, eine Benzofuran-Gruppe, eine Benzothiophen-Gruppe, eine Fluoren-Gruppe, eine Carbazol-Gruppe, eine Dibenzofuran-Gruppe, eine Dibenzothiophen-Gruppe, eine Dibenzosilol-Gruppe, eine Azafluoren-Gruppe, eine Azacarbazol-Gruppe, eine Azadibenzofuran-Gruppe, eine Azadibenzothiophen-Gruppe oder eine Azadibenzosilol-Gruppe sind, und/oder
wobei CY₃ eine Pyridingruppe, eine Pyrimidingruppe, eine Pyridazingruppe, eine Pyrazingruppe, eine Triazingruppe, eine Chinolingruppe, eine Isochinolingruppe, eine Chinoxalingruppe oder eine Chinazolingruppe ist.

4. Organometallverbindung nach einem der Ansprüche 1 bis 3, wobei R₁₀, R₂₀, R₃₀ und R₄₁ bis R₅₀ jeweils unabhängig voneinander sind:
Wasserstoff, Deuterium, -F, -Cl, -Br, -I, -CD₃, -CD₂H, -CDH₂, -CF₃, -CF₂H, -CFH₂, eine Hydroxygruppe, eine Cyanogruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, -SF₅, eine C₁-C₂₀-Alkylgruppe oder eine C₁-C₂₀-Alkoxygruppe;
eine C₁-C₂₀-Alkylgruppe oder eine C₁-C₂₀-Alkoxygruppe, jeweils substituiert mit Deuterium, -F, -Cl, -Br, -I, -CD₃, -CD₂H, -CDH₂, -CF₃, -CF₂H, -CFH₂, einer Hydroxygruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₁₀-Alkylgruppe, einer Cyclopentylgruppe, einer Cyclohexylgruppe, einer Cycloheptylgruppe, einer Cyclooctylgruppe, einer Adamantanylgruppe, einer Norbomanylgruppe, einer Norbornenylgruppe, einer Cyclopentenylgruppe, einer Cyclohexenylgruppe, einer Cycloheptenylgruppe, einer Phenylgruppe, einer Naphthylgruppe, einer Pyridinylgruppe, einer Pyrimidinylgruppe oder einer Kombination davon;
eine Cyclopentylgruppe, eine Cyclohexylgruppe, eine Cycloheptylgruppe, eine Cyclooctylgruppe, eine Adamantanylgruppe, eine Norbomanylgruppe, eine Norbornenylgruppe, eine Cyclopentenylgruppe, eine Cyclohexenylgruppe, eine Cycloheptenylgruppe, eine Phenylgruppe, eine Naphthylgruppe, eine Fluorenylgruppe, eine Phenanthrenylgruppe, eine Anthracenylgruppe, eine Fluoranthenylgruppe, eine Triphenylenylgruppe, eine Pyrenylgruppe, eine Chrysenylgruppe, eine Pyrrolylgruppe, eine Thiophenylgruppe, eine Furanylgruppe, eine Imidazolylgruppe, eine Pyrazolylgruppe, eine Thiazolylgruppe, eine Isothiazolylgruppe, eine Oxazolylgruppe, eine Isoxazolylgruppe, eine Pyridinylgruppe, eine Pyrazinylgruppe, eine Pyrimidinylgruppe, eine Pyridazinylgruppe, eine Isoindolylgruppe, eine Indolylgruppe, eine Indazolylgruppe, eine Purinylgruppe, eine Chinolinylgruppe, eine Isochinolinylgruppe, eine Benzochinolinylgruppe, eine Chinoxalinylgruppe, eine Chinazolinylgruppe, eine Cinnolinylgruppe, eine Carbazolylgruppe, eine Phenanthrolinylgruppe, eine Benzimidazolylgruppe, eine Benzofuranylgruppe, eine Benzothiophenylgruppe, eine Isobenzothiazolylgruppe, eine Benzoxazolylgruppe, eine Isobenzoxazolylgruppe, eine Triazolylgruppe, eine Tetrazolylgruppe, eine Oxadiazolylgruppe, eine Triazinylgruppe, eine Dibenzofuranylgruppe, eine Dibenzothiophenylgruppe, eine Benzocarbazolylgruppe, eine Dibenzocarbazolylgruppe, eine Imidazopyridinylgruppe oder eine Imidazopyrimidinylgruppe;
eine Cyclopentylgruppe, eine Cyclohexylgruppe, eine Cycloheptylgruppe, eine Cyclooctylgruppe, eine Adamantanylgruppe, eine Norbomanylgruppe, eine Norbornenylgruppe, eine Cyclopentenylgruppe, eine Cyclohexenylgruppe, eine Cycloheptenylgruppe, eine Phenylgruppe, eine Naphthylgruppe, eine Fluorenylgruppe, eine Phenanthrenylgruppe, eine Anthracenylgruppe, eine Fluoranthenylgruppe, eine Triphenylenylgruppe, eine Pyrenylgruppe, eine Chrysenylgruppe, eine Pyrrolylgruppe, eine Thiophenylgruppe, eine Furanylgruppe, eine Imidazolylgruppe, eine Pyrazolylgruppe, eine Thiazolylgruppe, eine Isothiazolylgruppe, eine Oxazolylgruppe, eine Isoxazolylgruppe, eine Pyridinylgruppe, eine Pyrazinylgruppe, eine Pyrimidinylgruppe, eine Pyridazinylgruppe, eine Isoindolylgruppe, eine Indolylgruppe, eine Indazolylgruppe, eine Purinylgruppe, eine Chinolinylgruppe, eine Isochinolinylgruppe, eine Benzochinolinylgruppe, eine Chinoxalinylgruppe, eine Chinazolinylgruppe, eine Cinnolinylgruppe, eine Carbazolylgruppe, eine Phenanthrolinylgruppe, eine Benzimidazolylgruppe, eine Benzofuranylgruppe, eine Benzothiophenylgruppe, eine Isobenzothiazolylgruppe, eine Benzoxazolylgruppe, eine Isobenzoxazolylgruppe, eine Triazolylgruppe, eine Tetrazolylgruppe, eine Oxadiazolylgruppe, eine Triazinylgruppe, eine Dibenzofuranylgruppe, eine Dibenzothiophenylgruppe, eine Benzocarbazolylgruppe, eine Dibenzocarbazolylgruppe, eine Imidazopyridinylgruppe oder eine Imidazopyrimidinylgruppe, jeweils substituiert mit Deuterium,-F, -Cl, -Br, -I, -CD₃, -CD₂H, -CDH₂, -CF₃, -CF₂H, -CFH₂, einer Hydroxygruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₂₀-Alkylgruppe, einer C₁-C₂₀-Alkoxygruppe, einer Cyclopentylgruppe, einer Cyclohexylgruppe, einer Cycloheptylgruppe, einer Cyclooctylgruppe, einer Adamantanylgruppe, einer Norbomanylgruppe, einer Norbornenylgruppe, einer Cyclopentenylgruppe, einer Cyclohexenylgruppe, einer Cycloheptenylgruppe, einer Phenylgruppe, einer Naphthylgruppe, einer Fluorenylgruppe, einer Phenanthrenylgruppe, einer Anthracenylgruppe, einer Fluoranthenylgruppe, einer Triphenylenylgruppe, einer Pyrenylgruppe, einer Chrysenylgruppe, einer Pyrrolylgruppe, einer Thiophenylgruppe, einer Furanylgruppe, einer Imidazolylgruppe, einer Pyrazolylgruppe, einer Thiazolylgruppe, einer Isothiazolylgruppe, einer Oxazolylgruppe, einer Isoxazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Chinolinylgruppe, einer Isochinolinylgruppe, einer Benzochinolinylgruppe, einer Chinoxalinylgruppe, einer Chinazolinylgruppe, einer Cinnolinylgruppe, einer Carbazolylgruppe, einer Phenanthrolinylgruppe, einer Benzimidazolylgruppe, einer Benzofuranylgruppe, einer Benzothiophenylgruppe, einer Isobenzothiazolylgruppe, einer Benzoxazolylgruppe, einer Isobenzoxazolylgruppe, einer Triazolylgruppe, einer Tetrazolylgruppe, einer Oxadiazolylgruppe, einer Triazinylgruppe, einer Dibenzofuranylgruppe, einer Dibenzothiophenylgruppe, einer Benzocarbazolylgruppe, einer Dibenzocarbazolylgruppe, einer Imidazopyridinylgruppe, einer Imidazopyrimidinylgruppe oder einer Kombination davon, oder
-Si(Q₁)(Q₂)(Q₃), -Ge(Q₁)(Q₂)(Q₃), -N(Q₄)(Q₅), -B(Q₆)(Q₇) oder -P(=O)(Q₈)(Q₉),
wobei Q₁ bis Q₉ jeweils unabhängig voneinander sind:
-CH₃, -CD₃, -CD₂H, -CDH₂, -CH₂CH₃, -CH₂CD₃, -CH₂CD₂H, -CH₂CDH₂, -CHDCH₃, - CHDCD₂H, -CHDCDH₂, -CHDCD₃, -CD₂CD₃, -CD₂CD₂H oder -CD₂CDH₂,
eine n-Propylgruppe, eine Isopropylgruppe, eine n-Butylgruppe, eine Isobutylgruppe, eine sec-Butylgruppe, eine tert-Butylgruppe, eine n-Pentylgruppe, eine Isopentylgruppe, eine sec-Pentylgruppe, eine tert-Pentylgruppe, eine Phenylgruppe oder eine Naphthylgruppe, oder
eine n-Propylgruppe, eine Isopropylgruppe, eine n-Butylgruppe, eine Isobutylgruppe, eine sec-Butylgruppe, eine tert-Butylgruppe, eine n-Pentylgruppe, eine Isopentylgruppe, eine sec-Pentylgruppe, eine tert-Pentylgruppe, eine Phenylgruppe oder eine Naphthylgruppe, jeweils substituiert mit Deuterium, einer C₁-C₁₀-Alkylgruppe, einer Phenylgruppe oder einer Kombination davon.

5. Organometallverbindung nach einem der Ansprüche 1 bis 4, wobei R₁₀, R₂₀, R₃₀ und R₄₁ bis R₅₀ jeweils unabhängig voneinander sind:
Wasserstoff, Deuterium, -F, -Cl, -Br, -I, -CD₃, -CD₂H, -CDH₂, -CF₃, -CF₂H, -CFH₂, eine C₁-C₆₀-Alkylgruppe, eine C₂-C₆₀-Alkenylgruppe, eine C₂-C₆₀-Alkinylgruppe, eine C₁-C₆₀-Alkoxygruppe, -Si(Q₁)(Q₂)(Q₃) oder -Ge(Q₁)(Q₂)(Q₃), oder
eine Gruppe, dargestellt durch eine von Formeln 9-1 bis 9-61, 9-201 bis 9-237, 10-1 bis 10-129 oder 10-201 bis 10-350: 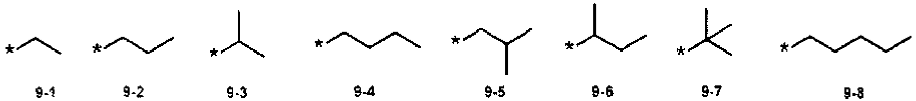 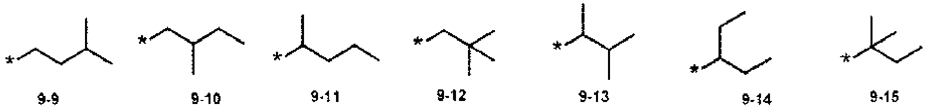 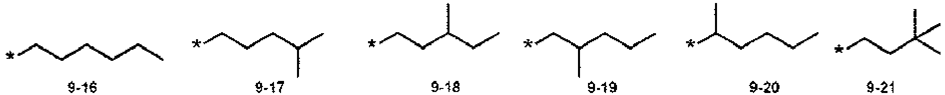 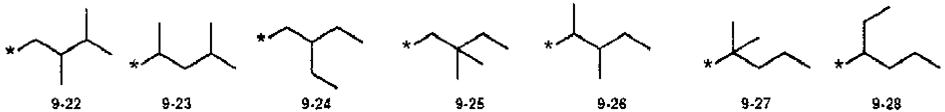 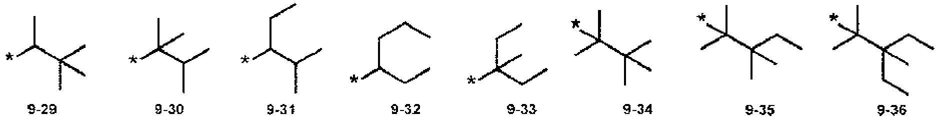 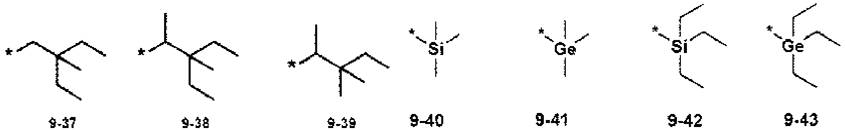 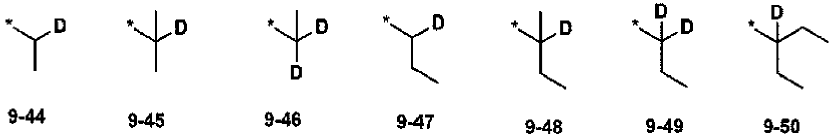 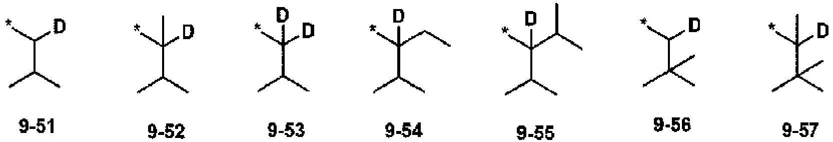 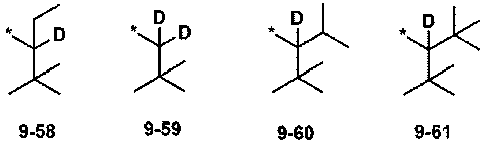 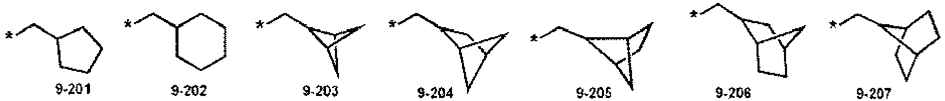 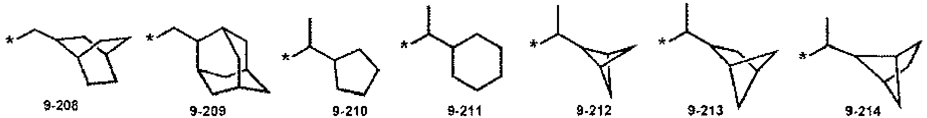 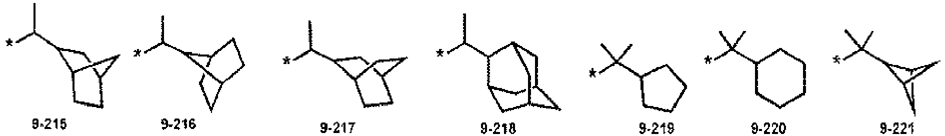 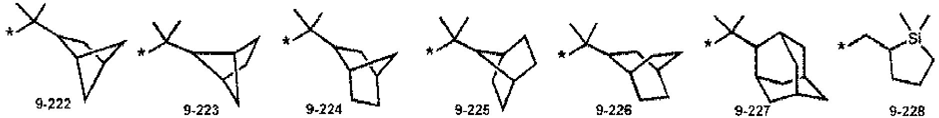 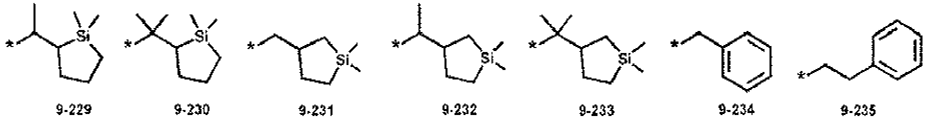 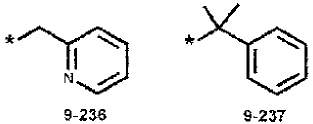 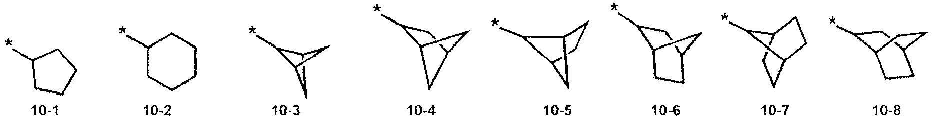 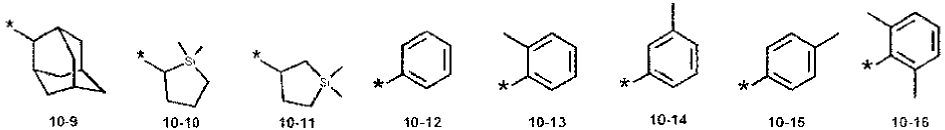 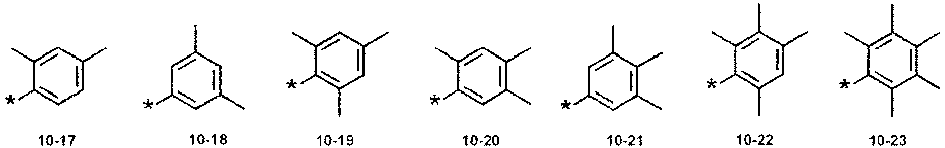 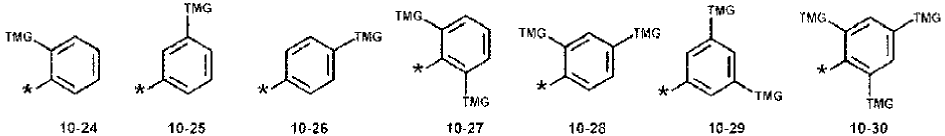 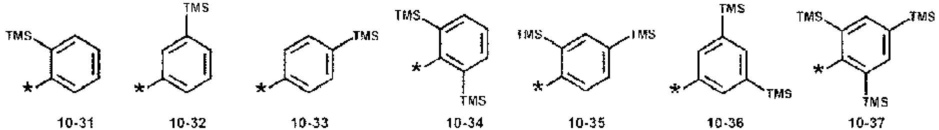 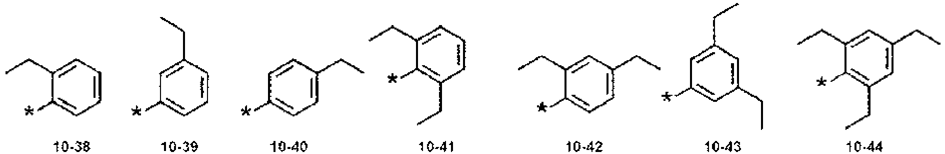 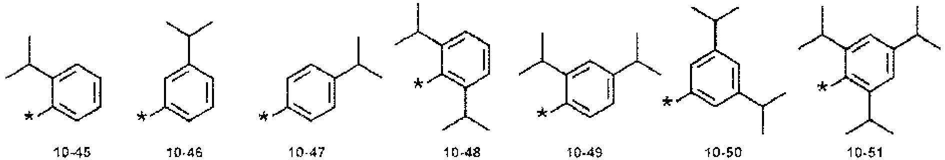 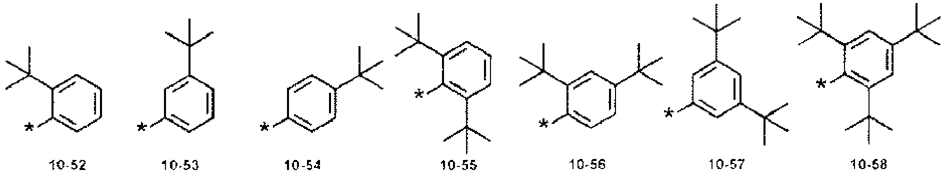 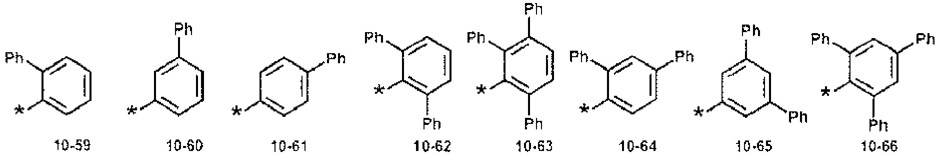 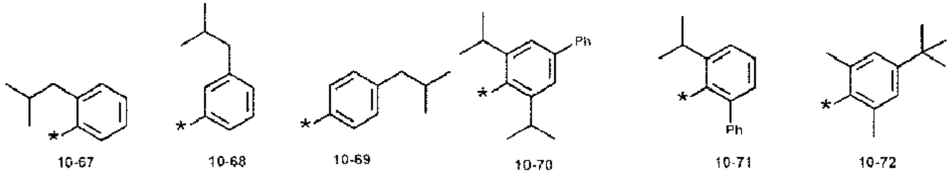 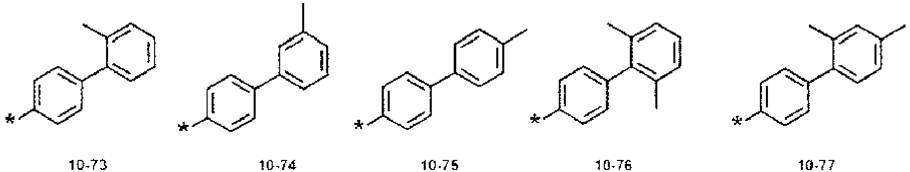 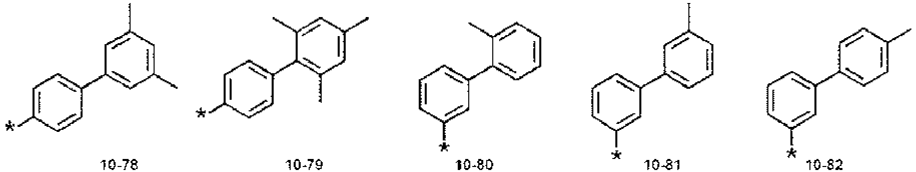 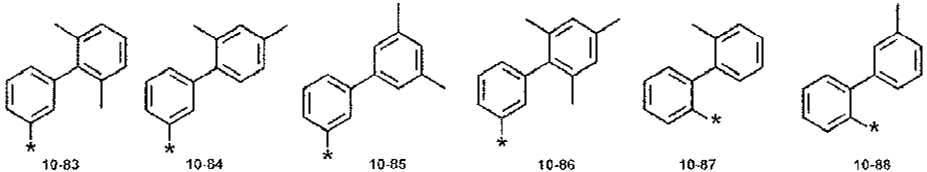 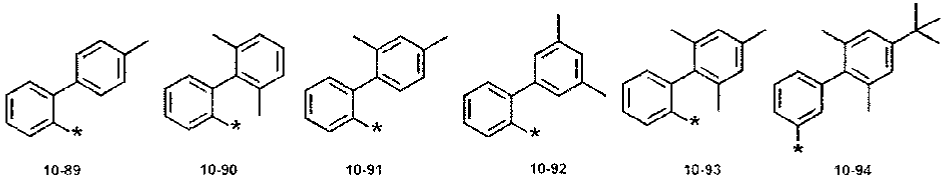 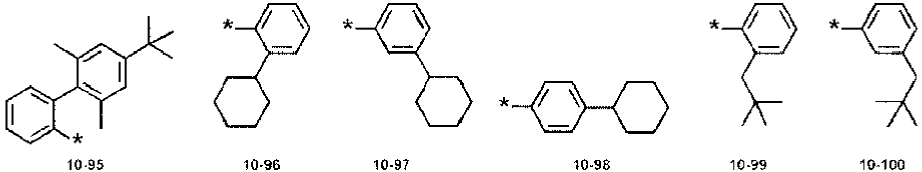 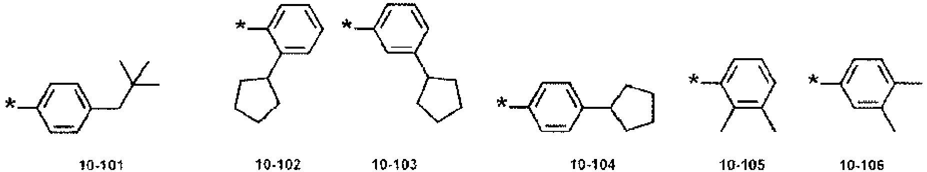 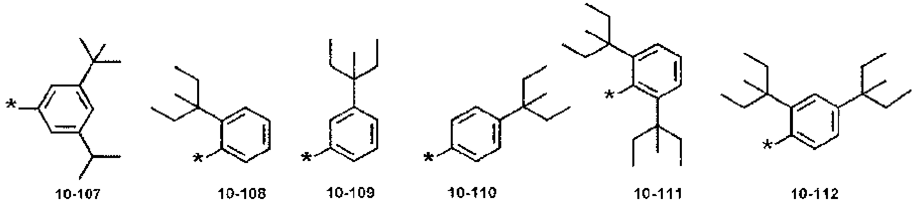 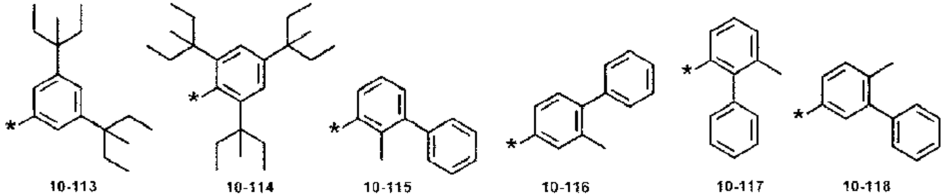 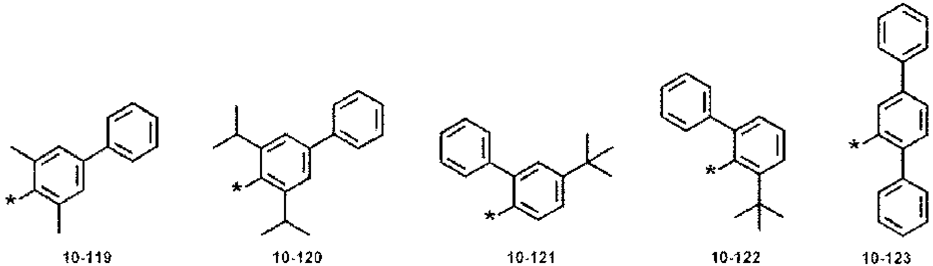 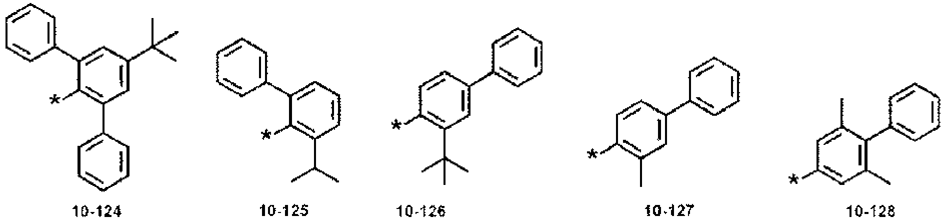 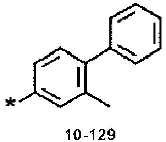 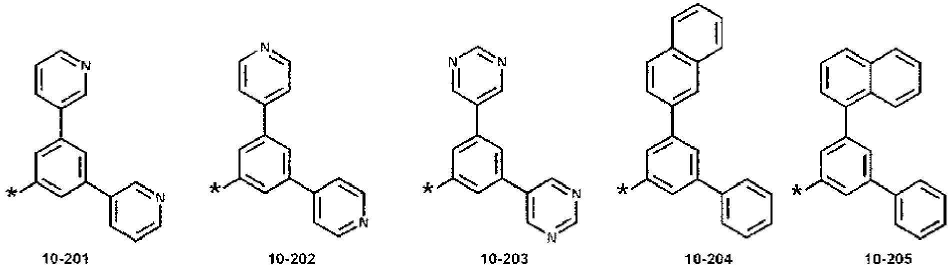 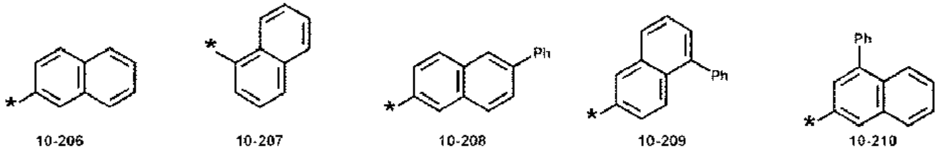 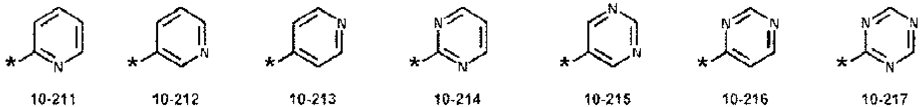 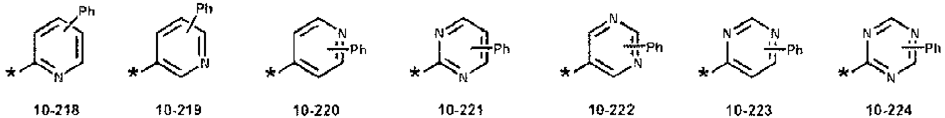 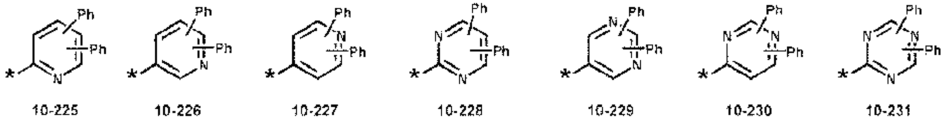 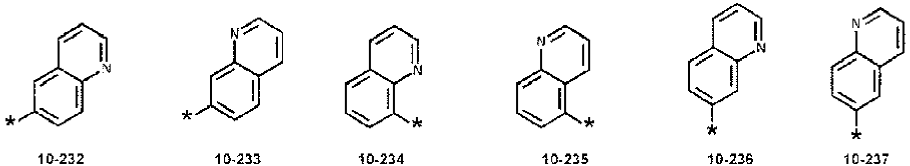 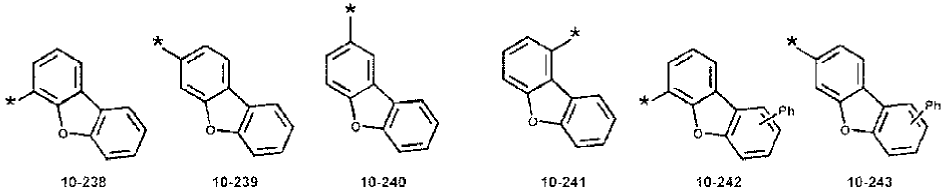 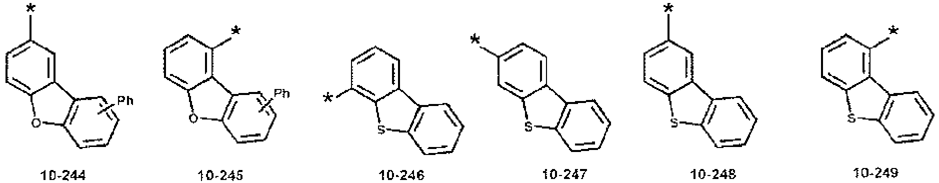 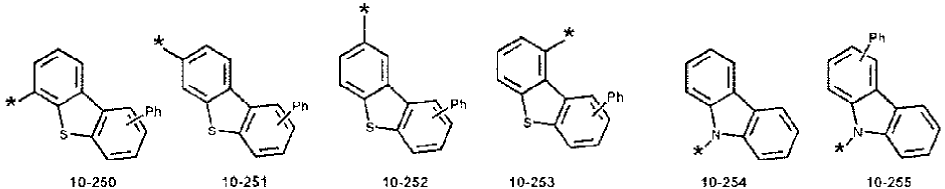 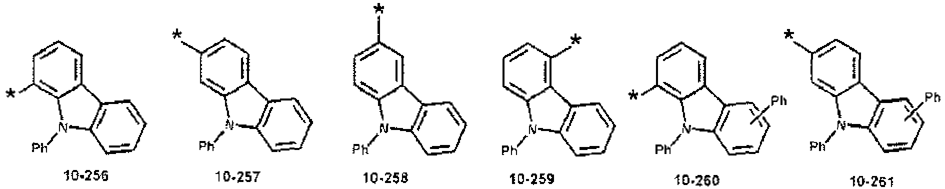 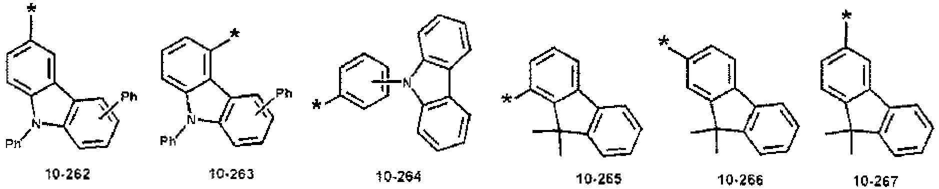 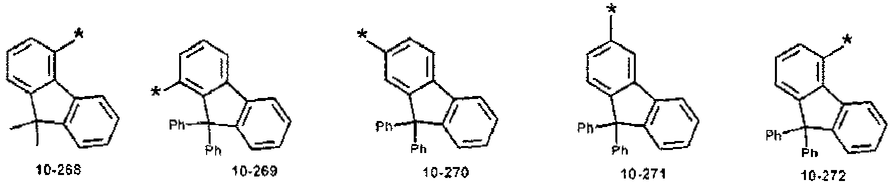 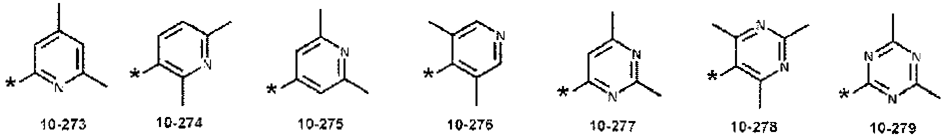 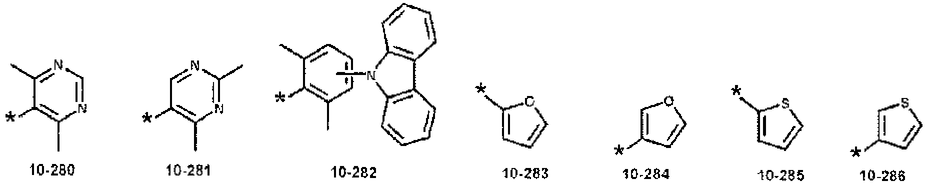 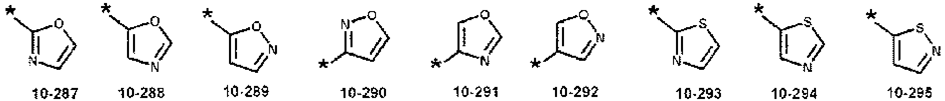 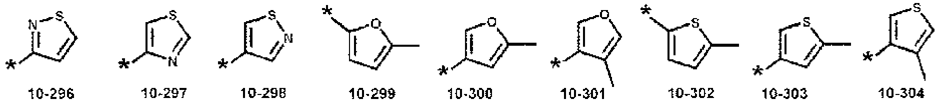 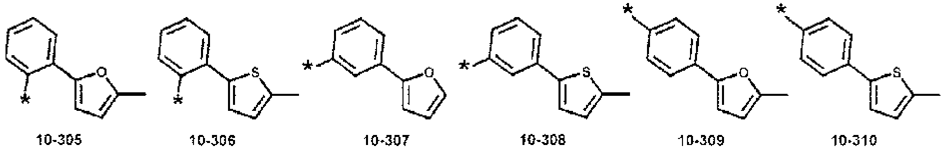 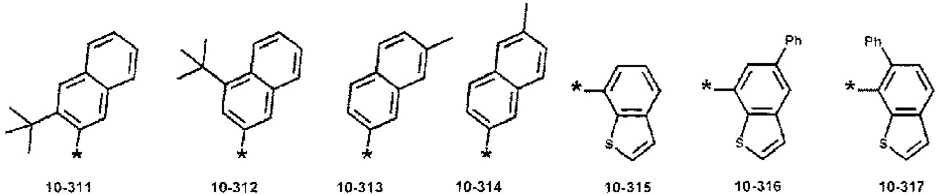 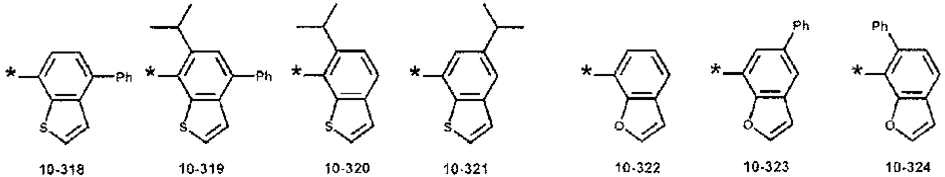 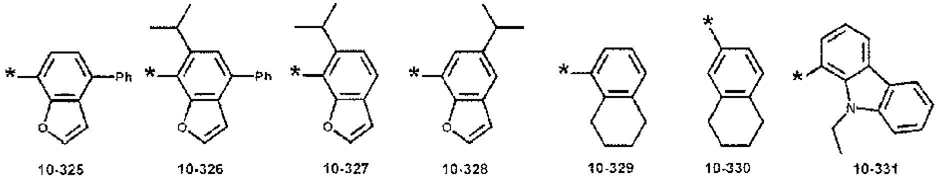 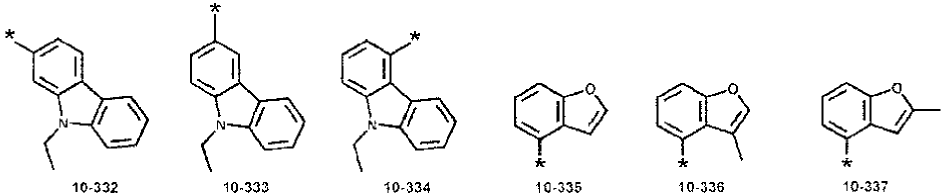 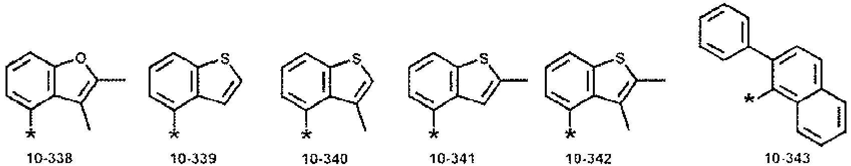 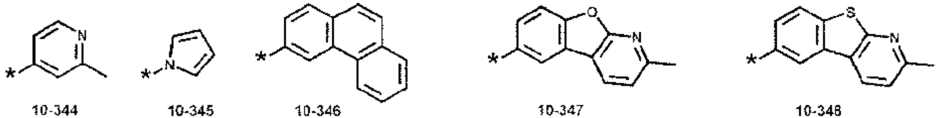 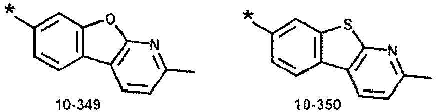
ein * in Formeln 9-1 bis 9-61, 9-201 bis 9-237, 10-1 bis 10-129 und 10-201 bis 10-350 bezeichnet eine Bindungsstelle zu einem benachbarten Atom; Ph bezeichnet eine Phenylgruppe, TMS bezeichnet eine Trimethylsilylgruppe und TMG bezeichnet eine Trimethylgermylgruppe.

6. Organometallverbindung nach einem der Ansprüche 1 bis 5, wobei die Organometallverbindung eine, zwei, drei oder vier Germanylgruppen umfasst, und/oder
wobei mindestens eines von R₃₀(s) in der Anzahl von b30 -Ge(Q₁)(Q₂)(Q₃) ist.

7. Organometallverbindung nach einem der Ansprüche 1-6, wobei CY₁ durch eine von Formeln 1-1 bis 1-16 dargestellt wird: 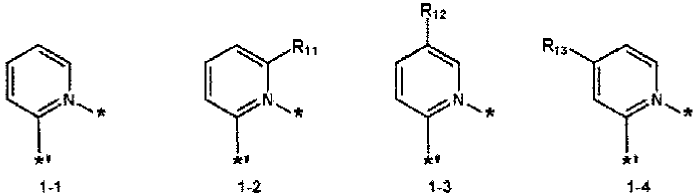 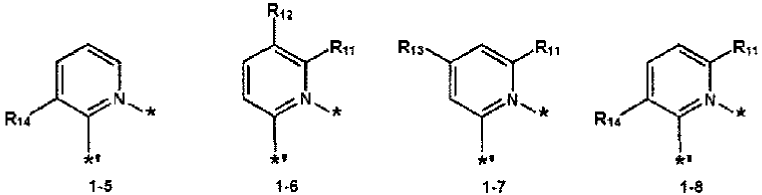 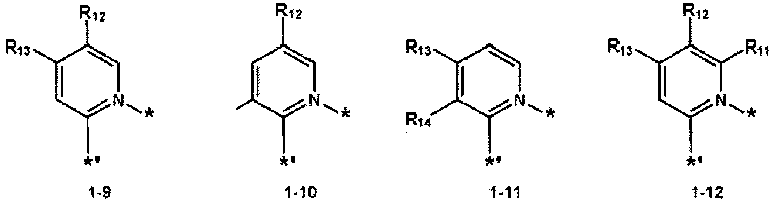 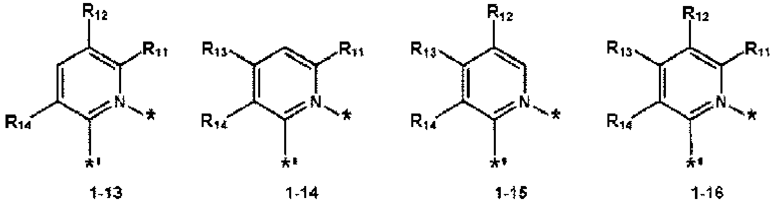 wobei in Formeln 1-1 bis 1-16,
R₁₁ bis R₁₄ jeweils unabhängig voneinander Deuterium, -F, -Cl, -Br, -I, -CD₃, -CD₂H, - CDH₂, -CF₃, -CF₂H, -CFH₂, eine Methylgruppe, eine Ethylgruppe, eine n-Propylgruppe, eine Isopropylgruppe, eine n-Butylgruppe, eine Isobutylgruppe, eine sec-Butylgruppe, eine tert-Butylgruppe, eine n-Pentylgruppe, eine Isopentylgruppe, eine 2-Methylbutylgruppe, eine sec-Pentylgruppe, eine tert-Pentylgruppe, eine Neo-Pentylgruppe, eine 3-Pentylgruppe, eine 3-Methyl-2-butylgruppe, eine Phenylgruppe, eine Biphenylgruppe, eine Naphthylgruppe, - Si(Q₁)(Q₂)(Q₃) oder -Ge(Q₁)(Q₂)(Q₃) sind;
Q₁ bis Q₃ jeweils unabhängig voneinander sind:
-CH₃, -CD₃, -CD₂H, -CDH₂, -CH₂CH₃, -CH₂CD₃, -CH₂CD₂H, -CH₂CDH₂, - CHDCH₃, - CHDCD₂H, -CHDCDH₂, -CHDCD₃, -CD₂CD₃, -CD₂CD₂H oder -CD₂CDH₂,
eine n-Propylgruppe, eine Isopropylgruppe, eine n-Butylgruppe, eine Isobutylgruppe, eine sec-Butylgruppe, eine tert-Butylgruppe, eine n-Pentylgruppe, eine Isopentylgruppe, eine sec-Pentylgruppe, eine tert-Pentylgruppe, eine Phenylgruppe oder eine Naphthylgruppe, oder
eine n-Propylgruppe, eine Isopropylgruppe, eine n-Butylgruppe, eine Isobutylgruppe, eine sec-Butylgruppe, eine tert-Butylgruppe, eine n-Pentylgruppe, eine Isopentylgruppe, eine sec-Pentylgruppe, eine tert-Pentylgruppe, eine Phenylgruppe oder eine Naphthylgruppe, die jeweils mit Deuterium substituiert sind, eine C₁-C₁₀-Alkylgruppe, eine Phenylgruppe oder eine Kombination davon, und
* und *' jeweils eine Bindungsstelle zu einem benachbarten Atom bezeichnen, und/oder
wobei CY₂ durch eine von Formeln 2-1 bis 2-16 dargestellt wird: 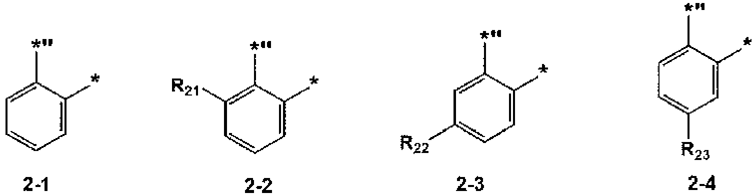 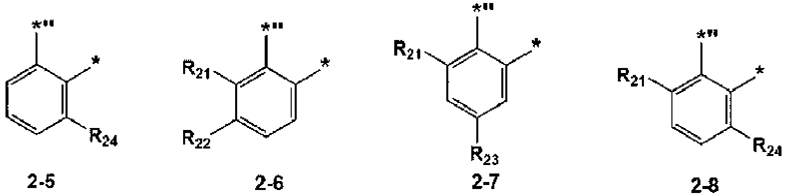 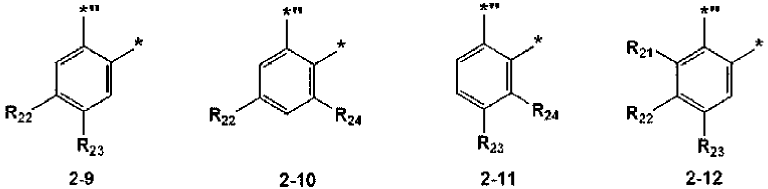 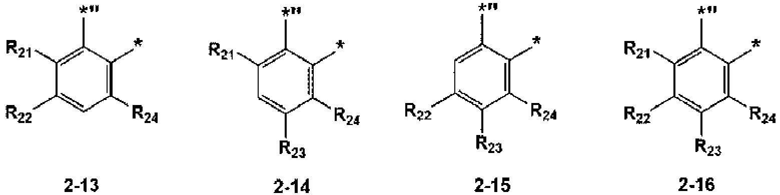
wobei in Formeln 2-1 bis 2-16,
R₂₁ bis R₂₄ jeweils unabhängig voneinander Deuterium, -F, -Cl, -Br, -I, -CD₃, -CD₂H, - CDH₂, -CF₃, -CF₂H, -CFH₂, eine Methylgruppe, eine Ethylgruppe, eine n-Propylgruppe, eine Isopropylgruppe, eine n-Butylgruppe, eine Isobutylgruppe, eine sec-Butylgruppe, eine tert-Butylgruppe, eine n-Pentylgruppe, eine Isopentylgruppe, eine 2-Methylbutylgruppe, eine sec-Pentylgruppe, eine tert-Pentylgruppe, eine Neo-Pentylgruppe, eine 3-Pentylgruppe, eine 3-Methyl-2-butylgruppe, eine Phenylgruppe, eine Biphenylgruppe, eine Naphthylgruppe, - Si(Q₁)(Q₂)(Q₃) oder -Ge(Q₁)(Q₂)(Q₃) sind,
Q₁ bis Q₃ jeweils unabhängig voneinander sind:
-CH₃, -CD₃, -CD₂H, -CDH₂, -CH₂CH₃, -CH₂CD₃, -CH₂CD₂H, -CH₂CDH₂, - CHDCH₃, - CHDCD₂H, -CHDCDH₂, -CHDCD₃, -CD₂CD₃, -CD₂CD₂H oder -CD₂CDH₂,
eine n-Propylgruppe, eine Isopropylgruppe, eine n-Butylgruppe, eine Isobutylgruppe, eine sec-Butylgruppe, eine tert-Butylgruppe, eine n-Pentylgruppe, eine Isopentylgruppe, eine sec-Pentylgruppe, eine tert-Pentylgruppe, eine Phenylgruppe oder eine Naphthylgruppe, oder
eine n-Propylgruppe, eine Isopropylgruppe, eine n-Butylgruppe, eine Isobutylgruppe, eine sec-Butylgruppe, eine tert-Butylgruppe, eine n-Pentylgruppe, eine Isopentylgruppe, eine sec-Pentylgruppe, eine tert-Pentylgruppe, eine Phenylgruppe oder eine Naphthylgruppe, die jeweils mit Deuterium substituiert sind, eine C₁-C₁₀-Alkylgruppe, eine Phenylgruppe oder eine Kombination davon, und
* und *' jeweils eine Bindungsstelle zu einem benachbarten Atom bezeichnen.

8. Organometallverbindung nach einem der Ansprüche 1-7, wobei CY₃ durch eine von Formeln 3-1 bis 3-40 dargestellt wird: 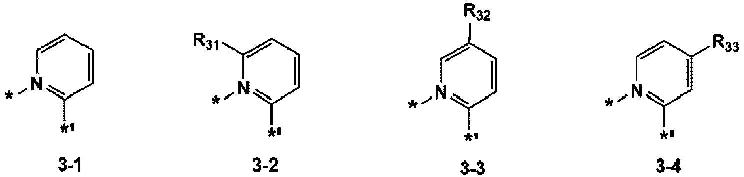 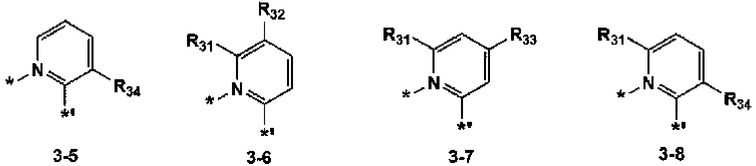 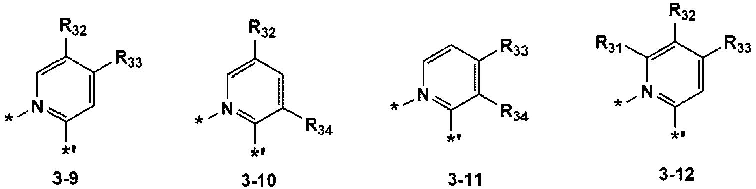 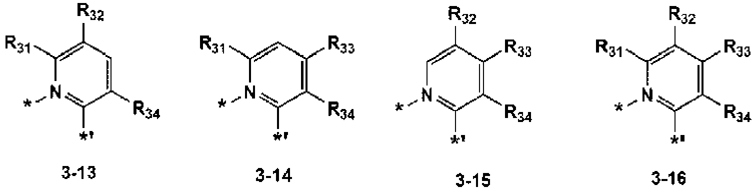 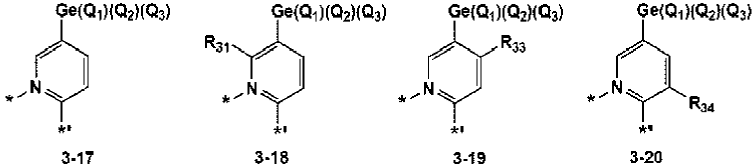 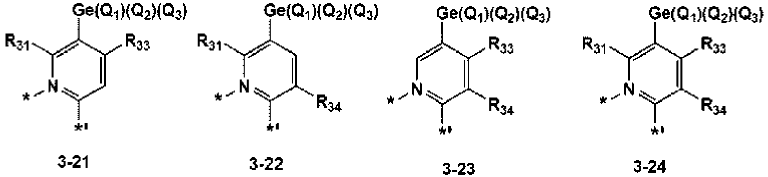 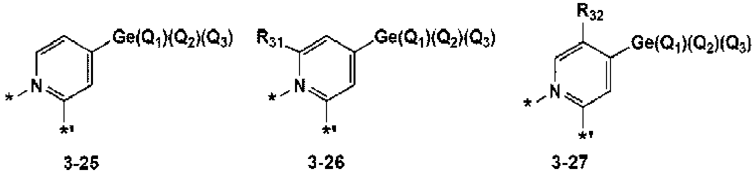 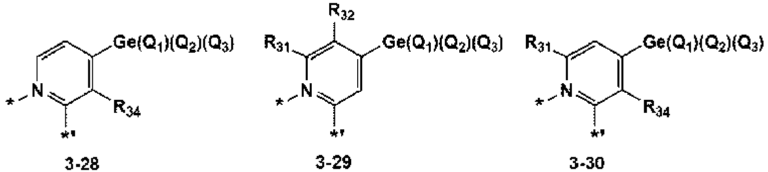 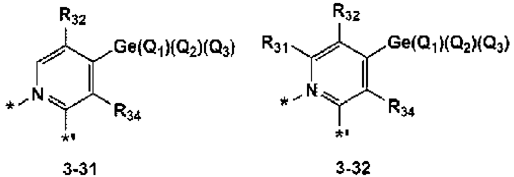 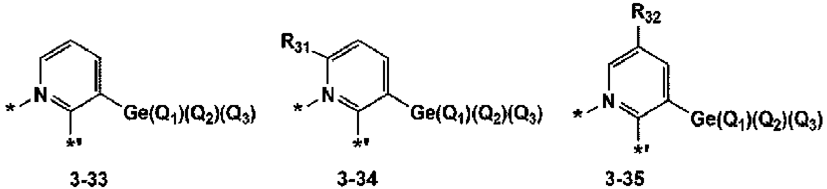 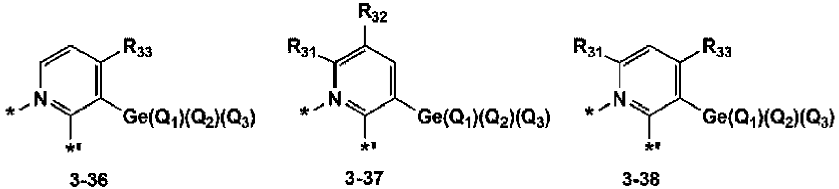 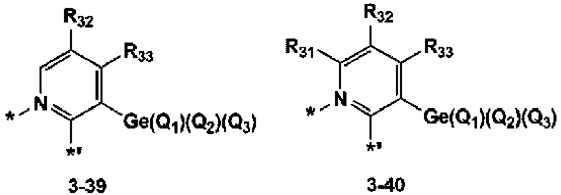 wobei in Formeln 3-1 bis 3-40,
R₃₁ bis R₃₄ jeweils unabhängig voneinander Deuterium, -F, -Cl, -Br, -I, -CD₃, -CD₂H, - CDH₂, -CF₃, -CF₂H, -CFH₂, eine Methylgruppe, eine Ethylgruppe, eine n-Propylgruppe, eine Isopropylgruppe, eine n-Butylgruppe, eine Isobutylgruppe, eine sec-Butylgruppe, eine tert-Butylgruppe, eine n-Pentylgruppe, eine Isopentylgruppe, eine 2-Methylbutylgruppe, eine sec-Pentylgruppe, eine tert-Pentylgruppe, eine Neo-Pentylgruppe, eine 3-Pentylgruppe, eine 3-Methyl-2-butylgruppe, eine Phenylgruppe, eine Biphenylgruppe, eine Naphthylgruppe, - Si(Q₁)(Q₂)(Q₃) oder -Ge(Q₁)(Q₂)(Q₃) sind,
Q₁ bis Q₃ jeweils unabhängig voneinander sind:
-CH₃, -CD₃, -CD₂H, -CDH₂, -CH₂CH₃, -CH₂CD₃, -CH₂CD₂H, -CH₂CDH₂, - CHDCH₃, - CHDCD₂H, -CHDCDH₂, -CHDCD₃, -CD₂CD₃, -CD₂CD₂H, oder CD₂CDH₂ ist,
eine n-Propylgruppe, eine Isopropylgruppe, eine n-Butylgruppe, eine Isobutylgruppe, eine sec-Butylgruppe, eine tert-Butylgruppe, eine n-Pentylgruppe, eine Isopentylgruppe, eine sec-Pentylgruppe, eine tert-Pentylgruppe, eine Phenylgruppe oder eine Naphthylgruppe, oder
eine n-Propylgruppe, eine Isopropylgruppe, eine n-Butylgruppe, eine Isobutylgruppe, eine sec-Butylgruppe, eine tert-Butylgruppe, eine n-Pentylgruppe, eine Isopentylgruppe, eine sec-Pentylgruppe, eine tert-Pentylgruppe, eine Phenylgruppe oder eine Naphthylgruppe, die jeweils mit Deuterium substituiert sind, eine C₁-C₁₀-Alkylgruppe, eine Phenylgruppe oder eine Kombination davon, und
* und *' jeweils eine Bindungsstelle zu einem benachbarten Atom bezeichnen.

9. Organometallverbindung nach einem der Ansprüche 1-8, wobei die Organometallverbindung durch Formel 11-1 dargestellt wird: 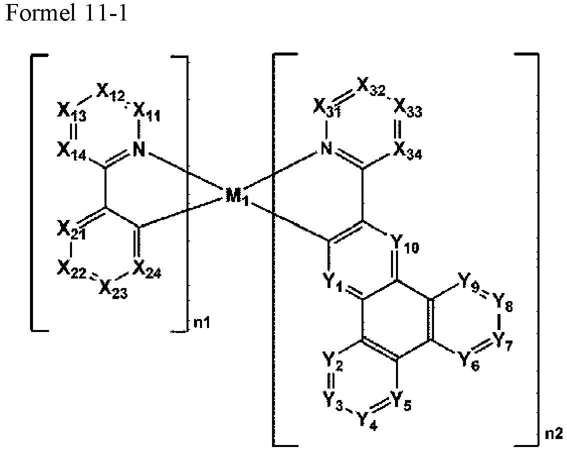 wobei in Formel 11-1,
M₁, n1, n2 und Y₁ bis Y₁₀ jeweils dieselben wie die in Anspruch 1 beschriebenen sind,
X₁₁ C(R₁₁) oder N ist, X₁₂ C(R₁₂) oder N ist, X₁₃ C(R₁₃) oder N ist, und X₁₄ C(R₁₄) oder N ist,
X₂₁ ist C(R₂₁) oder N ist, X₂₂ C(R₂₂) oder N ist, X₂₃ C(R₂₃) oder N ist, und X₂₄ C(R₂₄) oder N ist,
X₃₁ C(R₃₁) oder N ist, X₃₂ C(R₃₂) oder N ist, X₃₃ C(R₃₃) oder N ist, und X₃₄ C(R₃₄) oder N ist,
R₁₁ bis R₁₄ jeweils unabhängig voneinander dieselben wie die in Zusammenhang mit R₁₀ in Anspruch 1 beschriebenen sind,
R₂₁ bis R₂₄ jeweils unabhängig voneinander dieselben wie die in Zusammenhang mit R₂₀ in Anspruch 1 beschriebenen sind,
R₃₁ bis R₃₄ jeweils unabhängig voneinander dieselben wie die in Zusammenhang mit R₃₀ in Anspruch 1 beschriebenen sind,
zwei oder mehr von R₁₁ bis R₁₄ gegebenenfalls miteinander verbunden sind, um eine carbocyclische C₅-C₃₀-Gruppe zu bilden, die unsubstituiert oder mit mindestens einem R₁₀ₐ substituiert ist, oder eine heterocyclische C₁-C₃₀-Gruppe, die unsubstituiert oder mit mindestens einem R₁₀ₐ substituiert ist,
zwei oder mehr von R₂₁ bis R₂₄ gegebenenfalls miteinander verbunden sind, um eine carbocyclische C₅-C₃₀-Gruppe zu bilden, die unsubstituiert oder mit mindestens einem R₁₀ₐ substituiert ist, oder eine heterocyclische C₁-C₃₀-Gruppe, die unsubstituiert oder mit mindestens einem R₁₀ₐ substituiert ist,
zwei oder mehr von R₃₁ bis R₃₄ gegebenenfalls miteinander verbunden sind, um eine carbocyclische C₅-C₃₀-Gruppe zu bilden, die unsubstituiert oder mit mindestens einem R₁₀ₐ substituiert ist, oder eine heterocyclische C₁-C₃₀-Gruppe, die unsubstituiert oder mit mindestens einem R₁₀ₐ substituiert ist, und
R₁₀ₐ dasselbe wie das in Zusammenhang mit R₁₀ beschriebene ist.

10. Organometallverbindung nach Anspruch 1, wobei die Organometallverbindung eine von Verbindungen 1 bis 36 ist:

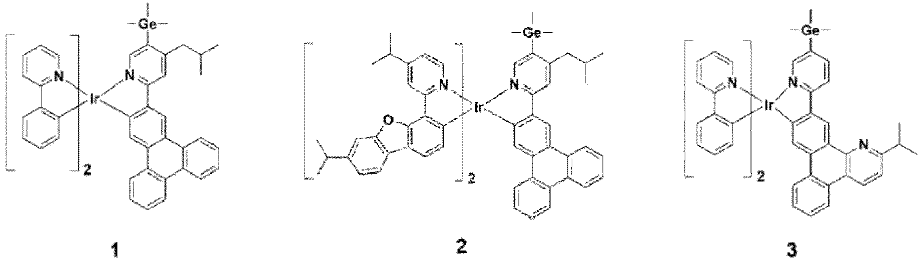

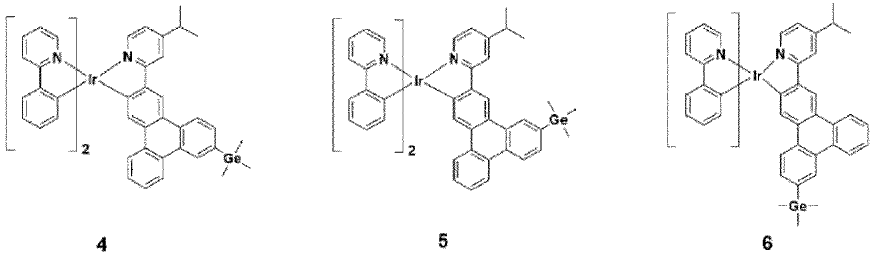

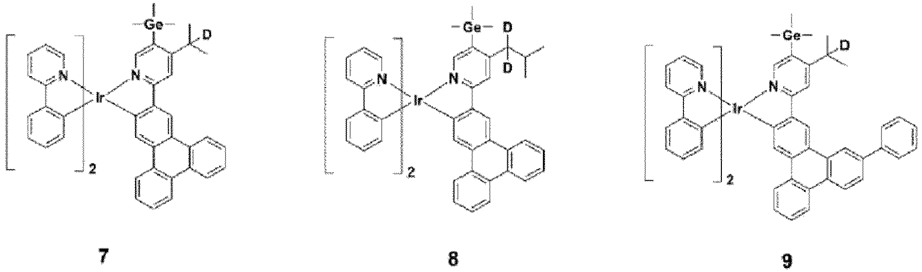

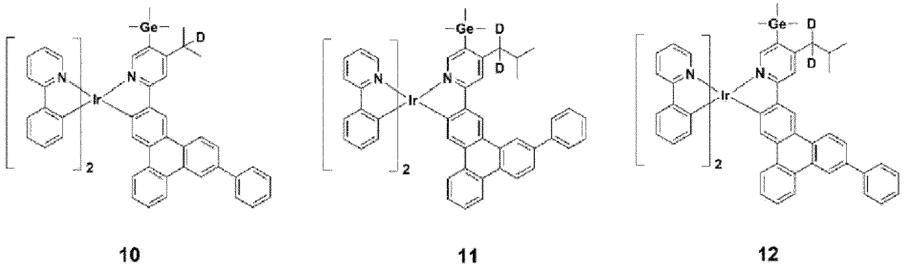

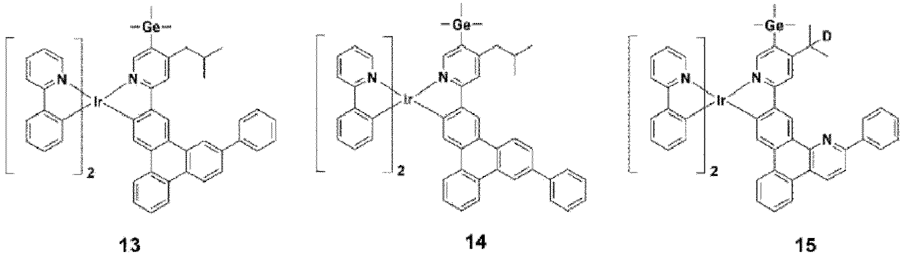

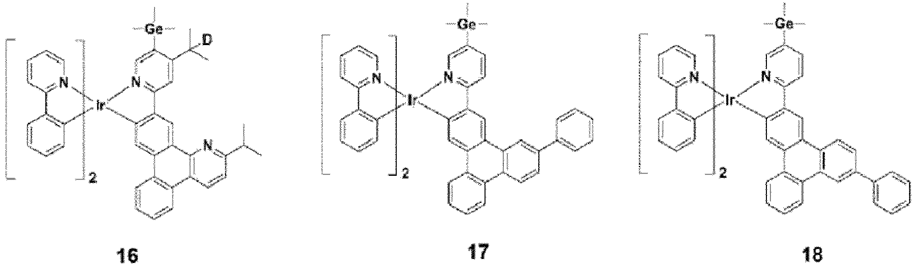

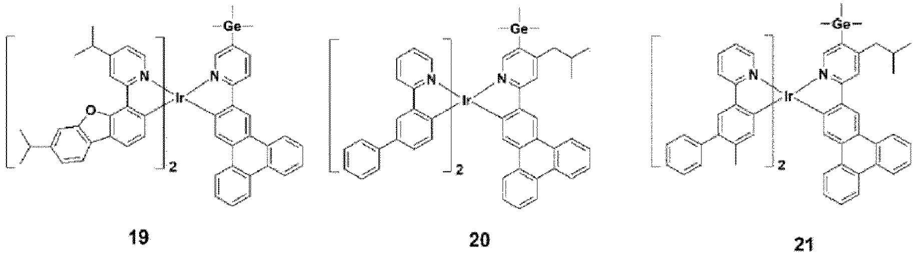

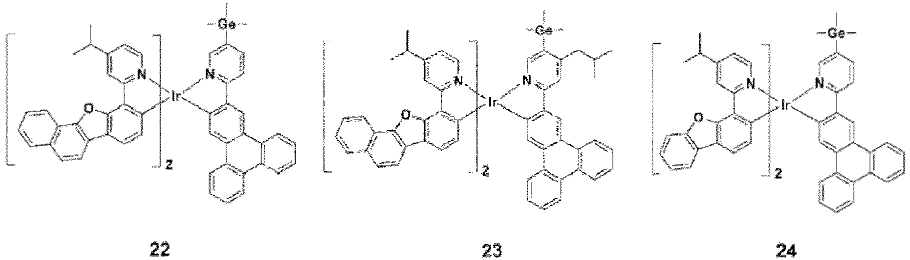

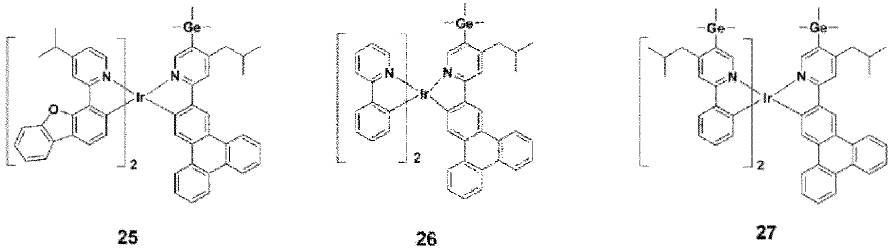

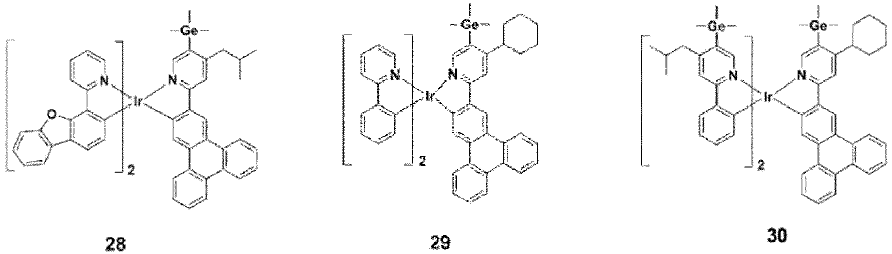

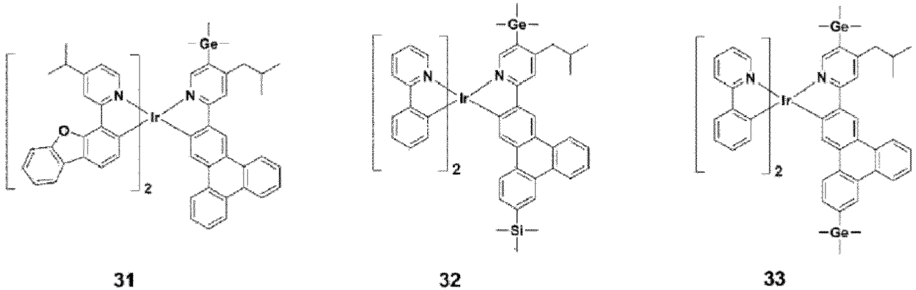

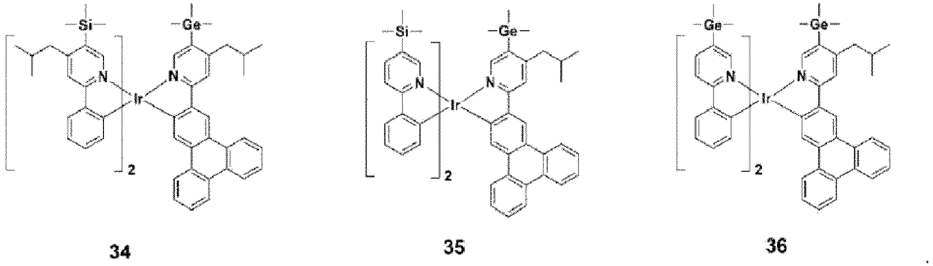

11. Organische lichtemittierende Vorrichtung, umfassend:
eine erste Elektrode,
eine zweite Elektrode, und
eine organische Schicht, die sich zwischen der ersten Elektrode und der zweiten Elektrode befindet und eine Emissionsschicht umfasst,
wobei die organische Schicht die Organometallverbindung nach einem der Ansprüche 1-10 umfasst.

12. Organische lichtemittierende Vorrichtung nach Anspruch 11, wobei die Organometallverbindung in der Emissionsschicht enthalten ist.

13. Organische lichtemittierende Vorrichtung nach Anspruch 12, wobei die Emissionsschicht ferner einen Wirt umfasst und die Menge des Wirts größer ist als die Menge der Organometallverbindung, und/oder
wobei die Emissionsschicht grünes Licht mit einer maximalen Emissionswellenlänge von 500 nm bis 600 nm emittiert.

14. Organische lichtemittierende Vorrichtung nach Anspruch 12 oder 13, wobei die erste Elektrode eine Anode ist,
die zweite Elektrode eine Kathode ist,
die organische Schicht ferner einen Lochtransportbereich, der sich zwischen der ersten Elektrode und der Emissionsschicht befindet, und einen Elektronentransportbereich, der sich zwischen der Emissionsschicht und der zweiten Elektrode befindet, umfasst, wobei
der Lochtransportbereich eine Lochinjektionsschicht, eine Lochtransportschicht, eine Elektronensperrschicht, eine Pufferschicht oder eine Kombination davon umfasst, und
der Elektronentransportbereich eine Lochsperrschicht, eine Elektronentransportschicht, eine Elektroneninjektionsschicht oder eine Kombination davon umfasst.

15. Diagnostische Zusammensetzung, die die Organometallverbindung nach einem der Ansprüche 1-10 umfasst.

## Revendications

1. Composé organométallique représenté par la Formule 1 :
Formule 1 M₁(Ln₁)ₙ₁(Ln₂)ₙ₂
dans la Formule 1,
M₁ est un métal de transition,
Ln₁ est un ligand représenté par la Formule 1-1,
Ln₂ est un ligand représenté par la Formule 1-2,
n1 vaut 0, 1 ou 2,
n2 vaut 1, 2 ou 3, 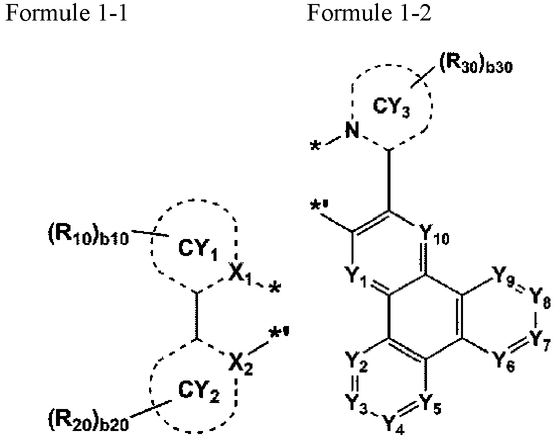
où, dans les formules 1-1 et 1-2,
X₁ est C ou N, et X₂ est C ou N,
Y₁ est C(R₄₁) ou N, Y₂ est C(R₄₂) ou N, Y₃ est C(R₄₃) ou N, Y₄ est C(R₄₄) ou N, Y₅ est C(R₄₅) ou N, Y₆ est C(R₄₆) ou N, Y₇ est C(R₄₇) ou N, Y₈ est C(R₄₈) ou N, Y₉ est C(R₄₉) ou N, et Y₁₀ est C(R₅₀) ou N,
CY₁ et CY₂ sont chacun indépendamment un groupe carbocyclique en C₅ à C₃₀ ou un groupe hétérocyclique en C₁ à C₃₀,
CY₃ est un groupe hétérocyclique en C₁ à C₃₀ contenant N,
R₁₀, R₂₀, R₃₀, et R₄₁ à R₅₀ sont chacun indépendamment hydrogène, deutérium, -F, -Cl, - Br, -I, -SF₅, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀ substitué ou non substitué, un groupe alcényle en C₂ à C₆₀ substitué ou non substitué, un groupe alcynyle en C₂ à C₆₀ substitué ou non substitué, un groupe alcoxy en C₁ à C₆₀ substitué ou non substitué, un groupe cycloalkyle en C₃ à C₁₀ substitué ou non substitué, un groupe hétérocycloalkyle en C₁ à C₁₀ substitué ou non substitué, un groupe cycloalcényle en C₃ à C₁₀ substitué ou non substitué, un groupe hétérocycloalcényle en C₂ à C₁₀ substitué ou non substitué, un groupe aryle en C₆ à C₆₀ substitué ou non substitué, un groupe aryloxy en C₆ à C₆₀ substitué ou non substitué, un groupe arylthio en C₆ à C₆₀ substitué ou non substitué, un groupe hétéroaryle en C₁ à C₆₀ substitué ou non substitué, un groupe hétéroaryloxy en C₁ à C₆₀ substitué ou non substitué, un groupe hétéroarylthio en C₁ à C₆₀ substitué ou non substitué, un groupe polycyclique condensé non aromatique monovalent substitué ou non substitué, un groupe hétéropolycyclique condensé non aromatique monovalent substitué ou non substitué, -Si(Q₁)(Q₂)(Q₃), -Ge(Q₁)(Q₂)(Q₃), -N(Q₄)(Q₅), - B(Q₆)(Q₇), ou -P(=O)(Q₈)(Q₉),
au moins un des ligands représentés par les formules 1-1 et 1-2 comprend au moins un - Ge(Q₁)(Q₂)(Q₃),
deux ou plus d'une pluralité de R₁₀ sont éventuellement liés ensemble pour former un groupe carbocyclique en C₅ à C₃₀ substitué ou non substitué ou un groupe hétérocyclique en C₁ à C₃₀ substitué ou non substitué,
deux ou plus d'une pluralité de R₂₀ sont éventuellement liés ensemble pour former un groupe carbocyclique en C₅ à C₃₀ substitué ou non substitué ou un groupe hétérocyclique en C₁ à C₃₀ substitué ou non substitué,
deux ou plus d'une pluralité de R₃₀ sont éventuellement liés ensemble pour former un groupe carbocyclique en C₅ à C₃₀ substitué ou non substitué ou un groupe hétérocyclique en C₁ à C₃₀ substitué ou non substitué,
deux ou plus groupes de R₁₀, R₂₀, R₃₀, et R₄₁ à R₅₀ adjacents sont éventuellement liés ensemble pour former un groupe carbocyclique en C₅ à C₃₀ substitué ou non substitué ou un groupe hétérocyclique en C₁ à C₃₀ substitué ou non substitué,
b10, b20 et b30 sont chacun indépendamment 1, 2, 3, 4, 5, 6, 7, 8, 9 ou 10,
au moins un substituent du groupe carbocyclique en C₅ à C₃₀ substitué, du groupe hétérocyclique en C₁ à C₃₀ substitué, du groupe alkyle en C₁ à C₆₀ substitué, du groupe alcényle en C₂ à C₆₀ substitué, du groupe alcynyle en C₂ à C₆₀ substitué, du groupe alcoxy en C₁ à C₆₀ substitué, du groupe cycloalkyle en C₃ à C₁₀ substitué, du groupe hétérocycloalkyle en C₁ à C₁₀ substitué, du groupe cycloalcényle en C₃ à C₁₀ substitué, du groupe hétérocycloalcényle en C₂ à C₁₀ substitué, du groupe aryle en C₆ à C₆₀ substitué, du groupe aryloxy en C₆ à C₆₀ substitué, du groupe arylthio en C₆ à C₆₀ substitué, du groupe hétéroaryle en C₁ à C₆₀ substitué, du groupe hétéroaryloxy en C₁ à C₆₀ substitué, du groupe hététoarylthio en C₁ à C₆₀ substitué, du groupe polycyclique condensé non aromatique monovalent substitué et du groupe hétéropolycyclique condensé non aromatique monovalent substitué est :
deutérium, -F, -Cl, -Br, -I, -CD₃, -CD₂H, -CDH₂, -CF₃, -CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀, un groupe alcényle en C₂ à C₆₀, un groupe alcynyle en C₂ à C₆₀ ou un groupe alcoxy en C₁ à C₆₀ ;
un groupe alkyle en C₁ à C₆₀, un groupe alcényle en C₂ à C₆₀, un groupe alcynyle en C₂ à C₆₀ ou un groupe alcoxy en C₁ à C₆₀, chacun substitué par le deutérium, -F, -Cl, -Br, -I, -CD₃, - CD₂H, -CDH₂, -CF₃, -CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe cycloalkyle en C₃ à C₁₀, un groupe hétérocycloalkyle en C₁ à C₁₀, un groupe cycloalcényle en C₃ à C₁₀, un groupe hétérocycloalcényle en C₂ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe hétéroaryloxy en C₁ à C₆₀, un groupe hétéroarylthio en C₁ à C₆₀, un groupe polycyclique condensé non aromatique monovalent, un groupe hétéropolycyclique condensé non aromatique monovalent, -Si(Q₁₁)(Q₁₂)(Q₁₃), - Ge(Q₁₁)(Q₁₂)(Q₁₃), -N(Q₁₄)(Q₁₅), -B(Q₁₆)(Q₁₇), -P(=O)(Q₁₈)(Q₁₉), ou une combinaison de ceux-ci ;
un groupe cycloalkyle en C₃ à C₁₀, un groupe hétérocycloalkyle en C₁ à C₁₀, un groupe cycloalcényle en C₃ à C₁₀, un groupe hétérocycloalcényle en C₂ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe hétéroaryloxy en C₁ à C₆₀, un groupe hétéroarylthio en C₁ à C₆₀, un groupe polycyclique condensé non aromatique monovalent ou un groupe hétéropolycyclique condensé non aromatique monovalent ;
un groupe cycloalkyle en C₃ à C₁₀, un groupe hétérocycloalkyle en C₁ à C₁₀, un groupe cycloalcényle en C₃ à C₁₀, un groupe hétérocycloalcényle en C₂ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique condensé non aromatique monovalent et un groupe hétéropolycyclique condensé non aromatique monovalent, chacun substitué par le deutérium, -F, -Cl, -Br, -I, -CD₃, - CD₂H, -CDH₂, -CF₃, -CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀, un groupe alcényle en C₂ à C₆₀, un groupe alcynyle en C₂ à C₆₀, un groupe alcoxy en C₁ à C₆₀, un groupe cycloalkyle en C₃ à C₁₀, un groupe hétérocycloalkyle en C₁ à C₁₀, un groupe cycloalcényle en C₃ à C₁₀, un groupe hétérocycloalcényle en C₂ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe hétéroaryloxy en C₁ à C₆₀, un groupe hétéroarylthio en C₁ à C₆₀, un groupe polycyclique condensé non aromatique monovalent, un groupe hétéropolycyclique condensé non aromatique monovalent, - Si(Q₂₁)(Q₂₂)(Q₂₃), -Ge(Q₂₁)(Q₂₂)(Q₂₃), -N(Q₂₄)(Q₂₅), -B(Q₂₆)(Q₂₇), -P(=O)(Q₂₈)(Q₂₉), ou une combinaison de ceux-ci ; ou
-Si(Q₃₁)(Q₃₂)(Q₃₃), -Ge(Q₃₁)(Q₃₂)(Q₃₃), -N(Q₃₄)(Q₃₅), -B(Q₃₆)(Q₃₇), ou -P(=O)(Q₃₈)(Q₃₉),
où Q₁ à Q₉, Q₁₁ à Q₁₉, Q₂₁ à Q₂₉, et Q₃₁ à Q₃₉ sont chacun indépendamment l'hydrogène, le deutérium, -F, -Cl, -Br, -I, -un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀ substitué ou non substitué, un groupe alcényle en C₂ à C₆₀ substitué ou non substitué, un groupe alcynyle en C₂ à C₆₀ substitué ou non substitué, un groupe alcoxy en C₁ à C₆₀ substitué ou non substitué, un groupe cycloalkyle en C₃ à C₁₀ substitué ou non substitué, un groupe hétérocycloalkyle en C₁ à C₁₀ substitué ou non substitué, un groupe cycloalcényle en C₃ à C₁₀ substitué ou non substitué, un groupe hétérocycloalcényle en C₂ à C₁₀ substitué ou non substitué, un groupe aryle en C₆ à C₆₀ substitué ou non substitué, un groupe aryloxy en C₆ à C₆₀ substitué ou non substitué, un groupe arylthio en C₆ à C₆₀ substitué ou non substitué, un groupe hétéroaryle en C₁ à C₆₀ substitué ou non substitué, un groupe hétéroaryloxy en C₁ à C₆₀, un groupe hétéroarylthio en C₁ à C₆₀, un groupe polycyclique condensé non aromatique monovalent substitué ou non substitué ou un groupe hétéropolycyclique condensé non aromatique monovalent substitué ou non substitué.

2. Composé organométallique selon la revendication 1, dans lequel M₁ est l'iridium (Ir), le platine (Pt), l'osmium (Os), le titane (Ti), le zirconium (Zr), l'hafnium (Hf), l'europium (Eu), le terbium (Tb), le thulium (Tm) ou le rhodium (Rh) ;
de préférence dans lequel M₁ est Ir, et la somme de n1 et n2 est 3.

3. Composé organométallique selon les revendications 1 ou 2, dans lequel CY₁ et CY₂ sont chacun indépendamment un groupe benzène, un groupe naphtalène, un groupe 1,2,3,4-tétrahydronaphtalène, un groupe phénanthrène, un groupe pyridine, un groupe pyrimidine, un groupe pyrazine, un groupe pyridazine, un groupe triazine, un groupe quinoléine, un groupe isoquinoléine, un groupe quinoxaline, un groupe quinazoline, un groupe phénanthroline, un groupe benzofurane, un groupe benzothiophène, un groupe fluorène, un groupe carbazole, un groupe dibenzofurane, un groupe dibenzothiophène, un groupe dibenzosilole, un groupe azafluorène, un groupe azacarbazole, un groupe azadibenzofurane, un groupe azadibenzothiophène ou un groupe azadibenzosilole ; et/ou
dans lequel CY₃ est un groupe pyridine, un groupe pyrimidine, un groupe pyridazine, un groupe pyrazine, un groupe triazine, un groupe quinoléine, un groupe isoquinoléine, un groupe quinoxaline ou un groupe quinazoline.

4. Composé organométallique selon l'une quelconque des revendications 1 à 3, dans lequel R₁₀, R₂₀, R₃₀, et R₄₁ à R₅₀ sont chacun indépendamment :
hydrogène, deutérium, -F, -Cl, -Br, -I, -CD₃, -CD₂H, -CDH₂, -CF₃, -CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, -SF₅, un groupe alkyle en C₁ à C₂₀, ou un groupe alcoxy en C₁ à C₂₀ ;
un groupe alkyle en C₁ à C₂₀ ou un groupe alcoxy en C₁ à C₂₀, chacun substitué par le deutérium, -F, -Cl, -Br, -I, -CD₃, -CD₂H, -CDH₂, -CF₃, -CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₁₀, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe pyridinyle, un groupe pyrimidinyle, ou une combinaison de ceux-ci ;
un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle , ou un groupe imidazopyrimidinyle ;
un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle ou un groupe imidazopyrimidinyle, chacun substitué par le deutérium, -F, -Cl, -Br, -I, -CD₃, -CD₂H, -CDH₂, - CF₃, -CF₂H, -CFH₂, un groupe hydroxyle, un groupe cyano, un groupe nitro, un groupe amino, un groupe amidino, un groupe hydrazine, un groupe hydrazone, un groupe carboxylique ou un sel de celui-ci, un groupe acide sulfonique ou un sel de celui-ci, un groupe acide phosphorique ou un sel de celui-ci, un groupe alkyle en C₁ à C₂₀, un groupe alcoxy en C₁ à C₂₀, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle, un groupe imidazopyrimidinyle, ou une combinaison de ceux-ci ; ou
-Si(Q₁)(Q₂)(Q₃), -Ge(Q₁)(Q₂)(Q₃), -N(Q₄)(Q₅), -B(Q₆)(Q₇), ou -P(=O)(Q₈)(Q₉),
où Q₁ à Q₉ sont chacun indépendamment :
-CH₃, -CD₃, -CD₂H, -CDH₂, -CH₂CH₃, -CH₂CD₃, -CH₂CD₂H, -CH₂CDH₂, - CHDCH₃,-CHDCD₂H, -CHDCDH₂, -CHDCD₃, -CD₂CD₃, -CD₂CD₂H, ou -CD₂CDH₂ ;
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle ou un groupe naphtyle ; ou
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle et un groupe naphtyle, chacun substitué par le deutérium, un groupe alkyle en C₁ à C₁₀, un groupe phényle, ou une combinaison de ceux-ci.

5. Composé organométallique selon l'une quelconque des revendications 1 à 4 dans lequel R₁₀, R₂₀, R₃₀, et R₄₁ à R₅₀ sont chacun indépendamment :
hydrogène, deutérium, -F, -Cl, -Br, -I, -CD₃, -CD₂H, -CDH₂, -CF₃, -CF₂H, -CFH₂, un groupe alkyle en C₁ à C₆₀, un groupe alcényle en C₂ à C₆₀, un groupe alcynyle en C₂ à C₆₀, un groupe alcoxy en C₁ à C₆₀, -Si(Q₁)(Q₂)(Q₃), ou -Ge(Q₁)(Q₂)(Q₃) ; ou
un groupe représenté par l'une des formules 9-1 à 9-61, 9-201 à 9-237, 10-1 à 10-129 ou 10-201 à 10-350 : 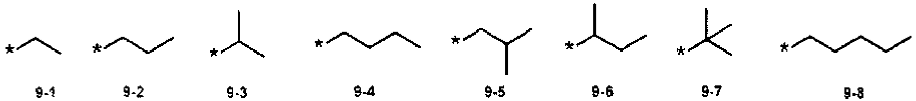 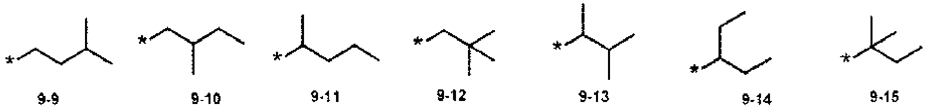 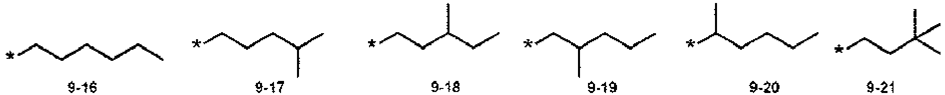 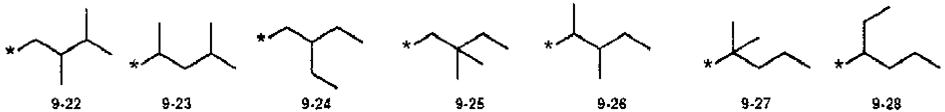 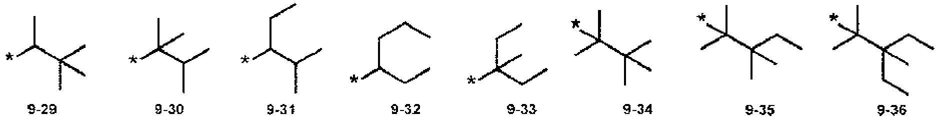 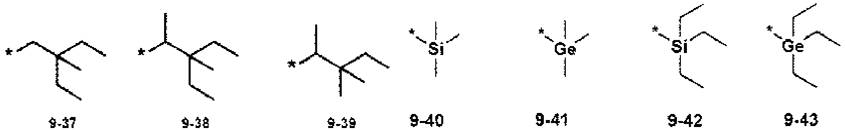 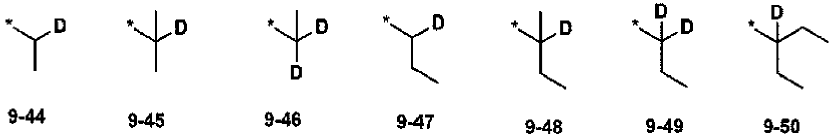 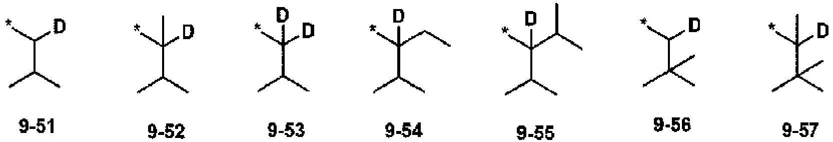 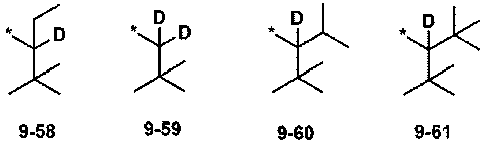 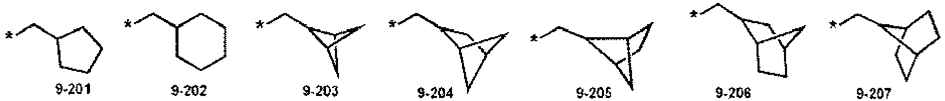 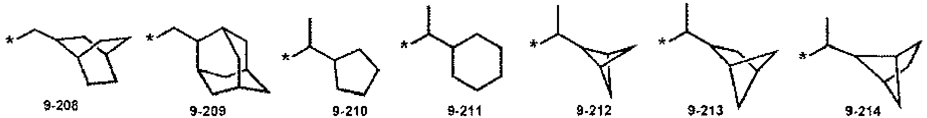 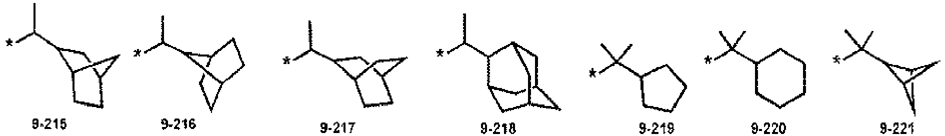 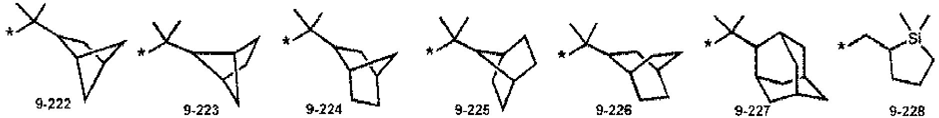 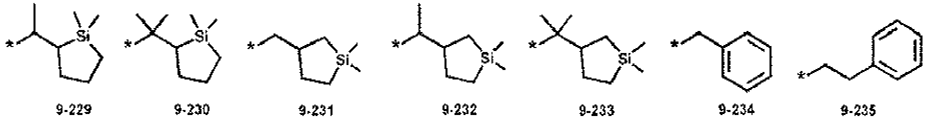 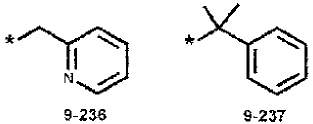 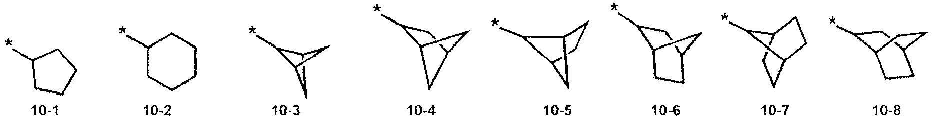 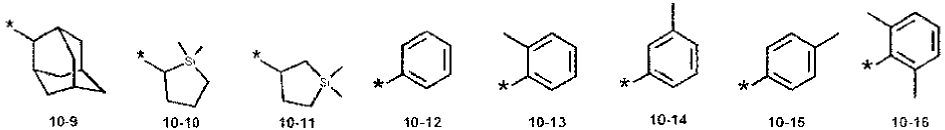 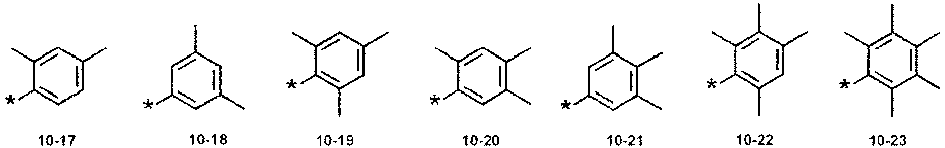 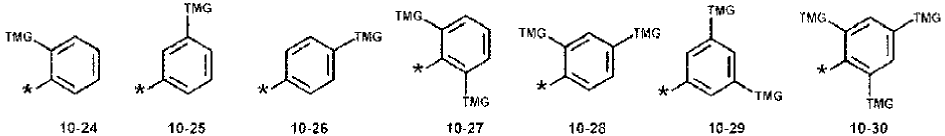 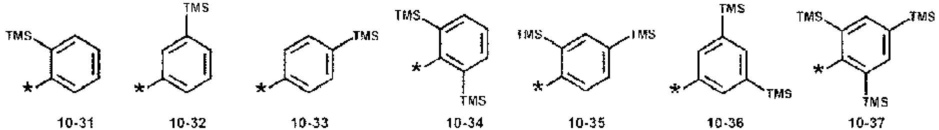 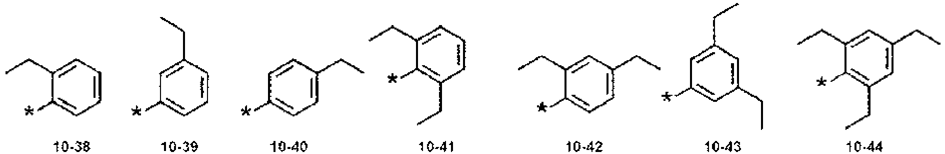 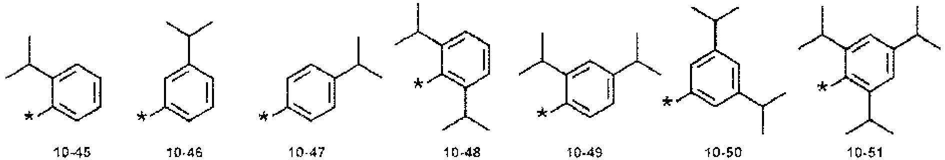 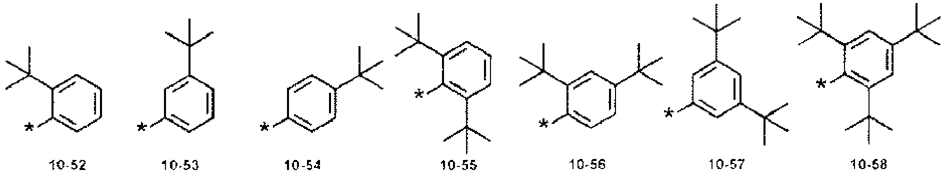 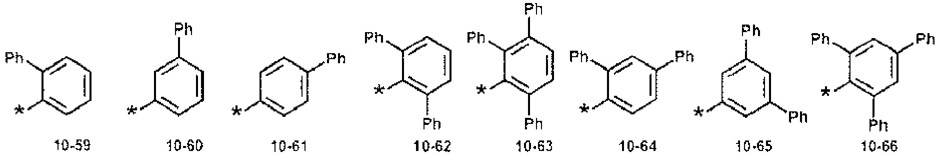 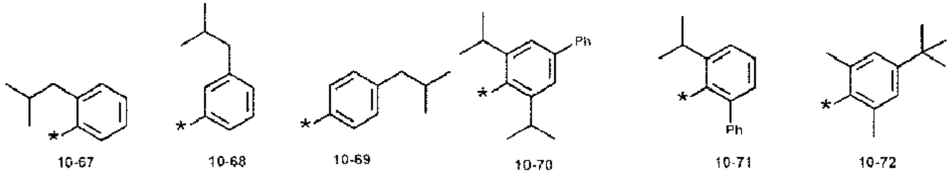 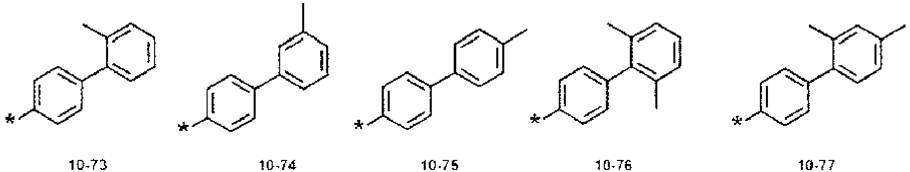 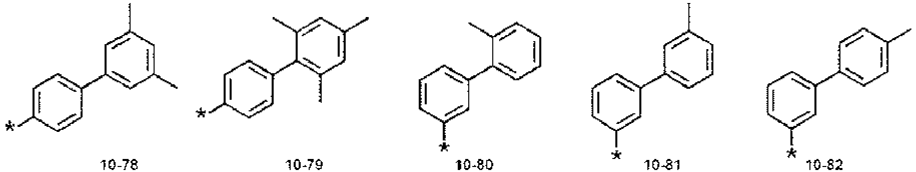 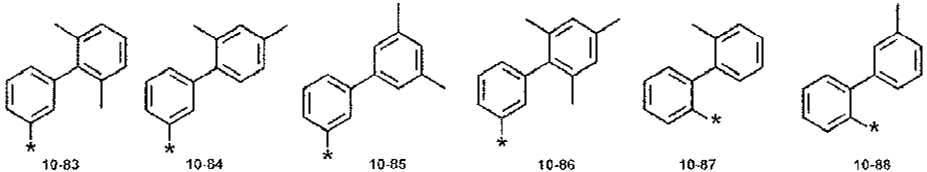 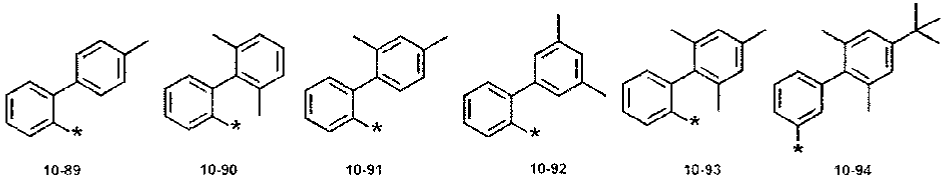 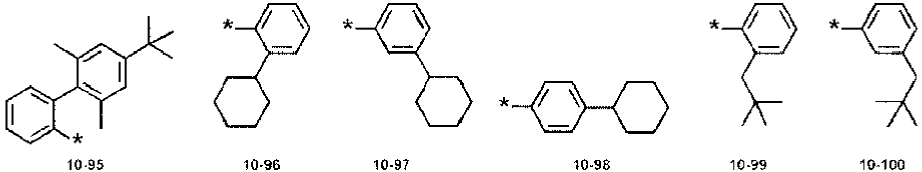 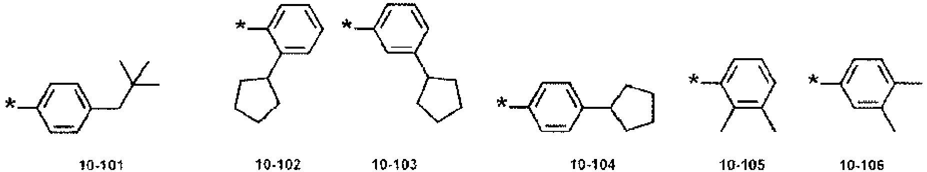 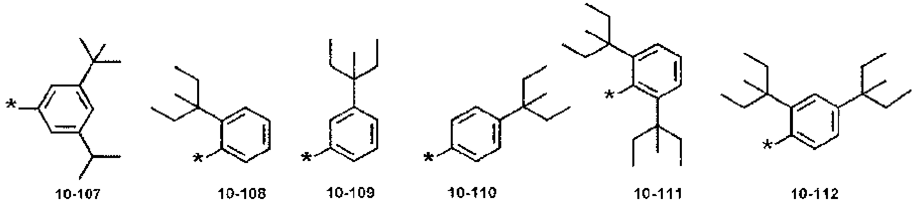 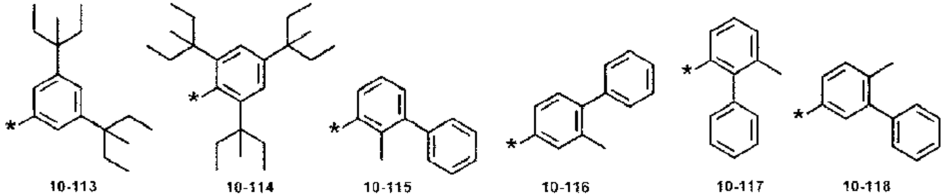 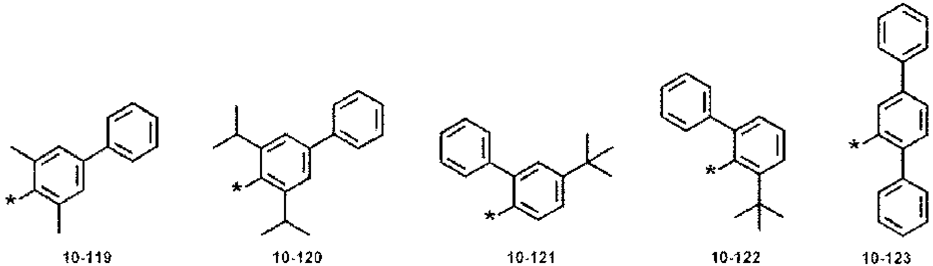 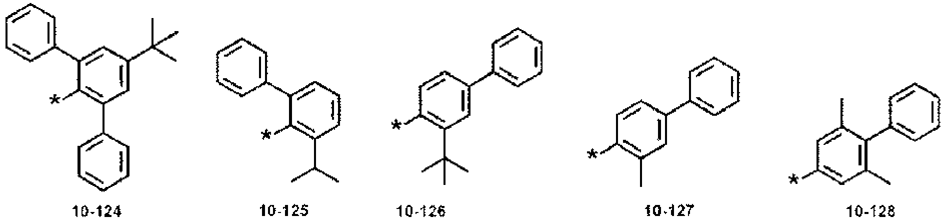 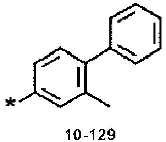 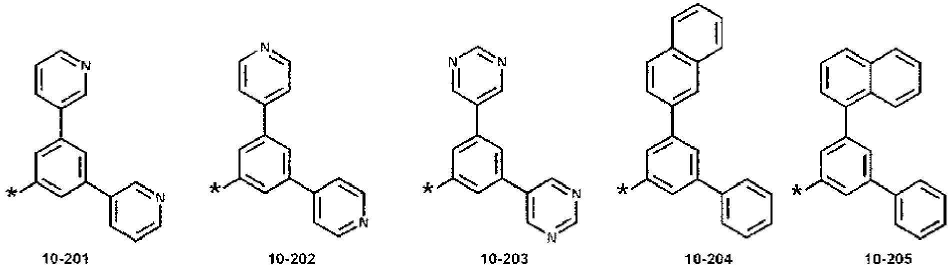 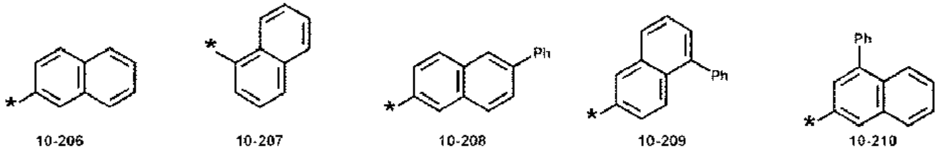 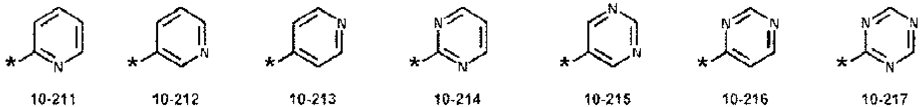 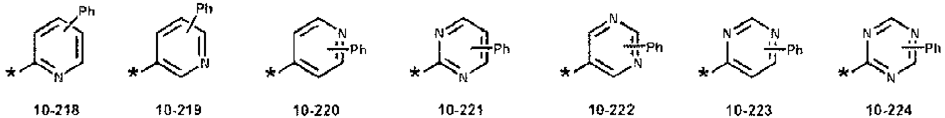 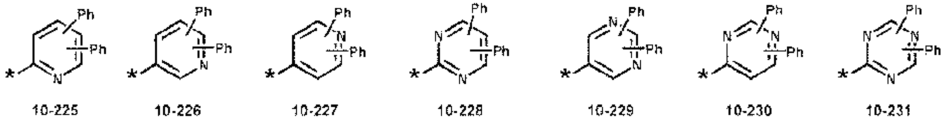 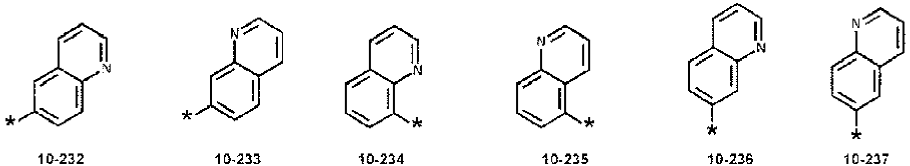 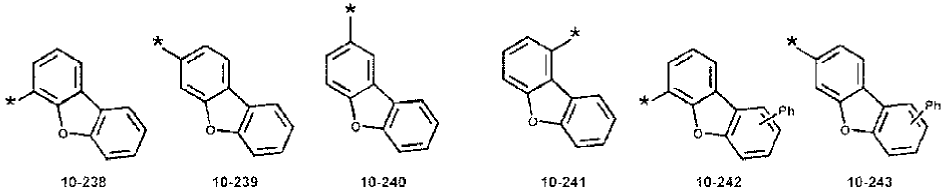 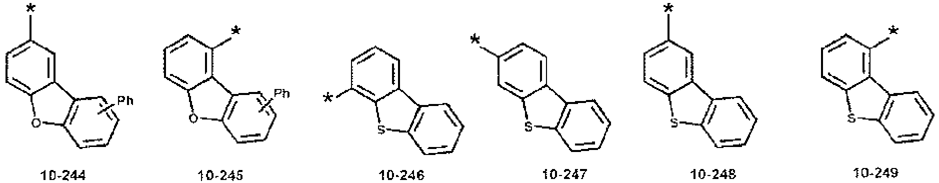 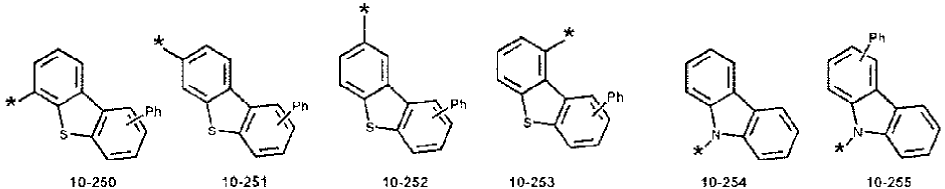 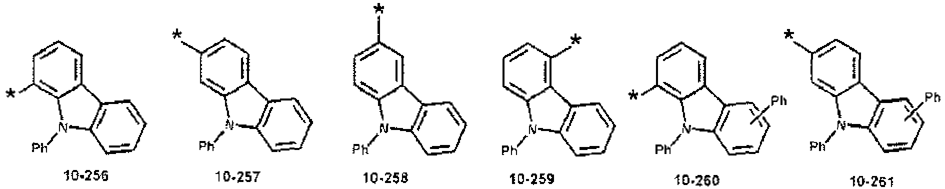 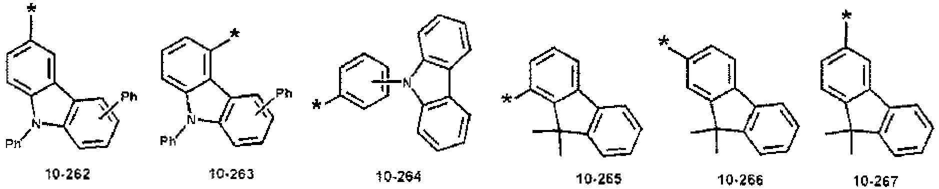 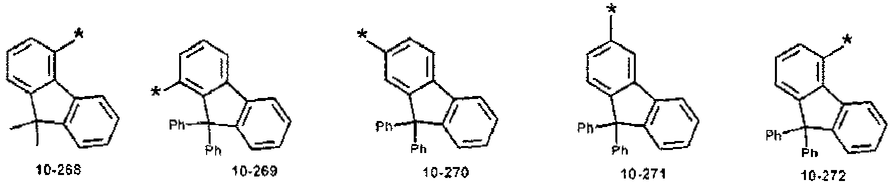 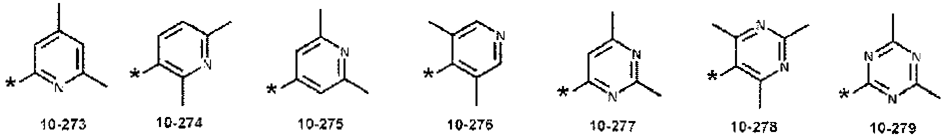 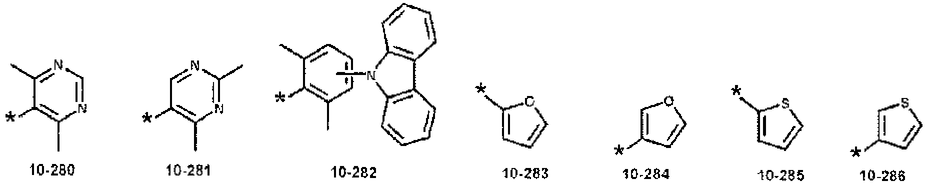 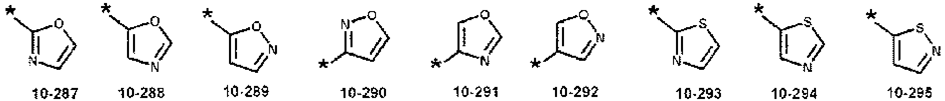 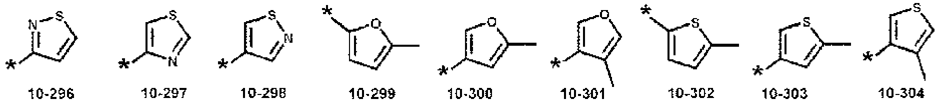 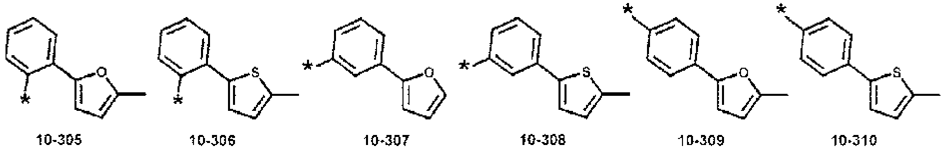 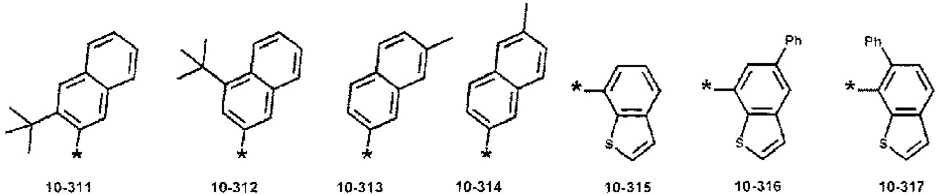 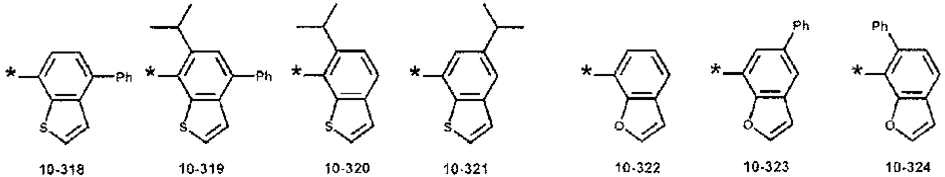 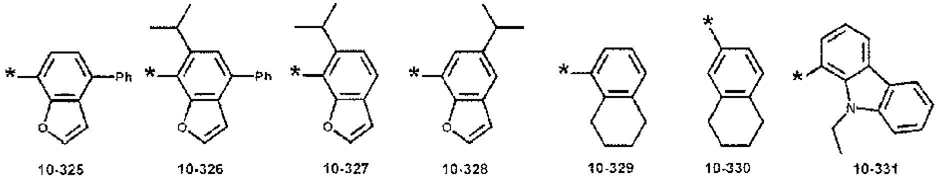 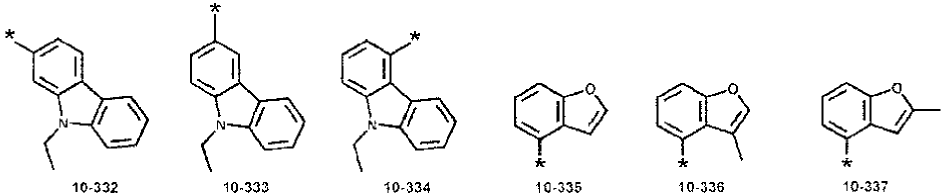 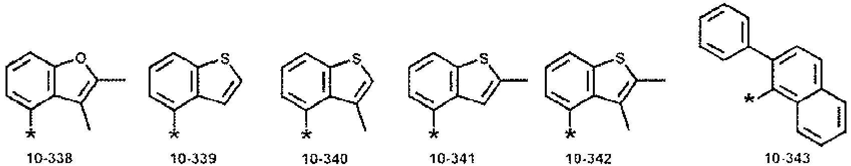 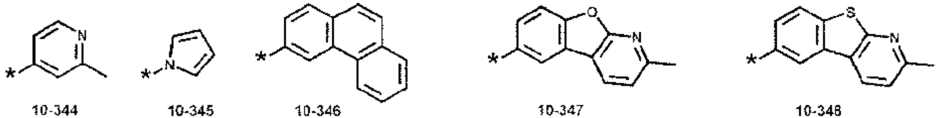 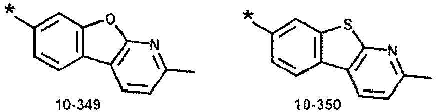
*dans les formules 9-1 à 9-61, 9-201 à 9-237, 10-1 à 10-129 et 10-201 à 10-350 indique un site de liaison à un atome voisin, Ph est un groupe phényle, TMS est un groupe triméthylsilyle et TMG est un groupe triméthylgermyle.

6. Composé organométallique selon l'une quelconque des revendications 1 à 5, dans lequel le composé organométallique comprend un, deux, trois ou quatre groupes germanyle ; et/ou
dans lequel au moins l'un des R₃₀(s) dans le nombre de b30 est -Ge(Q₁)(Q₂)(Q₃).

7. Composé organométallique selon l'une quelconque des revendications 1 à 6, dans lequel CY₁ est représenté par l'une des formules 1-1 à 1-16 : 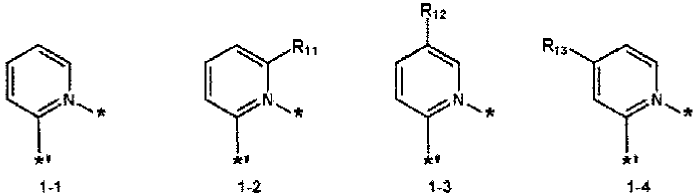 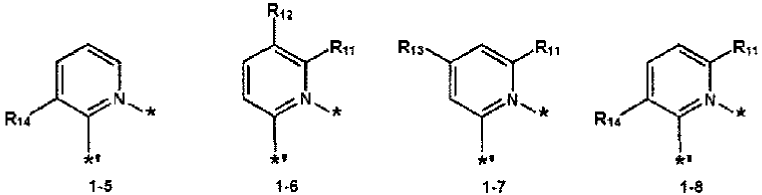 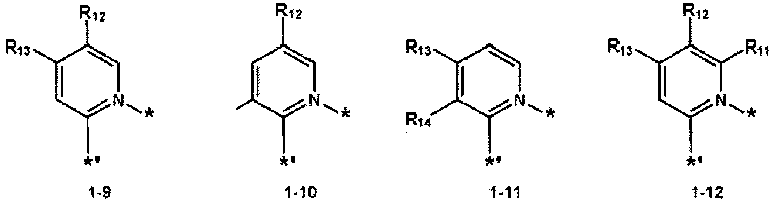 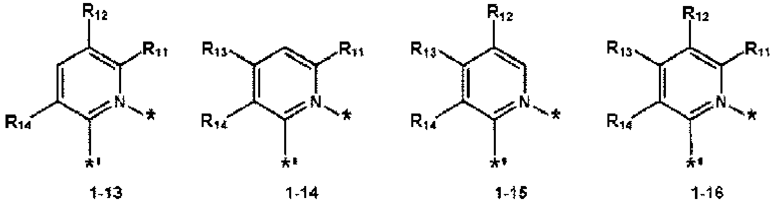 où, dans les formules 1-1 à 1-16,
R₁₁ à R₁₄ sont chacun indépendamment deutérium, -F, -Cl, -Br, -I, -CD₃, -CD₂H, - CDH₂, -CF₃, -CF₂H, -CFH₂, un groupe méthyle, un groupe éthyle, un groupe n-propyle un groupe isopropyle, un groupe n-butyle, un groupe isobutyle, un groupe sec-butyle, un groupe tert-butyle, un groupe n-pentyle, un groupe isopentyle, un groupe 2-méthylbutyle, un groupe sec-pentyle, un groupe tert-pentyle, un groupe néo-pentyle, un groupe 3-pentyle, un groupe 3-méthyl-2-butyle, un groupe phényle, un groupe biphényle, un groupe naphtyle, -Si(Q₁)(Q₂)(Q₃), ou -Ge(Q₁)(Q₂)(Q₃),
Q₁ à Q₃ sont chacun indépendamment :
-CH₃, -CD₃, -CD₂H, -CDH₂, -CH₂CH₃, -CH₂CD₃, -CH₂CD₂H, -CH₂CDH₂, - CHDCH₃, - CHDCD₂H, -CHDCDH₂, -CHDCD₃, -CD₂CD₃, -CD₂CD₂H, ou -CD₂CDH₂;
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle ou un groupe naphtyle ; ou
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle ou un groupe naphtyle, chacun substitué par le deutérium, un groupe alkyle en C₁ à C₁₀, un groupe phényle, ou une combinaison de ceux-ci, et
* et *' indiquent chacun un site de liaison à un atome voisin ; et/ou
dans lequel CY₂ est représenté par l'une des formules 2-1 à 2-16 : 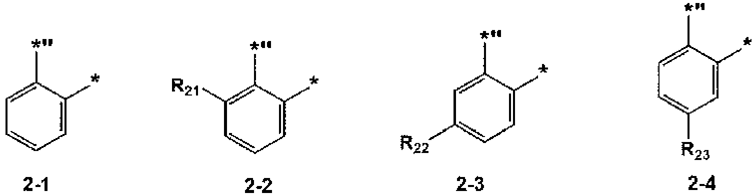 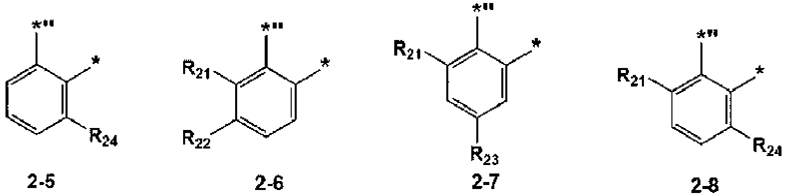 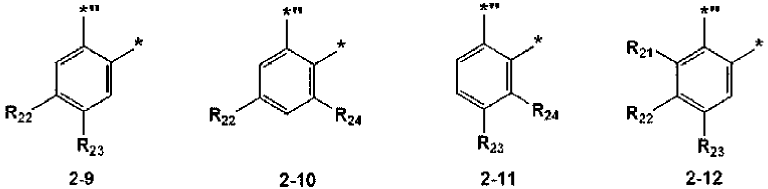 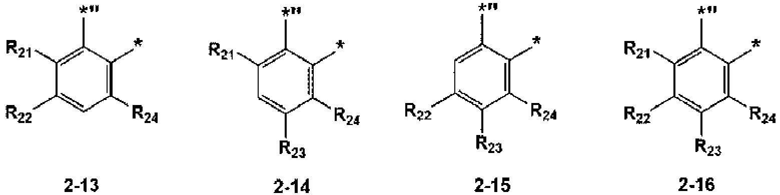
dans lequel, dans les formules 2-1 à 2-16,
R₂₁ à R₂₄ sont chacun indépendamment deutérium, -F, -Cl, -Br, -I, -CD₃, -CD₂H, - CDH₂, -CF₃, -CF₂H, -CFH₂, un groupe méthyle, un groupe éthyle, un groupe n-propyle un groupe isopropyle, un groupe n-butyle, un groupe isobutyle, un groupe sec-butyle, un groupe tert-butyle, un groupe n-pentyle, un groupe isopentyle, un groupe 2-méthylbutyle, un groupe sec-pentyle, un groupe tert-pentyle, un groupe néo-pentyle, un groupe 3-pentyle, un groupe 3-méthyl-2-butyle, un groupe phényle, un groupe biphényle, un groupe naphtyle, -Si(Q₁)(Q₂)(Q₃), ou -Ge(Q₁)(Q₂)(Q₃),
Q₁ à Q₃ sont chacun indépendamment :
-CH₃, -CD₃, -CD₂H, -CDH₂, -CH₂CH₃, -CH₂CD₃, -CH₂CD₂H, -CH₂CDH₂, -CHDCH₃,-CHDCD₂H, -CHDCDH₂, -CHDCD₃, -CD₂CD₃, -CD₂CD₂H, ou -CD₂CDH₂;
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle et un groupe naphtyle ; ou
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle ou un groupe naphtyle, chacun substitué par le deutérium, un groupe alkyle en C₁ à C₁₀, un groupe phényle, ou une combinaison de ceux-ci, et
* et *' indiquent chacun un site de liaison à un atome voisin.

8. Composé organométallique selon l'une quelconque des revendications 1 à 7, dans lequel CY₃ est représenté par l'une des formules 3-1 à 3-40 : 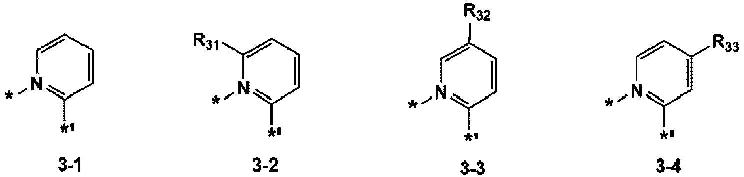 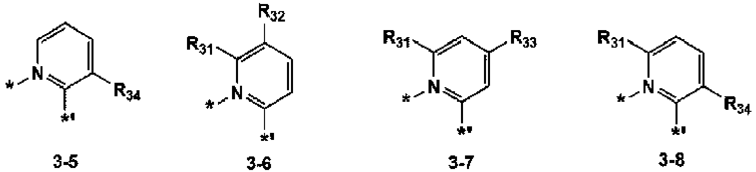 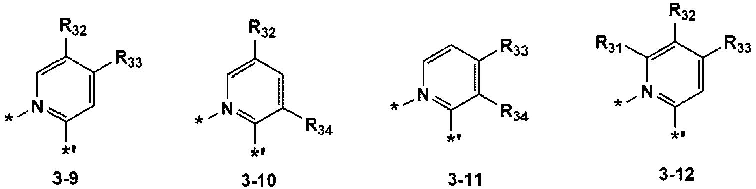 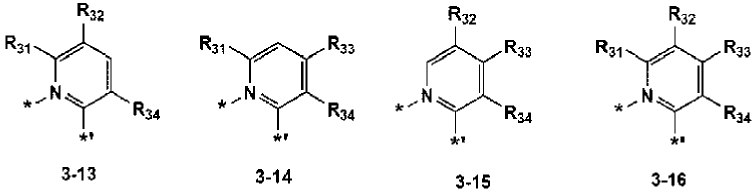 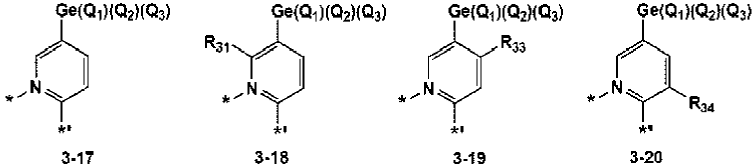 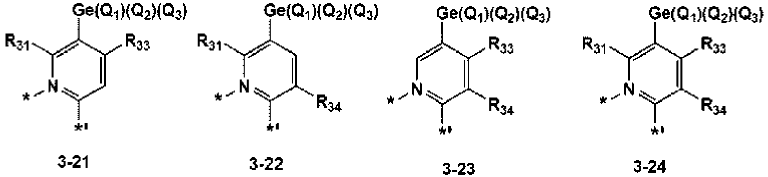 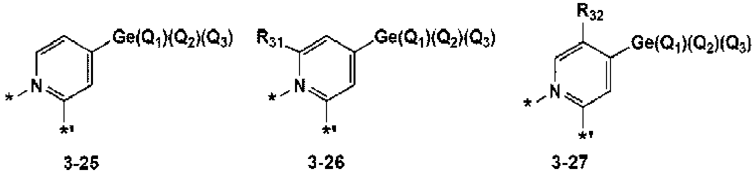 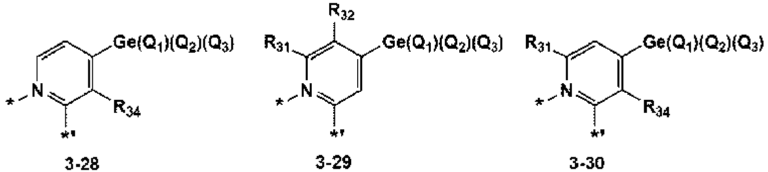 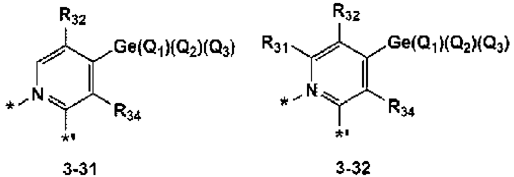 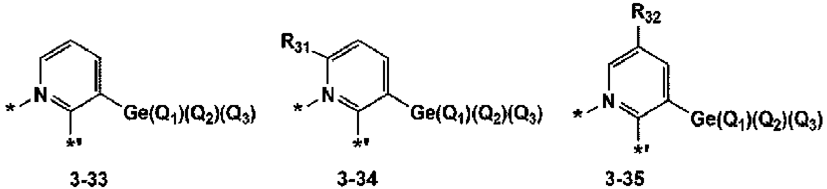 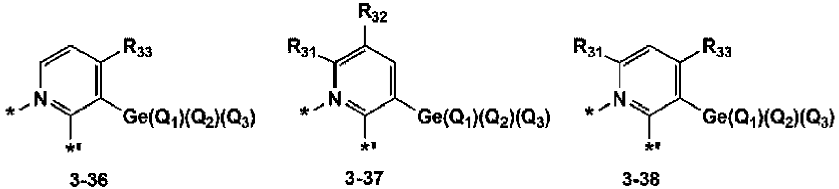 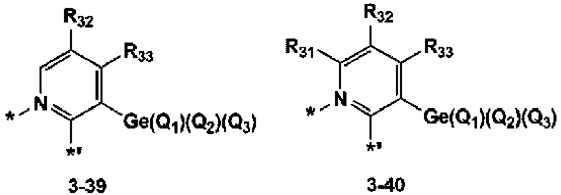 , dans lequel, dans les formules 3-1 à 3-40,
R₃₁ à R₃₄ sont chacun indépendamment deutérium, -F, -Cl, -Br, -I, -CD₃, -CD₂H, -CDH₂, -CF₃, -CF₂H, -CFH₂, un groupe méthyle, un groupe éthyle, un groupe n-propyle un groupe isopropyle, un groupe n-butyle, un groupe isobutyle, un groupe sec-butyle, un groupe tert-butyle, un groupe n-pentyle, un groupe isopentyle, un groupe 2-méthylbutyle, un groupe sec-pentyle, un groupe tert-pentyle, un groupe néo-pentyle, un groupe 3-pentyle, un groupe 3-méthyl-2-butyle, un groupe phényle, un groupe biphényle, un groupe naphtyle, -Si(Q₁)(Q₂)(Q₃), ou -Ge(Q₁)(Q₂)(Q₃),
Q₁ à Q₃ sont chacun indépendamment :
-CH₃, -CD3, -CD₂H, -CDH₂, -CH₂CH₃, -CH₂CD₃, -CH₂CD₂H, -CH₂CDH₂, -CHDCH₃,-CHDCD₂H, -CHDCDH₂, -CHDCD₃, -CD₂CD₃, -CD₂CD₂H, ou -CD₂CDH₂;
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle ou un groupe naphtyle ; ou
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle et un groupe naphtyle, chacun substitué par le deutérium, un groupe alkyle en C₁ à C₁₀, un groupe phényle, ou une combinaison de ceux-ci, et
* et *' indiquent chacun un site de liaison à un atome voisin.

9. Composé organométallique selon l'une quelconque des revendications 1 à 8, dans lequel le composé organométallique est représenté par la formule 11-1 : 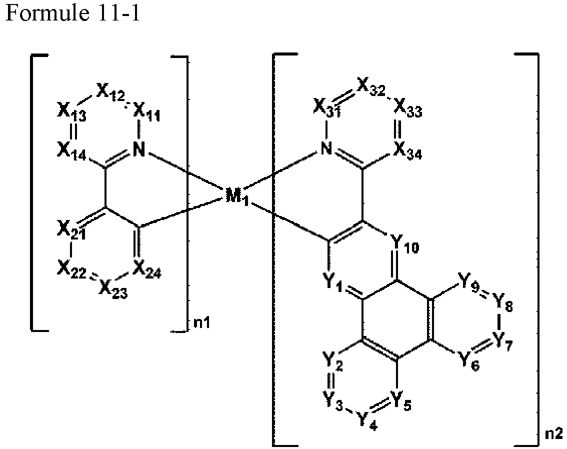 dans lequel, dans la Formule 11-1,
M₁, n1, n2, et Y₁ à Y₁₀ sont chacun identiques à ceux décrits dans la revendication 1,
X₁₁ est C(R₁₁) ou N, X₁₂ est C(R₁₂) ou N, X₁₃ est C(R₁₃) ou N, et X₁₄ est C(R₁₄) ou N,
X₂₁ est C(R₂₁) ou N, X₂₂ est C(R₂₂) ou N, X₂₃ est C(R₂₃) ou N, et X₂₄ est C(R₂₄) ou N,
X₃₁ est C(R₃₁) ou N, X₃₂ est C(R₃₂) ou N, X₃₃ est C(R₃₃) ou N, et X₃₄ est C(R₃₄) ou N,
Ru à R₁₄ sont chacun indépendamment identiques à ceux décrits en relation avec R₁₀ dans la revendication 1,
R₂₁ à R₂₄ sont chacun indépendamment identiques à ceux décrits en relation avec R₂₀ dans la revendication 1,
R₃₁ à R₃₄ sont chacun indépendamment identiques à ceux décrits en relation avec R₃₀ dans la revendication 1,
deux ou plus de Ru à R₁₄ sont éventuellement liés l'un à l'autre pour former un groupe carbocyclique en C₅ à C₃₀ substitué ou non substitué par au moins un R₁₀ₐ ou un groupe hétérocyclique en C₁ à C₃₀ substitué ou non substitué par au moins un R₁₀ₐ,
deux ou plus de R₂₁ à R₂₄ sont éventuellement liés l'un à l'autre pour former un groupe carbocyclique en C₅ à C₃₀ substitué ou non substitué par au moins un R₁₀ₐ ou un groupe hétérocyclique en C₁ à C₃₀ substitué ou non substitué par au moins un R₁₀ₐ,
deux ou plus de R₃₁ à R₃₄ sont éventuellement liés l'un à l'autre pour former un groupe carbocyclique en C₅ à C₃₀ substitué ou non substitué par au moins un R₁₀ₐ ou un groupe hétérocyclique en C₁ à C₃₀ substitué ou non substitué par au moins un R₁₀ₐ, et R₁₀ₐ est le même que décrit en relation avec R₁₀.

10. Composé organométallique selon la revendication 1, dans lequel le composé organométallique est l'un quelconque des composés 1 à 36 :

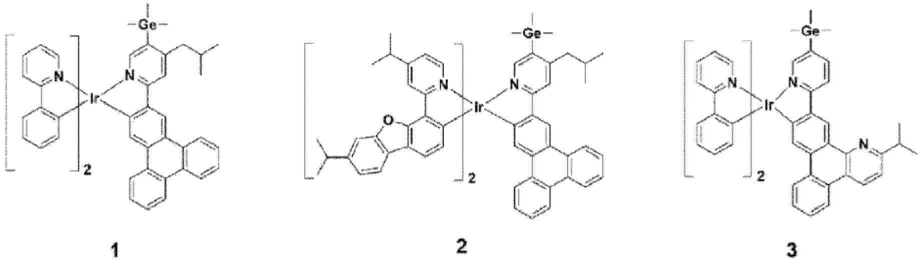

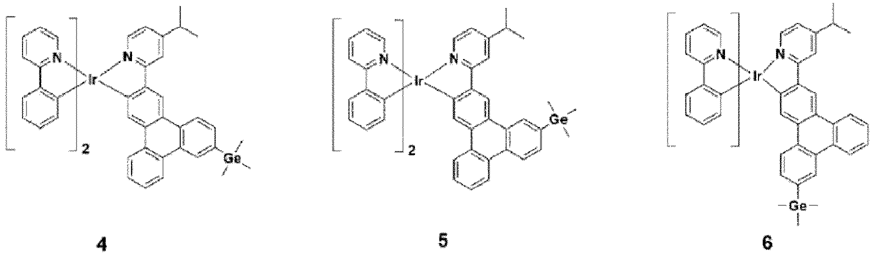

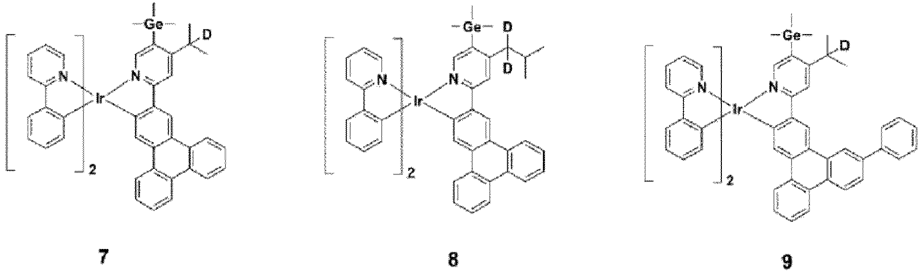

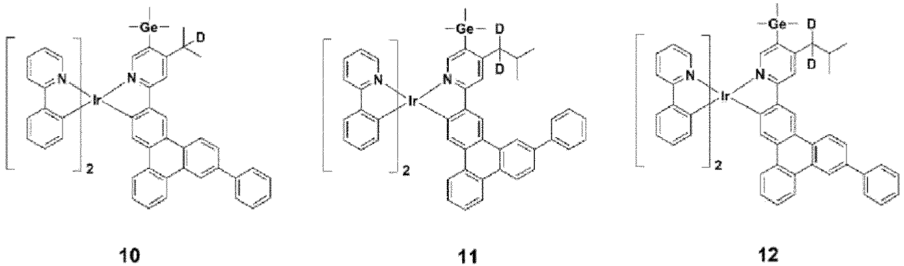

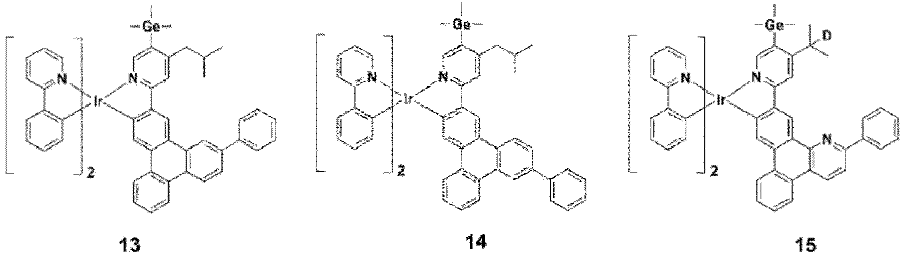

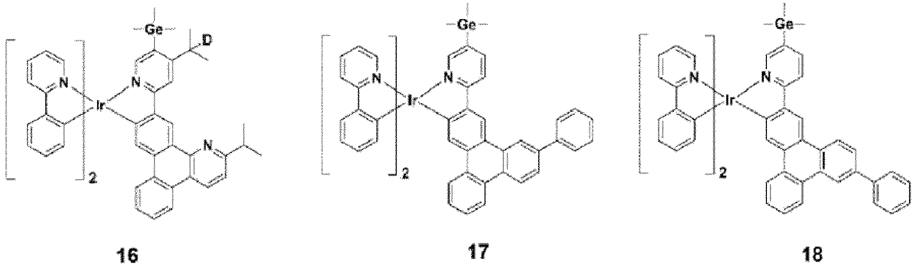

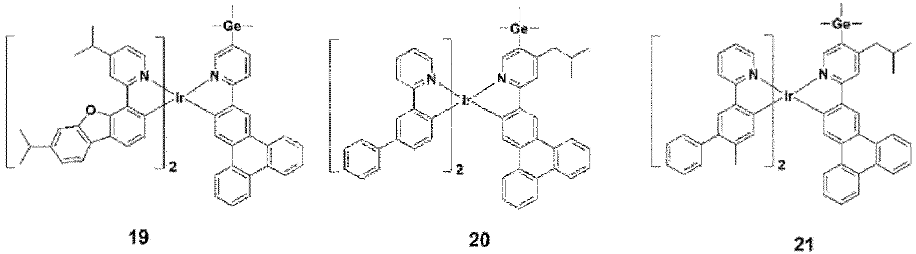

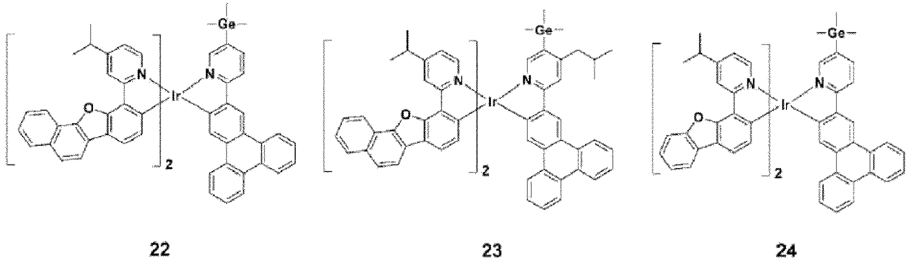

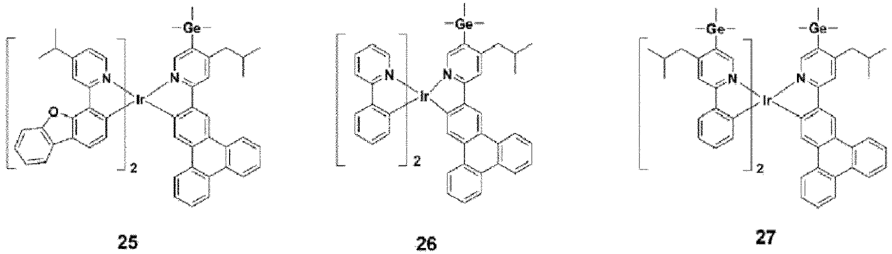

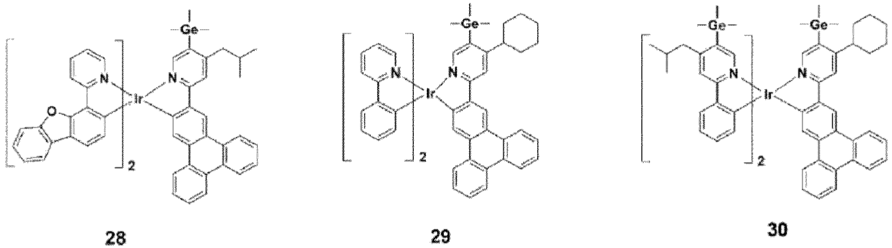

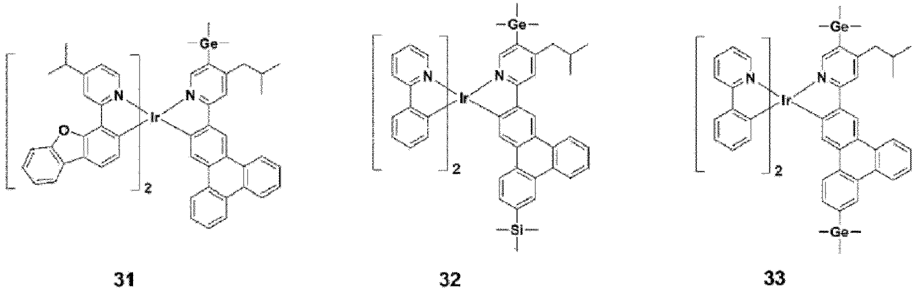

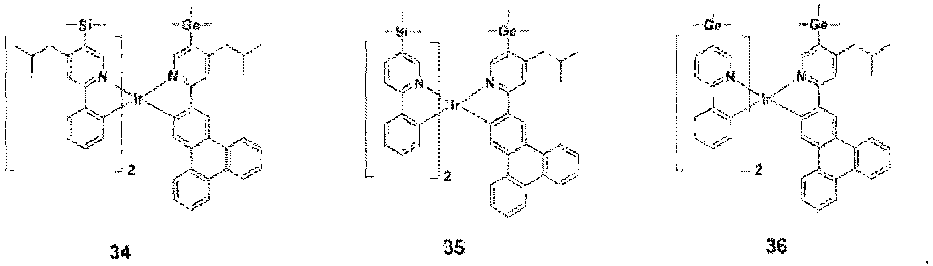

11. Dispositif électroluminescent organique, comprenant :
une première électrode ;
une seconde électrode ; et
une couche organique disposée entre la première électrode et la seconde électrode et comprenant une couche d'émission,
dans lequel la couche organique comprend le composé organométallique selon l'une quelconque des revendications 1 à 10.

12. Dispositif électroluminescent organique selon la revendication 11, dans lequel le composé organométallique est inclus dans la couche d'émission.

13. Dispositif électroluminescent organique selon la revendication 12, dans lequel la couche d'émission comprend en outre un hôte et la quantité de l'hôte est supérieure à la quantité du composé organométallique ; et/ou
dans lequel la couche d'émission émet une lumière verte comportant une longueur d'onde d'émission maximale de 500 nm à 600 nm.

14. Dispositif électroluminescent organique selon la revendication 12 ou 13, dans lequel la première électrode est une anode,
la seconde électrode est une cathode,
la couche organique comprend en outre une région de transport de trous disposée entre la première électrode et la couche d'émission et une région de transport d'électrons disposée entre la couche d'émission et la seconde électrode,
la région de transport de trous comprend une couche d'injection de trous, une couche de transport de trous, une couche de blocage d'électrons, une couche tampon, ou une combinaison de celles-ci, et
la région de transport d'électrons comprend une couche de blocage de trous, une couche de transport d'électrons, une couche d'injection d'électrons ou une combinaison de celles-ci.

15. Composition de diagnostic comprenant au moins l'un des composés organométalliques selon l'une quelconque des revendications 1 à 10.
